# EUROPEAN PATENT SPECIFICATION

(11) **EP 3 600 374 B1**
(45) Date of publication and mention of the grant of the patent: **15.05.2024**
(21) Application number: 18774987.4
(22) Date of filing: 29.03.2018
(51) Int. Cl.: A61K 38/10, A61K 39/39, A61P 31/16, A61P 11/00

(54) **TREATMENT OF RESPIRATORY INFECTION**
BEHANDLUNG VON ATEMWEGSINFEKTION
TRAITEMENT D'UNE INFECTION RESPIRATOIRE

(30) Priority: 31.03.2017 AU 2017901180; 21.12.2017 AU 2017905124; 21.12.2017 AU 2017905128; 09.02.2018 AU 2018900409
(43) Date of publication of application: 05.02.2020
(73) Proprietor: ENA Respiratory Pty Ltd, Sydney, NSW 2000 (AU)
(72) Inventor: BARTLETT, Nathan, Melbourne, VIC 3000 (AU); GIRKIN, Jason, Melbourne, VIC 3000 (AU); JACKSON, David, Melbourne, VIC 3000 (AU); ZENG, Weiguang, Melbourne, VIC 3000 (AU); HOLMES, Ian, Melbourne, VIC 3000 (AU); DEMAISON, Christophe, Melbourne, VIC 3000 (AU)
(74) Representative: Weickmann & Weickmann PartmbB
(86) International application number: PCT/AU2018/050295
(87) International publication number: WO 2018/176099

(56) References cited:
- WO-A1-2011/119759
- WO-A1-2016/037240
- WO-A2-2005/079419
- WO-A2-2009/137103
- AMABEL C. L. TAN ET AL: "Intranasal Administration of the TLR2 Agonist Pam2Cys Provides Rapid Protection against Influenza in Mice", MOLECULAR PHARMACEUTICS, vol. 9, no. 9, 3 August 2012 (2012-08-03), pages 2710-2718, XP055620503, US ISSN: 1543-8384, DOI: 10.1021/mp300257x
- CONTOLI, M. et al.: "Viral infections in exacerbations of asthma and chronic obstructive pulmonary disease", Minerva Medica, vol. 100, no. 6, 2009, pages 467-478, XP009516840, ISSN: 0026-4806
- VOSS, S. et al.: "The activity of lipopeptide TLR2 agonists critically depends on the presence of solubilizers", European Journal of Immunology, vol. 37, 2007, XP002518778, DOI: doi:10.1002/EJI.200737537

## Description

### Field of the invention

The present invention relates to compounds for use in the prevention or treatment of respiratory conditions. In particular, the compounds are for use in the prevention and/or treatment of rhinovirus infection and the prevention and/or treatment of respiratory exacerbations.

### Background of the invention

Respiratory infections are among the most common causes of human disease worldwide and are commonly caused by viruses. Rhinoviruses (RV) are one of the most common types of virus to infect humans and are known to cause the common cold. Unlike sporadic pandemic and seasonal influenza outbreaks, rhinovirus infections occur throughout the year with multiple different serotypes. On average children experience 5-10 colds per year and well over half of all colds are caused by RV infection.

Viral respiratory infections can worsen the severity of diseases of the respiratory conditions leading to exacerbations (attacks). Exacerbations can occur for conditions such as asthma and chronic obstructive pulmonary disease (COPD). Asthma and COPD exacerbations are the most clinically and economically important forms of the diseases. Rhinovirus is the most common viral infection associated with asthma exacerbations and therefore accounts for the greatest burden in terms of morbidity, mortality and health care cost.

The vast majority of exacerbations, particularly in asthma, continue to occur despite use of the best available current therapies. When exacerbations do occur, treatment options are limited and have developed little in recent years. Treatment involves increasing doses of inhaled bronchodilators and systemic or oral corticosteroids - which are the same drugs that failed to prevent the exacerbation occurring in the first place.

There is therefore a need for new or improved therapies for the treatment and / or prevention of rhinovirus mediated respiratory conditions. In addition, there is a need for new or improved therapies for the treatment and/or prevention of viral mediated exacerbations.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

### Summary of the invention

The present invention provides a compound comprising a TLR2 agonist for use in treating or preventing a respiratory condition associated with rhinovirus in a subject, wherein the TLR2 agonist comprises a lipopeptide or a lipid moiety, wherein the lipid moiety is selected from palmitoyl, myristoyl and stearoyl, and wherein the TLR2 agonist is linked to a solubilising moiety, wherein the solubilising moiety comprises a polyethyleneglycol (PEG).

Further, the present invention provides a compound comprising a TLR2 agonist for use in treating or preventing a viral mediated exacerbation of a respiratory condition in a subject, wherein the viral mediated exacerbation is a rhinovirus mediated exacerbation, wherein the TLR2 agonist comprises a lipopeptide or a lipid moiety, wherein the lipid moiety is selected from palmitoyl, myristoyl and stearoyl, and wherein the TLR2 agonist is linked to a solubilising moiety, wherein the solubilising moiety comprises a polyethyleneglycol (PEG). Preferably, the intended use is in a method that comprises administering only a compound comprising a TLR2 agonist. In other words, the method does not comprise administering agonists of TLRs other than TLR2 homodimers or heterodimers.

The compound may be administered in a composition. Typically, the composition further comprises a pharmaceutically acceptable carrier, diluent or excipient. The composition may be formulated for administration to the respiratory tract, for example by inhalation or intranasally. The composition may be free of compounds that are agonists of TLRs other than TLR2 homodimers or heterodimers. Preferably, the composition consists essentially of, or consists of a compound comprising a TLR2 agonist and a pharmaceutically acceptable carrier, diluent or excipient.

For example, the respiratory condition may be chronic obstructive pulmonary disease (COPD), asthma, cystic fibrosis or lung conditions associated with lung transplantation or chronic glucocorticosteroid use.

According to the invention, the respiratory condition is caused by a rhinovirus or exacerbation of the respiratory condition is mediated by rhinovirus. For example, the viral mediated exacerbation of asthma is caused by a rhinovirus. The rhinovirus may be any serotype as described herein. Typically, the rhinovirus is a rhinovirus serotype 1 B (RV1 B).

The TLR2 agonist comprises a lipopeptide or a lipid moiety, wherein the lipid moiety is selected from palmitoyl, myristoyl, and stearoyl. The TLR2 agonist may be selected from the group consisting of: Pam2Cys, Pam3Cys, and Ste2Cys. In a preferred embodiment, the TLR2 agonist comprises Pam2Cys.

In any aspect of the invention, the TLR2 agonist may be conjugated with other compounds or functional groups. Other compounds or functional groups are any of those described herein. Preferred compounds are selected on the basis to assist in dissolving the TLR2 agonist in a carrier, diluent, excipient or solvent.

Solubility of the TLR2 agonist is increased by a solubilising agent comprising polyethyleneglycol (PEG). Therefore, the compound comprises a TLR2 agonist linked to a solubilising moiety comprising PEG.

In any aspect of the invention, the compound comprises Pam2Cys conjugated to PEG_{11.} Preferably, the Pam2Cys and PEG₁₁ molecules are separated by two serines (PEG₁₁-SS-Pam2Cys).

In any aspect of the invention, the TLR2 agonist is not Pam3Cys.

Although the following describes medical treatment procedures and administration to a subject, these steps are not to be considered as included within the scope of the invention. Instead, the invention relates to compounds for use in the methods described, as defined in the appended claims. In any aspect of the invention, the TLR2 agonist is provided for administration once daily, once weekly or twice weekly. Generally, the step of administration to a subject is not within the scope of the invention.

Where prevention or prophylaxis is intended or required, the compound is administered to the subject before any clinically or biochemically detectable symptoms of viral infection, preferably rhinovirus infection.

The compound can be administered in a composition. Typically, the composition further comprises a pharmaceutically acceptable carrier, diluent or excipient. The composition may be free of compounds that are agonists of TLRs other than TLR2 homodimers or heterodimers. Preferably, the composition consists essentially of, or consists of, a compound comprising a TLR2 agonist and a pharmaceutically acceptable carrier, diluent or excipient.

The compound or composition can be administered to the respiratory tract. Typically, the compound or composition is administered to the upper and/or lower respiratory tract. For example, the compound or composition may be administered via inhalation or intranasally to the subject.

Administration of the TLR2 agonist to a subject may reduce viral load in a subject. Preferably, the viral load is reduced in the respiratory tract, for example the upper and/or lower respiratory tract. Preferably, the viral load is reduced in the lungs.

Administration of the TLR2 agonist to a subject may reduce levels of CXCL1 or TNFα.

In any aspect of the invention, treatment or prevention of a viral mediated exacerbation of asthma does not significantly induce interferon expression.

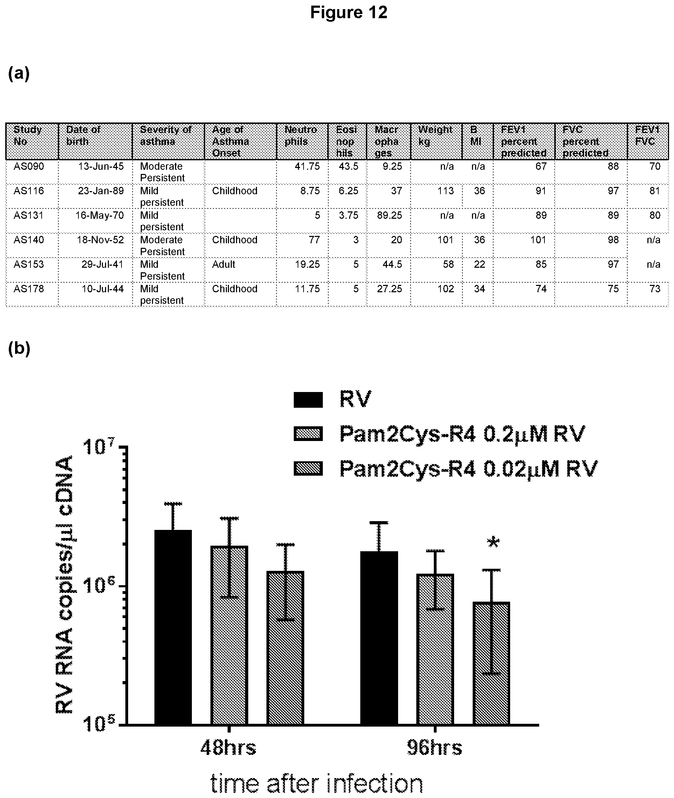

In any aspect of the invention, the subject suffers from mild or moderate asthma. The asthma may be childhood or adult onset. The asthma sufferer may have any characteristics of the condition as outlined in Figure 12a.

The compound or composition may be administered with a corticosteroid. Specifically, any method or use may further comprise administering a corticosteroid. The compound or composition may be administered simultaneously or sequentially to the corticosteroid. The compound or composition may be administered one, two or more times over a 24 hour or 7 day period before the corticosteroid is administered.

The subject to whom the compound or composition is administered may be receiving, or has received, a corticosteroid.

In any aspect of the invention, the corticosteroid may be a glucocorticoid. Preferably the glucocorticoid is an agonist, partial agonist or allosteric modulator of a glucocorticoid receptor. Preferably, the glucocorticoid is an inhalable glucocorticoid. Even more preferably, the glucocorticoid is budesonide, ciclosenide, mometasone, beclomethasone, betamethasone, dexamethasone, prednisolone, prednisone or any other glucocorticoid described herein such as fluticasone propionate.

Preferably, the compound is any one described herein, even more preferably any one of INNA-001 to INNA-015.

Preferably, the corticosteroid is a glucocorticoid. Preferably the glucocorticoid is an agonist, partial agonist or allosteric modulator of a glucocorticoid receptor. Preferably, the glucocorticoid is an inhalable glucocorticoid. Even more preferably, the glucocorticoid is budesonide, ciclosenide, mometasone, beclomethasone, betamethasone, dexamethasone, prednisolone, prednisone or any other glucocorticoid described herein such as fluticasone propionate.

In this aspect, the composition further comprises a pharmaceutically acceptable diluent, carrier or excipient. Typically the diluent, carrier or excipient is suitable for inhalation or intranasal delivery.

In one embodiment, the only active agents in the composition are a compound comprising a TLR2 agonist and a corticosteroid.

The composition may be formulated or adapted for administration to the respiratory tract, for example the upper or lower respiratory tract. Preferably, the composition is formulated or adapted for inhalation or intranasal administration. The composition may be an inhalant composition and formulated as a dry powder suitable for use in a dry powder inhaler device. Alternatively, the composition may be formulated as a spray, mist, or aerosol.

In a preferred embodiment, the composition is formulated as a nasal spray or as nasal drops.

In one aspect, the compound for use according to the present invention comprises the structure:

A - Y - B

wherein A comprises or consists of: 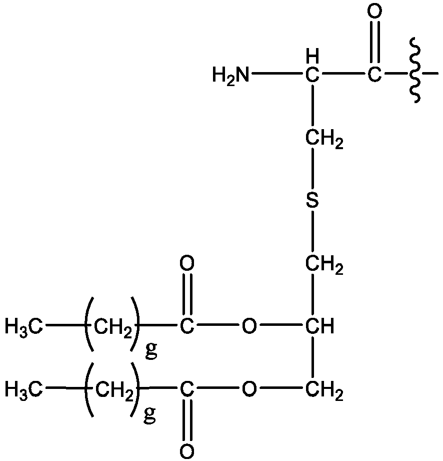 wherein each g is independently 12, 14, or 16;
Y is 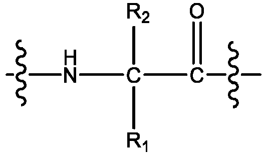 wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
and
B comprises or consists of Polyethylene Glycol (PEG),
or a pharmaceutically acceptable salt or prodrug thereof.
The compound may comprise Pam2Cys and PEG, wherein the Pam2Cys and PEG are linked by a serine, homoserine, threonine or phosphoserine residue,
wherein
Pam2Cys in the compound has the structure:

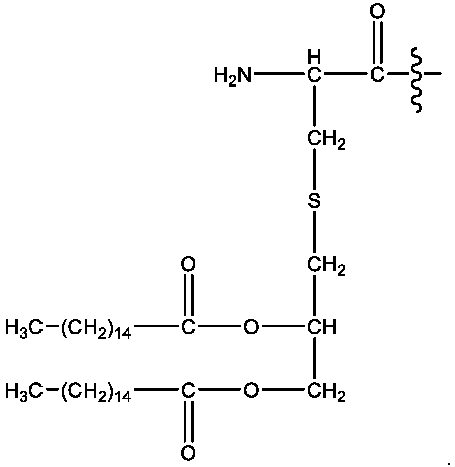

In one aspect, the compound comprises: 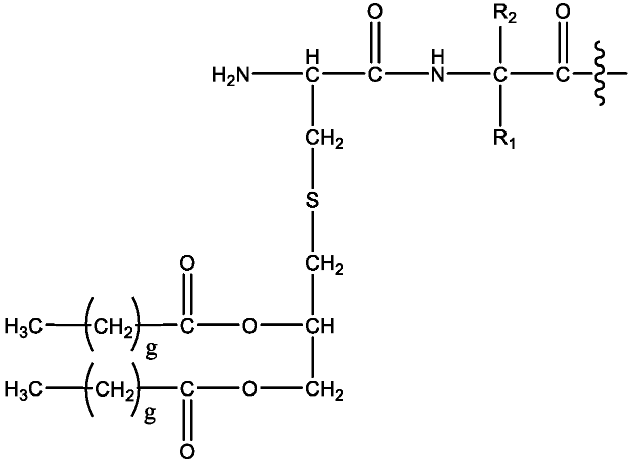
wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
covalently linked to polyethylene glycol (PEG),
or a pharmaceutically acceptable salt or prodrug thereof.

In one aspect, the compound has the structure of formula (I): 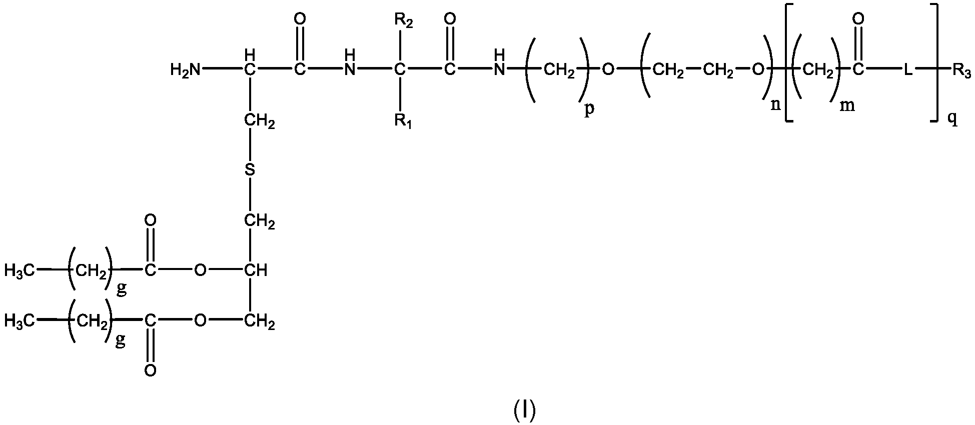 wherein
n is 3 to 100;
m is 1, 2, 3 or 4;
each g is independently 12, 14, or 16;
p is 2, 3 or 4;
q is null or 1;
R₁ and R₂ are independently selected from the group consisting of H, -CH₂OH, - CH₂CH₂OH, -CH(CH₃)OH and -CH₂OPO(OH)₂, wherein any one of the alkyl hydrogens can be replaced with a halogen, and wherein R₁ and R₂ are not both H;
wherein when q= 1, R₃ is -NH₂ or -OH;
wherein when q=0, R₃ is H;
L is null or consists of 1 to 10 units, wherein each unit is a natural alpha amino acid or derived from a natural alpha amino acid, and has the formula: 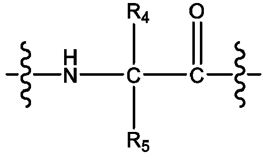
wherein R₄ is H; and
Rs is the side chain, or second hydrogen of the amino acid
or a pharmaceutically acceptable salt or prodrug thereof.

In one embodiment, the compound is represented by formula (II):

A-Y-NH-(CH₂)ₚ-O-(CH₂-CH₂-O)ₙ-[(CH₂)ₘ-CO-L-]_{q}R₃ (II)

wherein
A has the structure: 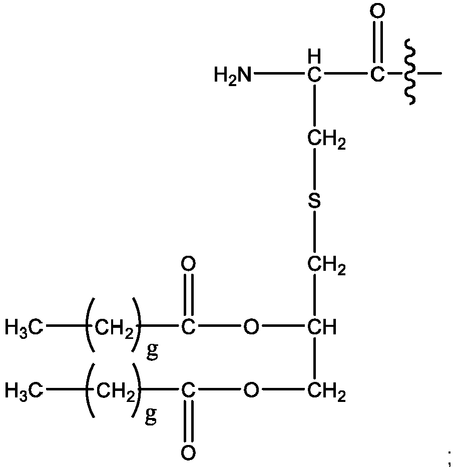
Y is 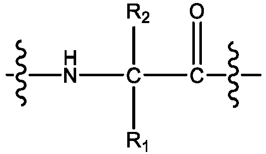
wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
n is 3 to 100;
m is 1, 2, 3 or 4;
each g is independently 12, 14, or 16;
p is 2, 3 or 4;
q is null or 1;
wherein when q= 1, Rs is -NH₂ or -OH;
wherein when q=0, Rs is H;
L is null or consists of 1 to 10 units, wherein each unit is a natural alpha amino acid or derived from a natural alpha amino acid, and has the formula: 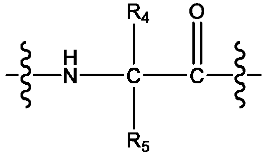
wherein R₄ is H; and
Rs is the side chain, or second hydrogen of the amino acid,
or a pharmaceutically acceptable salt or prodrug thereof.

In one embodiment, the compound has the formula (III):

Pam2Cys-Y-NH-(CH₂)ₚ-O-(CH₂-CH₂-O)ₙ-[(CH₂)ₘ-CO-L-]_{q}R₃ (III)

wherein
Pam2Cys has the structure: 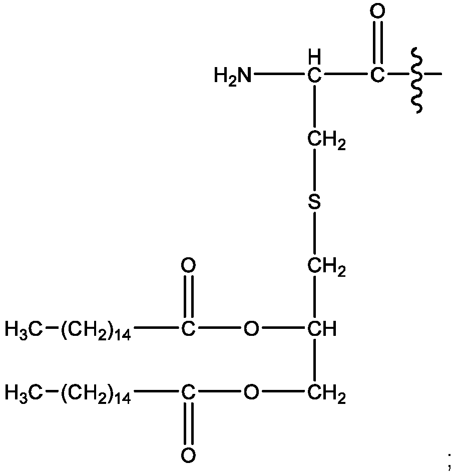
Y is: 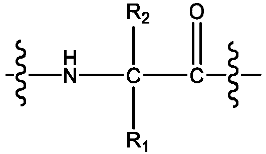
wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
n is 3 to 100;
m is 1, 2, 3 or 4;
p is 2, 3 or 4;
q is null or 1;
wherein when q= 1, R₃ is H, -NH₂ or -OH;
wherein when q=0, R₃ is H;
L is null or consists of 1 to 10 units, wherein each unit is a natural alpha amino acid or derived from a natural alpha amino acid, and has the formula: 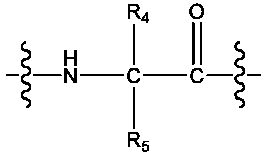
wherein R₄ is H; and
Rs is the side chain, or second hydrogen of the amino acid,
or a pharmaceutically acceptable salt or prodrug thereof.

In one embodiment, the compound has the formula (IV):

Pam2Cys-Ser-NH-(CH₂)ₚ-O-(CH₂-CH₂-O)ₙ-[(CH₂)ₘ-CO-L-]_{q}R₃ (IV)

wherein
Pam2Cys-Ser has the structure: 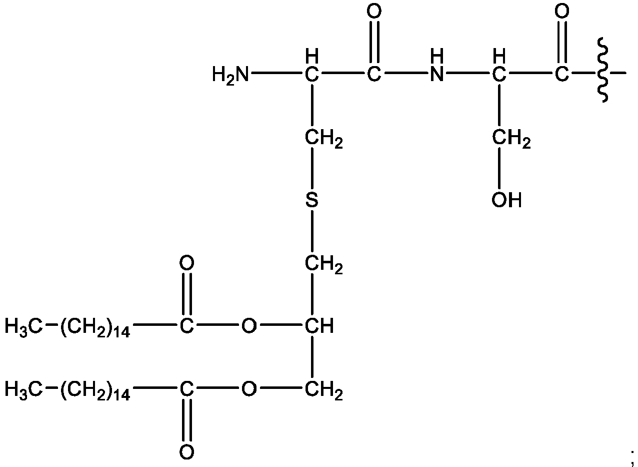
n is 3 to 100;
m is 1, 2, 3 or 4;
p is 2, 3 or 4;
q is null or 1;
wherein when q= 1, R₃ is -NH₂ or -OH;
wherein when q=0, R₃ is H;
L is null or consists of 1 to 10 units, wherein each unit is a natural alpha amino acid or derived from a natural alpha amino acid, and has the formula: 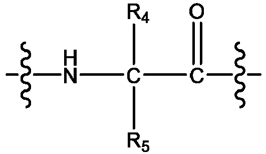
wherein R₄ is H; and
Rs is the side chain, or second hydrogen of the amino acid,
or a pharmaceutically acceptable salt or prodrug thereof.

In one embodiment, the compound has the formula (V): 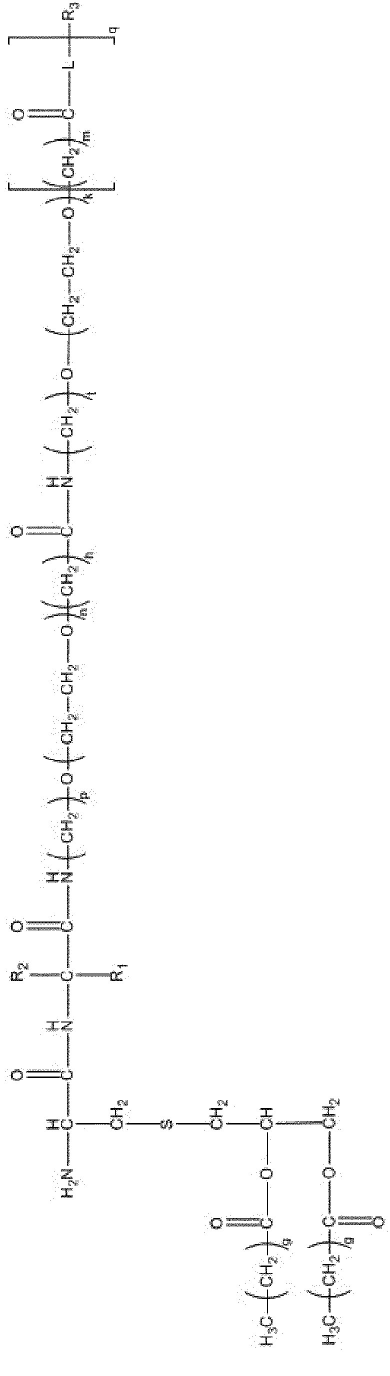 wherein
n is 3 to 100;
k is 3 to 100;
m is 1, 2, 3 or 4;
each g is independently 12, 14, or 16;
p is 2, 3 or 4;
t is 2, 3 or 4;
h is 1, 2, 3 or 4;
q is null or 1;
R₁ and R₂ are independently selected from the group consisting of H, -CH₂OH, - CH₂CH₂OH, -CH(CH₃)OH and -CH₂OPO(OH)₂, wherein any one of the alkyl hydrogens can be replaced with a halogen, and wherein R₁ and R₂ are not both H;
wherein when q= 1, R₃ is -NH₂ or -OH;
wherein when q=0, R₃ is H;
L is null or consists of 1 to 10 units, wherein each unit is a natural alpha amino acid or derived from a natural alpha amino acid, and has the formula: 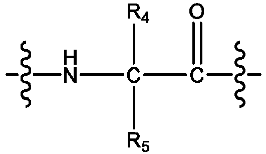
wherein R₄ is H; and
R₅ is the side chain, or second hydrogen of the amino acid,
or a pharmaceutically acceptable salt or prodrug thereof.

In one preferred embodiment, the compound has the structure of compound (1): 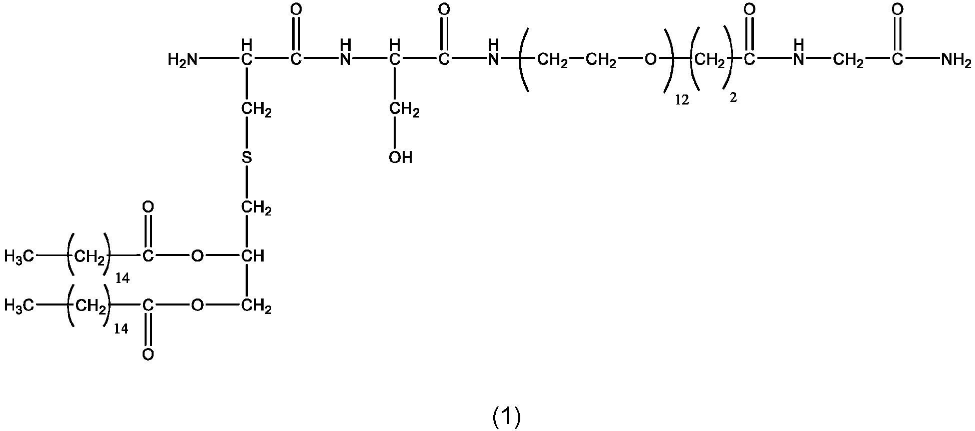 or a pharmaceutically acceptable salt or prodrug thereof.

This compound may also be referred to herein as 'Pam₂Cys-Ser-PEG', or `INNA-006'.

In other preferred embodiments, the compound is selected from the group consisting of: 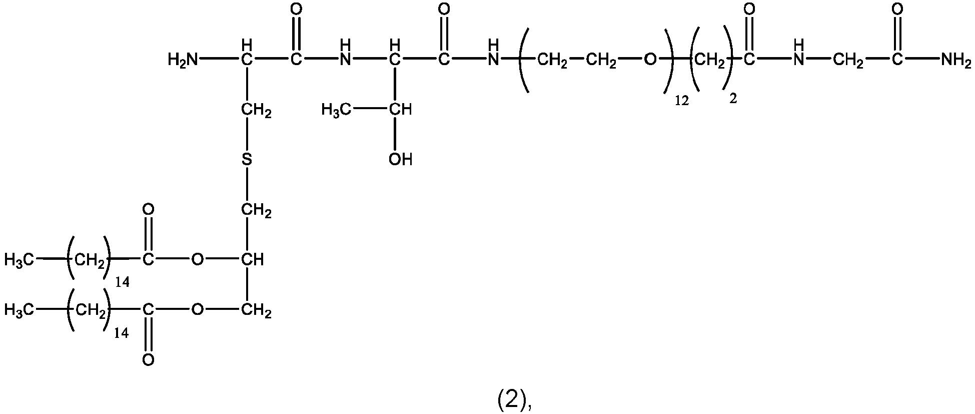 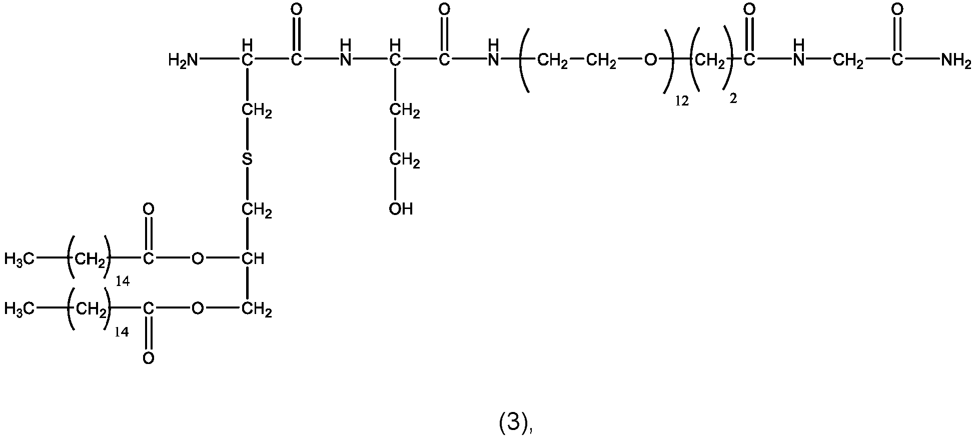 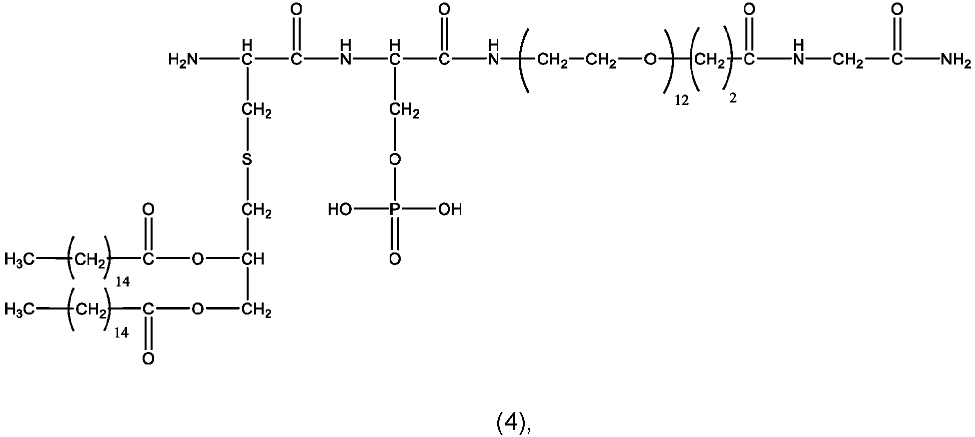 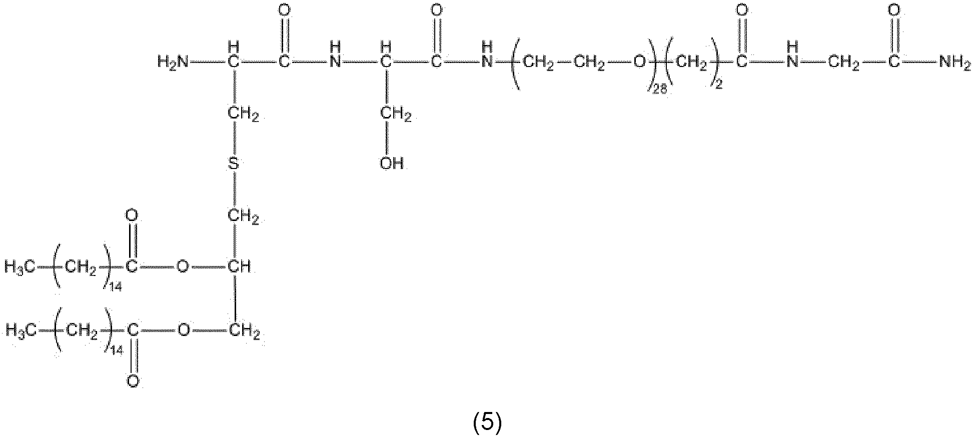 and

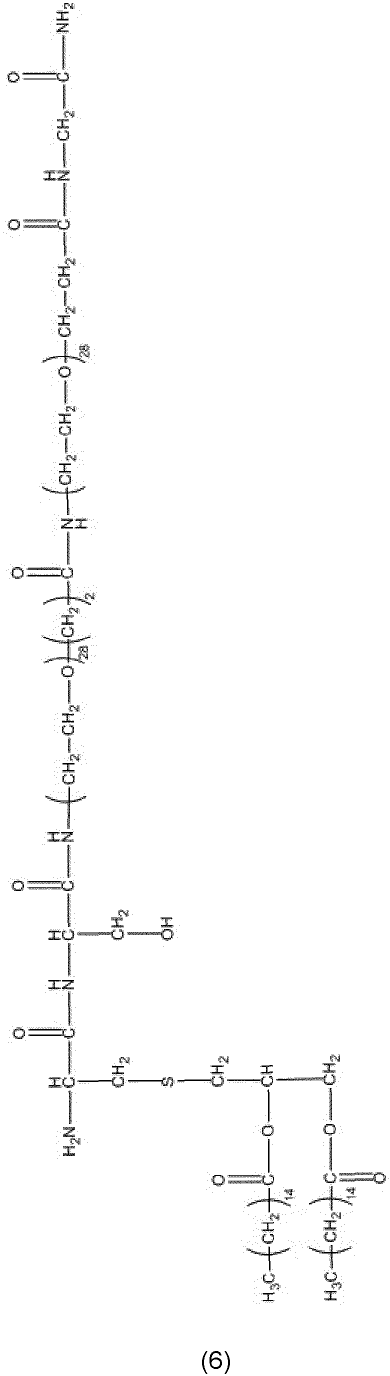

In one particularly preferred embodiment, the compound is:

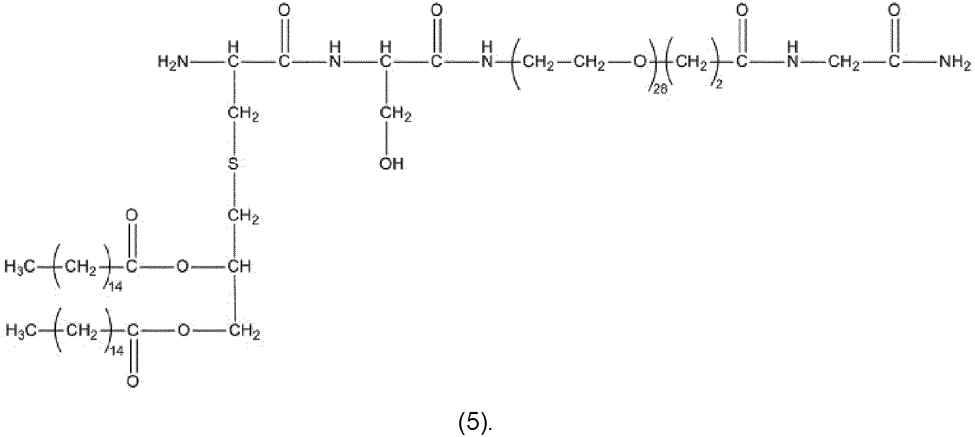

As used herein, except where the context requires otherwise, the term "comprise" and variations of the term, such as "comprising", "comprises" and "comprised", are not intended to exclude further additives, components, integers or steps.

Further aspects of the present invention and further embodiments of the aspects described in the preceding paragraphs will become apparent from the following description, given by way of example and with reference to the accompanying drawings. The description of examples is not intended to be construed as broadening the scope of the invention as defined by the appended claims. Any aspects outside the claims are described for illustrative purposes only.

### Brief description of the drawings

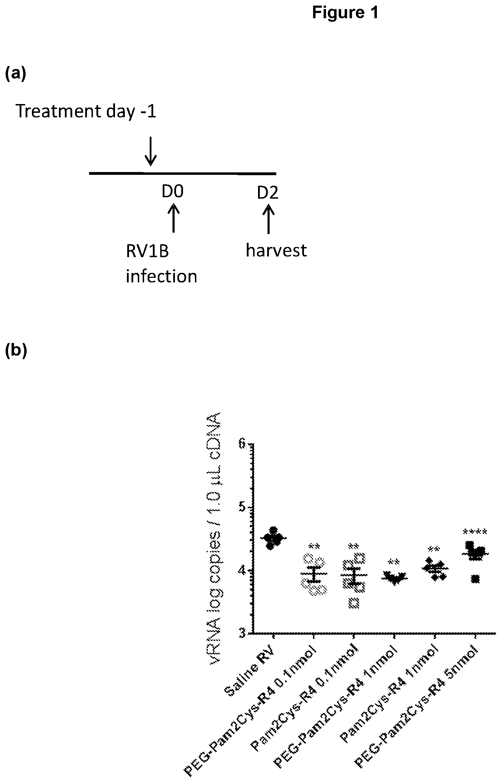

**Figure** 1: **Treatment with high dose TLR-2 agonist reduces viral RNA at 2 days post infection.** (a) Schematic showing treatment regime with representative TLR-2 agonists PEG-Pam2Cys-R4 or Pam2Cys-R4. (b) Quantification of viral RNA showing that representative TLR-2 agonists PEG-Pam2Cys-R4 and Pam2Cys-R4 reduce viral RNA at the indicated doses in RV infected mice. Lungs were collected at days 2 post infection and total RNA extracted and viral RNA measured by qPCR. Mean +/- SEM ****p<0.0001, **p<0.01 reduced viral RNA compared to saline treated RV infected mice, as assessed by One Way ANOVA.

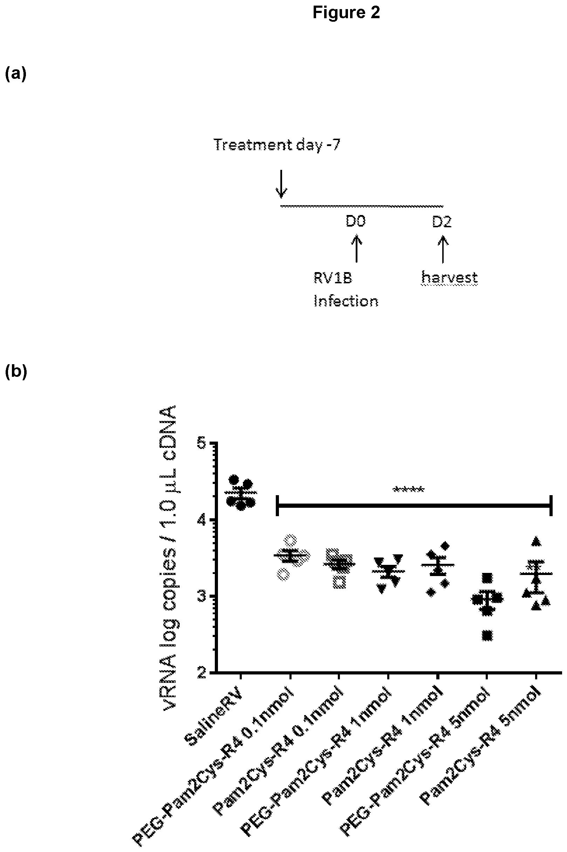

**Figure 2****: Potent anti-viral effect of with high dose TLR-2 agonist treatment 7 days before infection of mice with RV. (a)** Schematic showing treatment regime with representative TLR-2 agonists PEG-Pam2Cys-R4 or Pam2Cys-R4. (b) Agonist treatment with all doses resulted in highly significant reduction in viral load compared to saline treated, RV infected controls in the presence of representative TLR-2 agonists PEG-Pam2Cys-R4 or Pam2Cys-R4. Viral RNA in lung was measured by qPCR two days after infection. ****p<0.0001 reduced viral RNA compared to saline treated RV infected mice, as measured by One Way ANOVA.

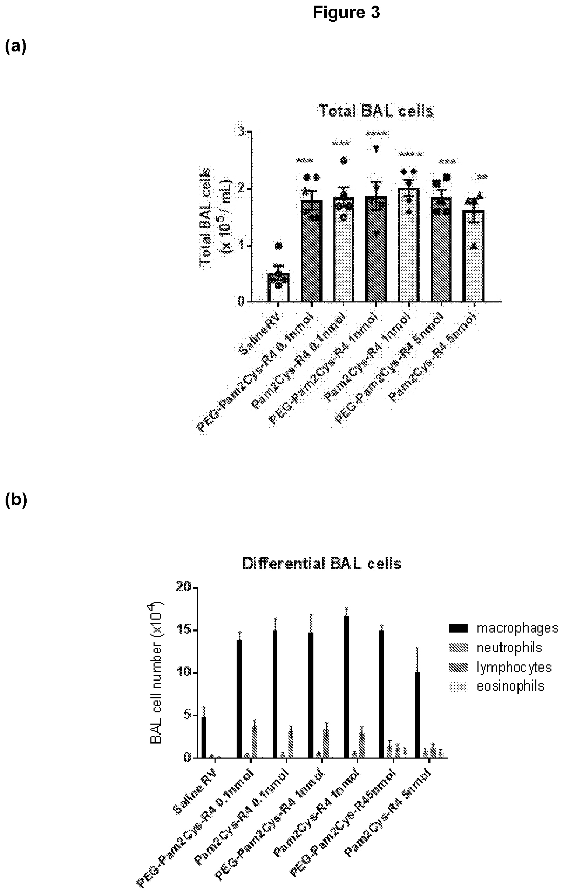

**Figure 3****: Airway cellular inflammation expression with high dose TLR-2 agonist treatment 7 days before infection of mice with RV. (a-b)** Analysis of bronchoalveolar lavage (BAL) cells at day 2 post-infection indicated that all treatments significantly increased the total number of immune cells, the majority of which were macrophages. Increased numbers of lymphocytes were observed at lower agonist treatment doses. Inflammatory cells in BAL were counted and populations identified by differential staining at 2 days post-infection. Mean +/- SEM **p<0.01, ***p<0.001, ****p<0.0001 increased BAL cells in treated groups compared to saline treated RV infected mice.

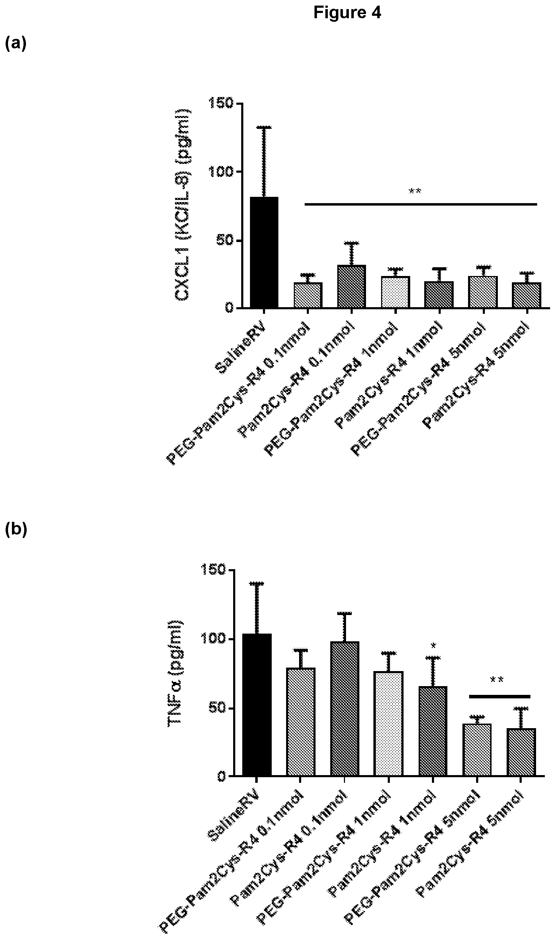

**Figure 4****: High dose TLR-2 agonist treatment seven days prior to RV infection suppresses expression of inflammatory cytokines.** Inflammatory cytokines in bronchoalveolar lavage (BAL) were measured by ELISA. (a) Significantly reduced production of the neutrophil recruiting chemokine CXCL1 was observed for all treatment groups compared to saline treated RV infected mice. (b) Reduced expression of TNFα was also observed for the higher doses of agonist treatment groups compared to saline treated RV infected mice, as well as in response to 1nmol Pam2Cys-R4. Mean +/- SEM *p<0.05, **p<0.01 reduced protein level compared to saline treated RV infected mice, as assessed by One Way ANOVA.

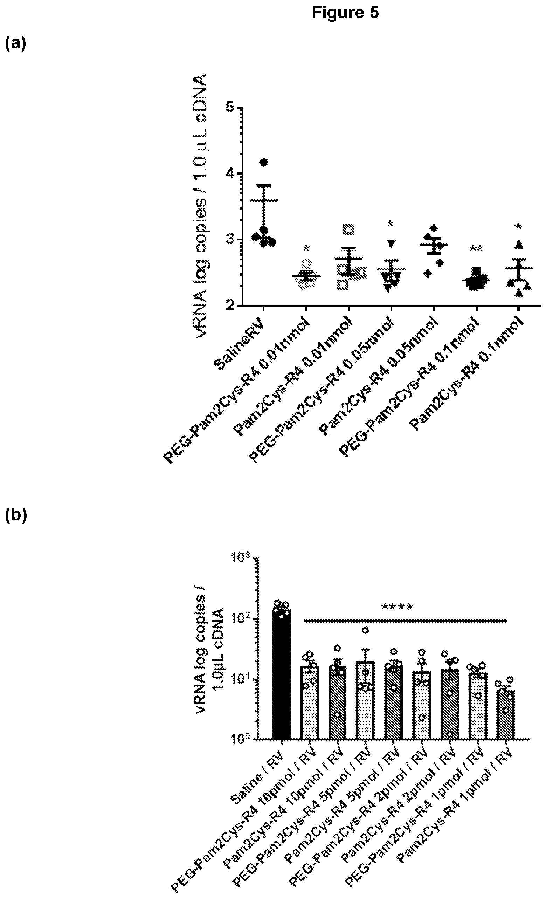

**Figure 5****: Low dose PEG-Pam2Cys-R4 treatment reduces viral load.** (a, b) All doses of PEG-Pam2Cys-R4 significantly inhibited RV replication. The indicated doses of Pam2Cys-R4 also caused a significant reduction in viral RNA compared to untreated saline RV infected controls. Viral RNA in lung tissue at 2 days post-infection was assessed by qPCR. Mean +/- SEM *p<0.05, **p<0.01, as assessed by One way ANOVA.

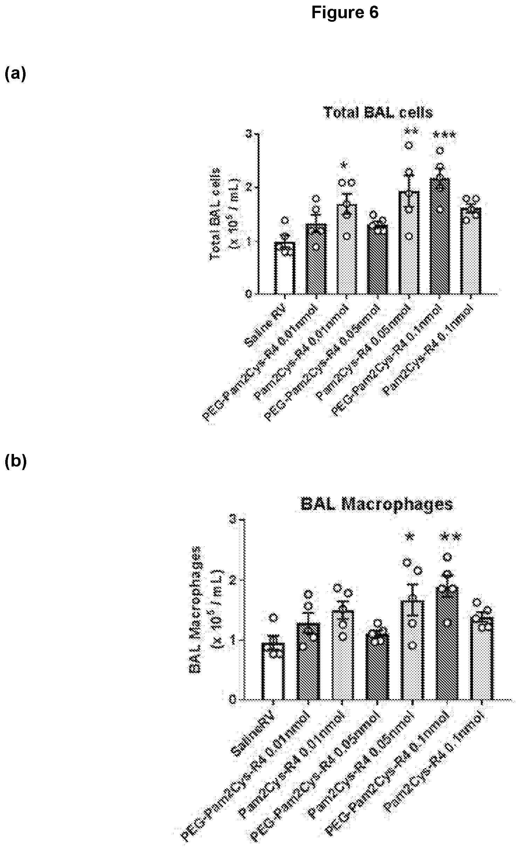

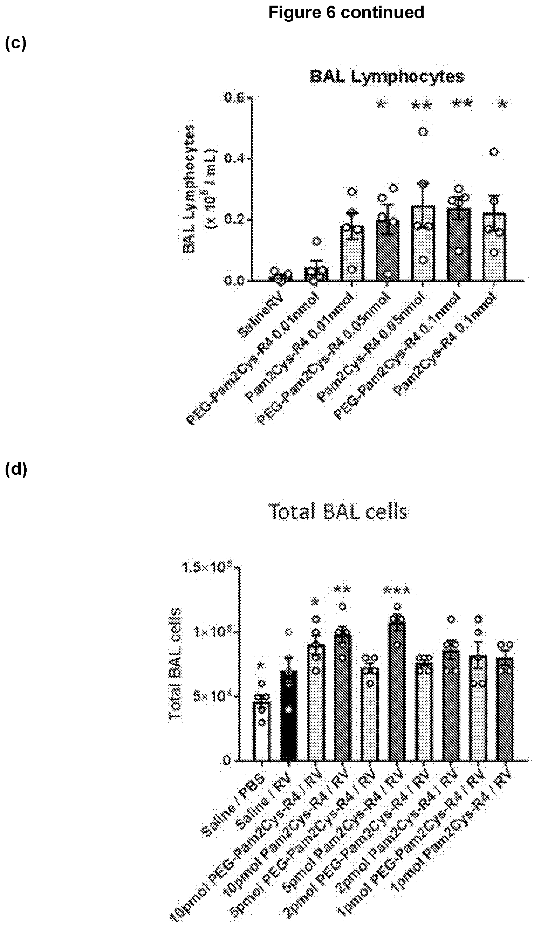

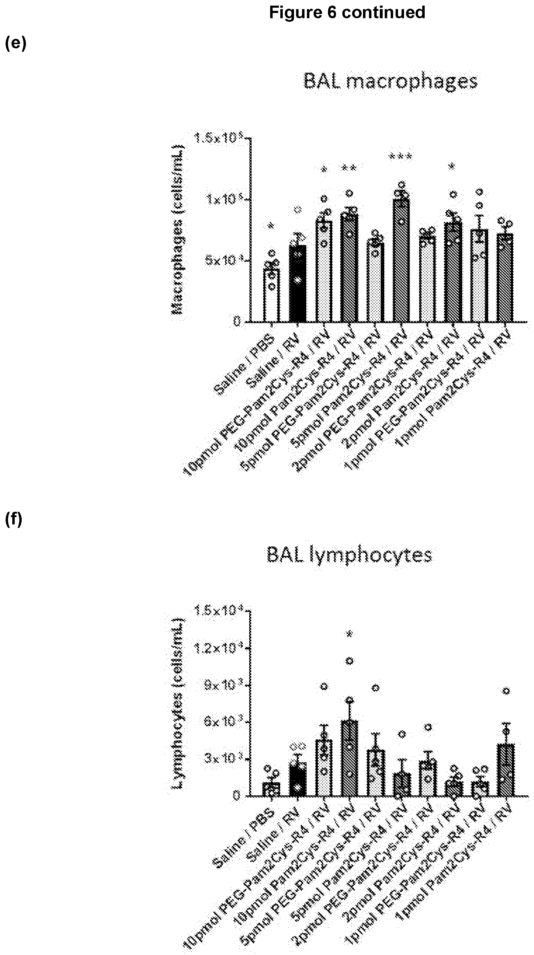

**Figure 6****: Increased BAL macrophages and lymphocytes with low dose TLR-2 agonist treatment, (a, d)** Pam2Cys-R4 caused significant increases in numbers of immune cells following treatment with the indicated doses as compared to untreated saline RV infected controls. **(b, e)** Increased BAL cells were primarily driven by increased macrophage numbers. **(c, f)** Significant increases in numbers of lymphocyte numbers was also observed at the indicated doses. Cells were stained and counted at 2 days post-infection. Mean +/- SEM *p<0.05, **p<0.01, ***p<0.001, as assessed by One Way ANOVA.

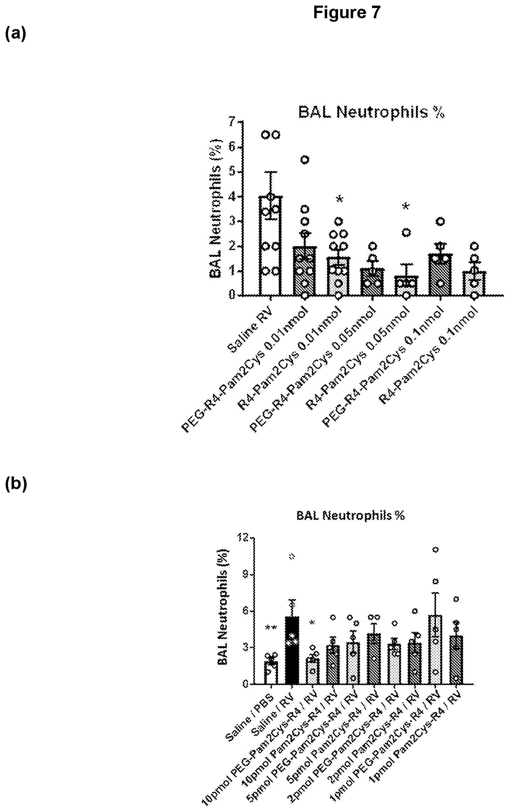

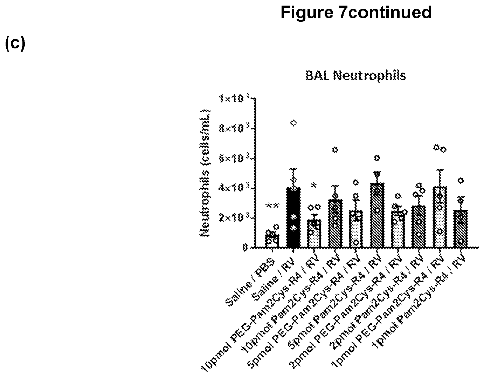

**Figure 7****: Low dose TLR-2 agonist treatment reduces viral neutrophilic inflammation, (a-b)** A significant reduction in neutrophils expressed as a percentage of total BAL cells or total neutrophil number was also observed at the indicated doses. Neutrophils were identified by differential staining and expressed as a percentage of total BAL cells at 2 days post-infection. **(c)** Compared to saline treated RV infected mice, significant reductions to neutrophil number at the indicated doses were observed when expressed as absolute number of total BAL cells. Mean +/- SEM, * = P<0.05.

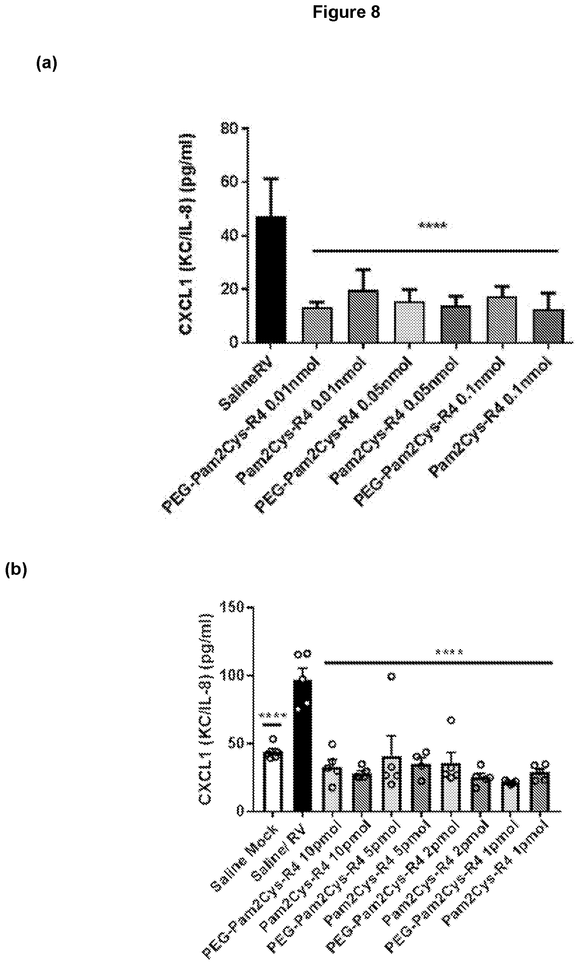

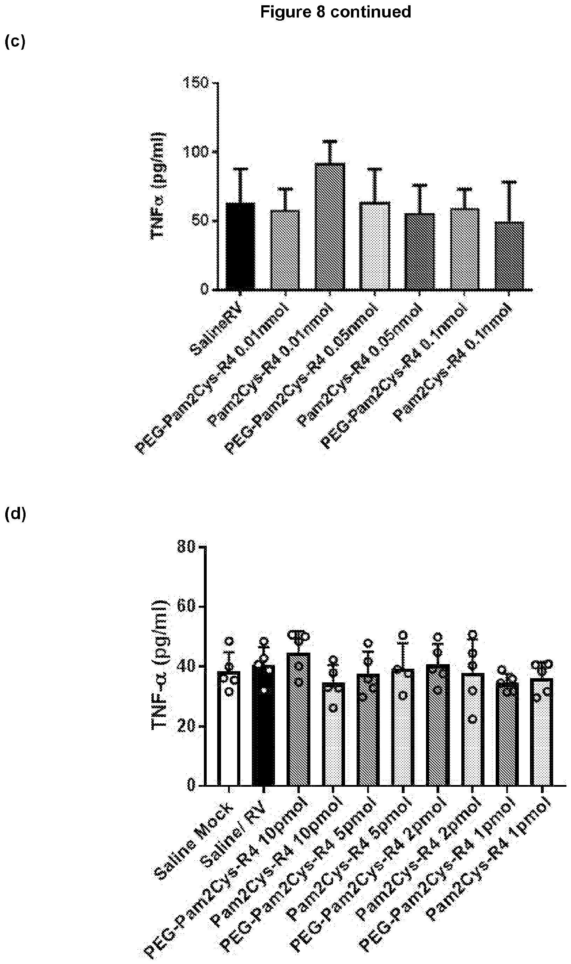

**Figure 8****: Low dose TLR-2 agonist treatment causes a highly significant reduction of neutrophil chemokine CXCL1. (a, b)** A highly significant reduction in CXCL1 expression was observed in response to all doses of Pam2Cys-R4 and PEG-Pam2Cys-R4 compared to untreated saline RV infected controls. **(c, d)** Treatment had no effect on TNFα production. Protein mediators in BAL were measured by ELISA 2 days post-infection. Mean +/- SEM ****p<0.0001, as assessed by One Way ANOVA. Multiple comparisons assessed by Holm-Sidak test.

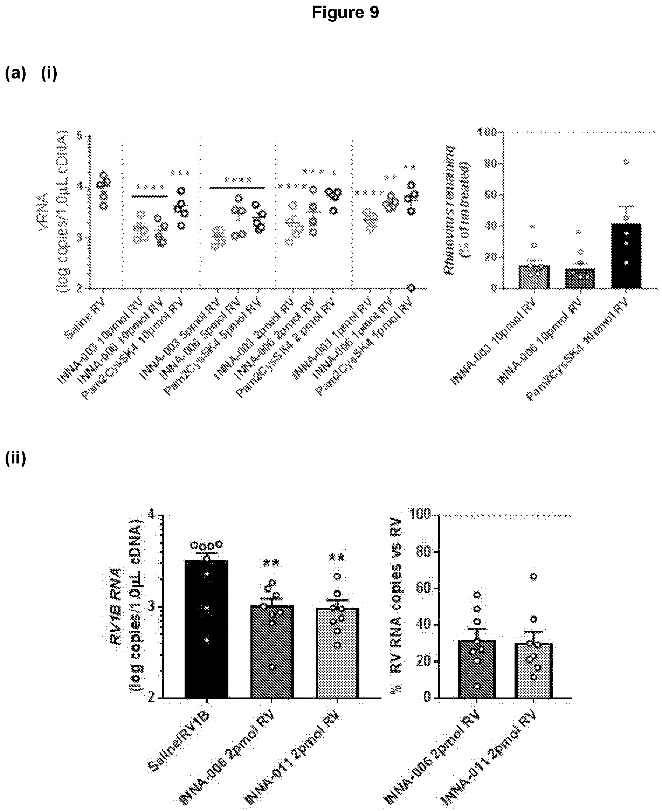

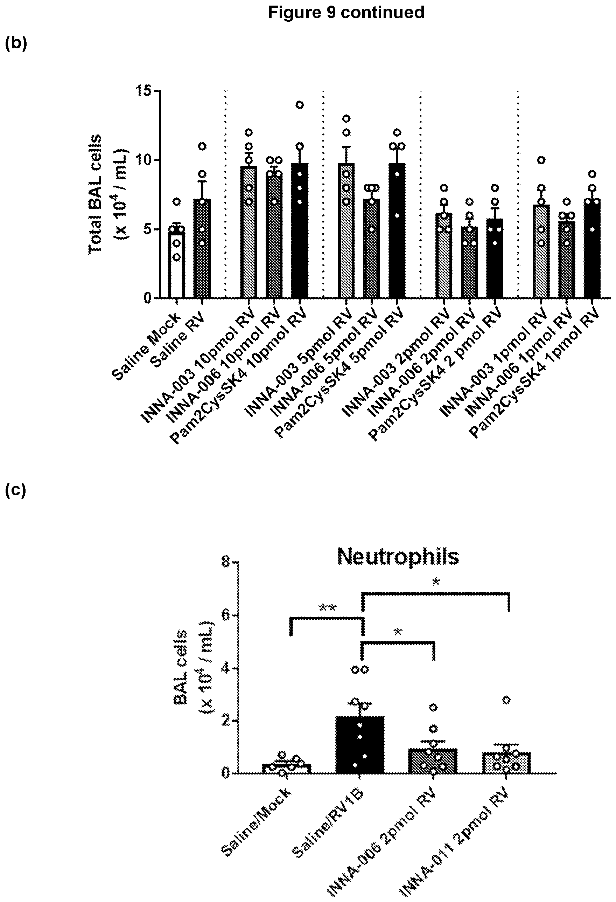

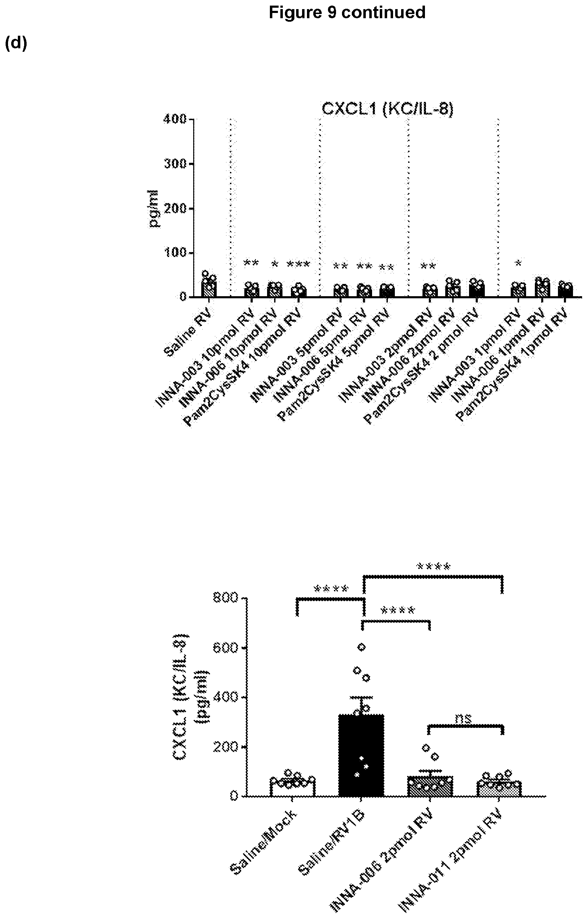

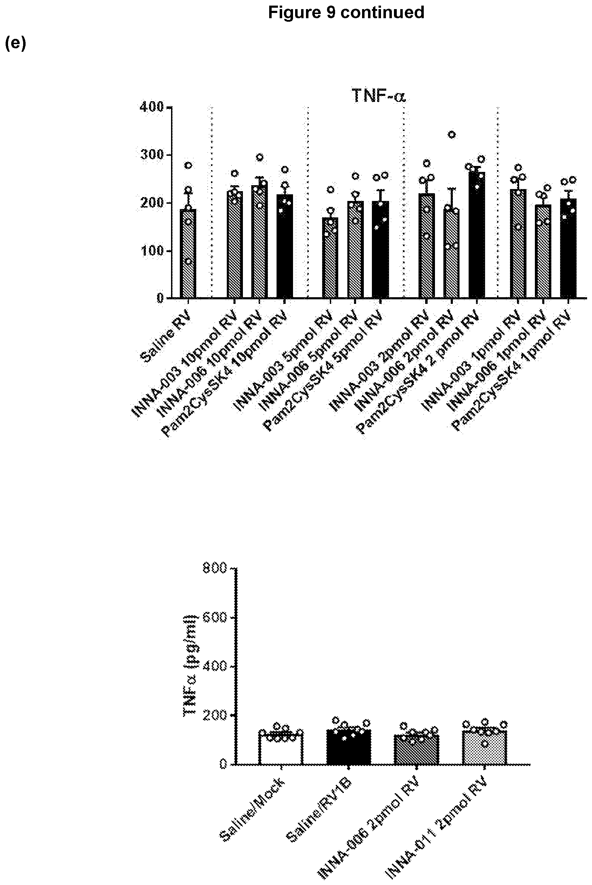

**Figure 9****: Comparison of treatment of (i) Peg-SS-Pam2Cys, Peg-S-Pam2Cys and Pam2CysSK4; and (ii) INNA-011 and Peg-S-Pam2Cys (INNA-006) 7 days before infection (dose range 1pmol- 10pmol) (a)** TLR2 agonist treatment causes a highly significant reduction of RV1B copy numbers in the lung. Viral RNA in lung tissue at day 2 p.i. was assessed by qPCR. Mean +/- SEM *p<0.05, **p<0.01, ***p=0.001, ****p<0.0001 reduced viral RNA compared to untreated (saline) RV infected controls (vRNA copy numbers), 10pmol Peg-SS-Pam2Cys and Peg-S-Pam2Cys (Rhinovirus reduction panel), or 2 pmol INNA-011, when assessed by 1 way ANOVA. (b) BAL leukocytes are not significantly increased by TLR-agonist treatment. Total BAL leukocytes assessed by trypan blue exclusion dye on a haemocytometer 2 days post-infection. Mean +/- SEM with 1 way ANOVA analysis. **(c)** Inflammatory cell analysis indicated that Peg-SPam2Cys and INNA-011 reduced RV-induced BAL neutrophilic inflammation, when assessed by 1 way ANOVA. Cells were differentially stained and counted by light microscopy. *p<0.05, **p<0.01, ****p<0.001 significantly different cell numbers compared to Saline/RV1B. **(d-e)** Treatment with TLR-agonist reduces BAL CXCL1 but do not change levels of TNF-α. Protein mediators in BAL were measured by ELISA at day 2 p.i. Mean +/- SEM *p<0.05, **p<0.01, ***p<0.001, ****p<0.0001 reduced CXCL1 compared to Saline RV group by 1 way ANOVA.

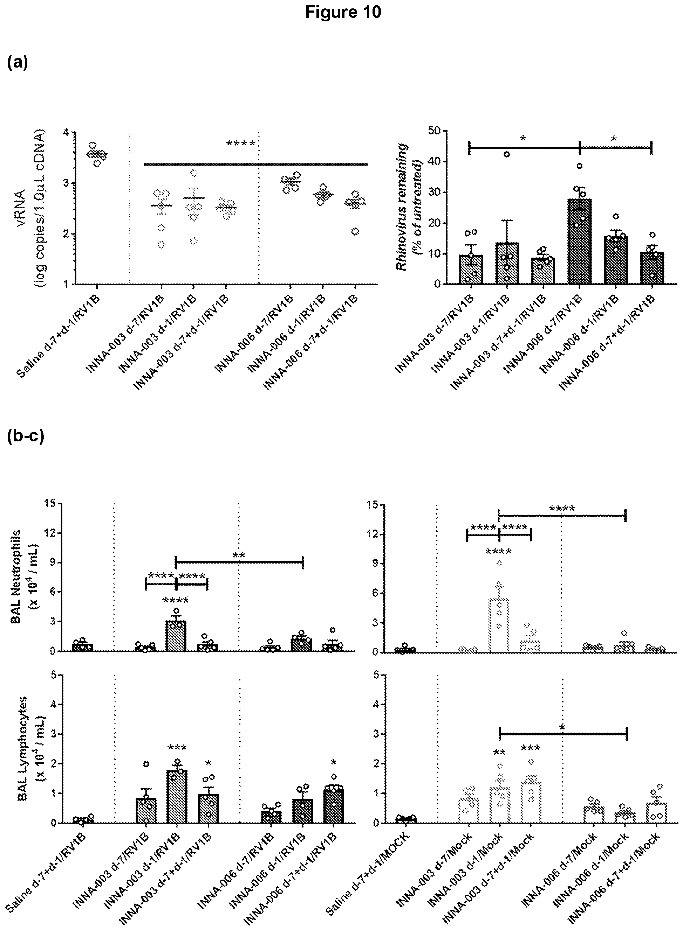

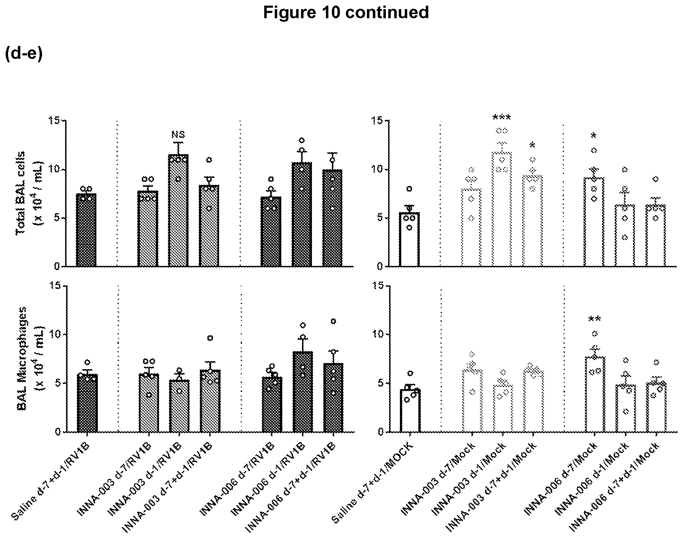

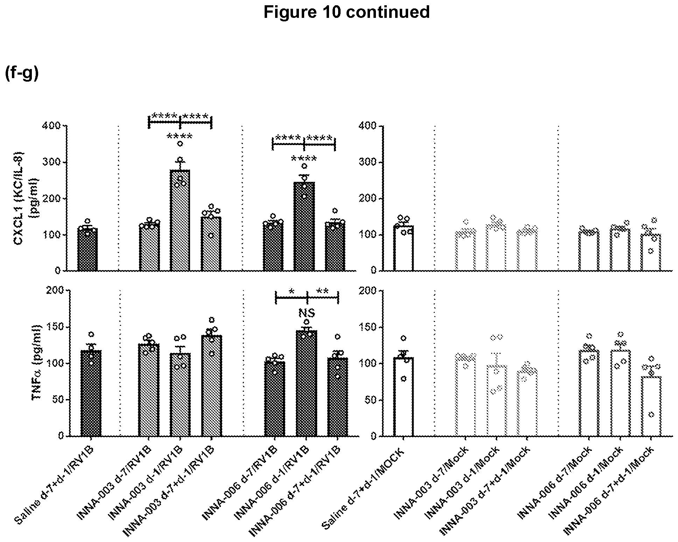

**Figure 10****: Combined drug timing interaction and effect upon infection (a-b)** TLR2 agonist treatment, at different time points and combinations of administration times, causes a highly significant reduction of RV1B copy numbers in the lung. Viral RNA in lung tissue at day 2 p.i. was assessed by qPCR. Mean +/- SEM *p<0.05, ****p<0.0001 reduced viral RNA compared to untreated (saline) RV1B infected controls (unless indicated otherwise) by 1 way ANOVA. **(c-d)** BAL neutrophils and lymphocytes are significantly increased by TLR2-agonist treatment. Differential staining of BAL cells 2 days post-infection. Mean +/- SEM #p<0.05, ###p<0.001, ####p<0.0001 compared to Saline d-7+d-1/MOCK. *p<0.05, **p<0.01, ***p<0.001, ****p<0.0001 significantly different cell number compared to Saline d-7+d-1/RV1B group (unless otherwise indicated) by 1 way ANOVA. **(e-f)** BAL leukocytes are significantly increased by TLR2-agonist treatment. Total BAL leukocytes assessed by trypan blue exclusion dye on a haemocytometer and BAL macrophages assessed by differential cell count 2 days post-infection. Mean +/- SEM, *p<0.05, **p<0.01, ****p<0.0001 compare to Saline d-7+d-1/Mock by 1 way ANOVA analysis. **(g-h)** Treatment with TLR2-agonist on day -1 increases BAL CXCL1 in RV infected mice, but not with prior treatment on day-7. Protein mediators in BAL were measured by ELISA at day 2 p.i. Mean +/- SEM ****p<0.0001 compared to Saline RV group unless shown otherwise determined by 1 way ANOVA.

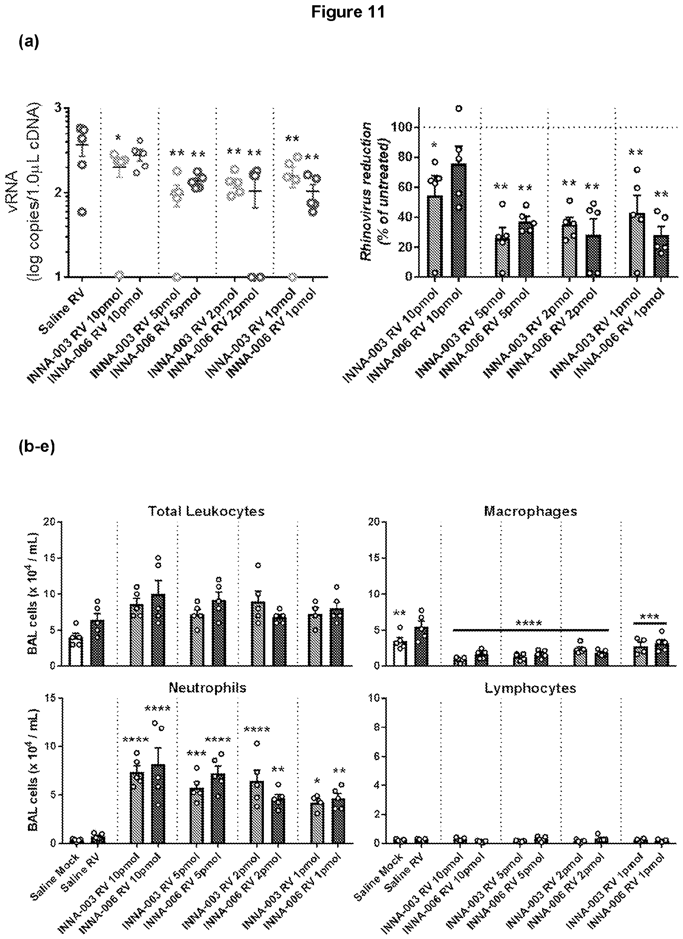

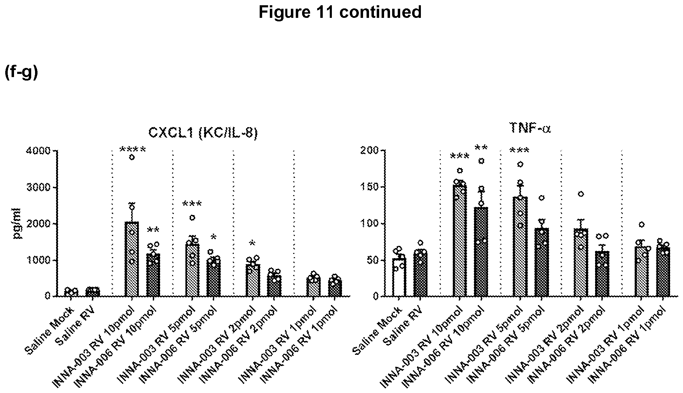

**Figure 11****: Study 1G Treatment during RV infection (a)** Peg-SS-Pam2Cys and Peg-SPam2Cys treatment during established infection reduces RV1B copy numbers in the lung. Mice were infected intranasally with RV1B and Peg-SS-Pam2Cys and Peg-S-Pam2Cys were administered intranasally on the following day. Viral RNA in lung tissue at day 2 p.i. was assessed by qPCR. Mean +/- SEM *p<0.05, **p<0.01 reduced viral RNA compared to untreated (saline) RV1B infected. **(b-e)** Peg-SS-Pam2Cys or Peg-S-Pam2Cys treatment during active infection (day 1 post infection) drastically increases neutrophil numbers in BAL. Differential staining of BAL cells 2 days post-infection. Results are graphed as Mean +/- SEM. ***p<0.001, ****p<0.0001 significantly different cell number compared to Saline RV group by 1 way ANOVA. **(f-g)** Treatment with Peg-SS-Pam2Cys or Peg-S-Pam2Cys on day 1 post infection causes dose-dependent inflammatory cytokine production. Protein mediators in BAL were measured by ELISA at day 2 p.i. Results are graphed as Mean +/- SEM. *p<0.05, **p<0.01 ***p<0.001, ****p<0.0001 compared to Saline RV group by 1 way ANOVA.

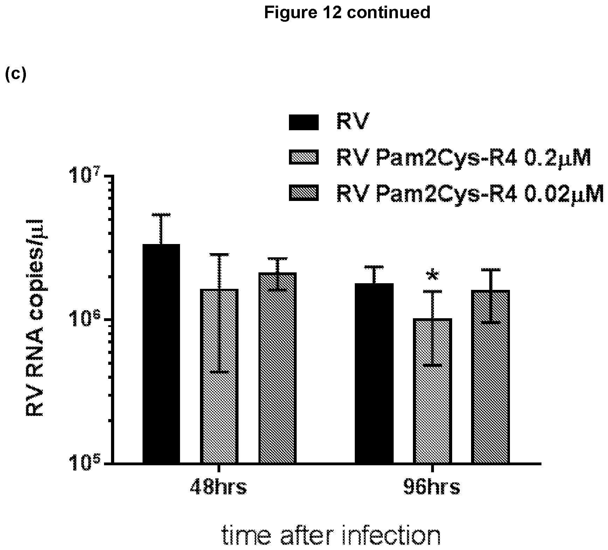

**Figure 12****: TLR-2 agonist treatment reduces the level of rhinovirus replication in asthmatic epithelial cells. (a)** Patient profile of subjects with either mild persistent or moderate persistent asthma. Asthmatic epithelial air liquid interface (ALI) cultures were prepared from the bronchial epithelial cells from these asthmatic donors and were infected with rhinovirus (RV) and treated with Pam2Cys-R4 **(b)** 24 hours before RV infection (pre-treatment) where Pam2Cys-R4 significantly reduced viral load after 96 hours at 0.02µM; or **(c)** 2 hours after RV infection (post-treatment) where Pam2Cys-R4 significantly reduced viral load after 96 hours at 0.2µM. Total cell RNA was purified and the level of viral RNA was measured by qRT-PCR at 48 hours and 96 hours post-infection. Mean +/-SEM *=p<0.05 compared to RV group, as assessed by paired t test.

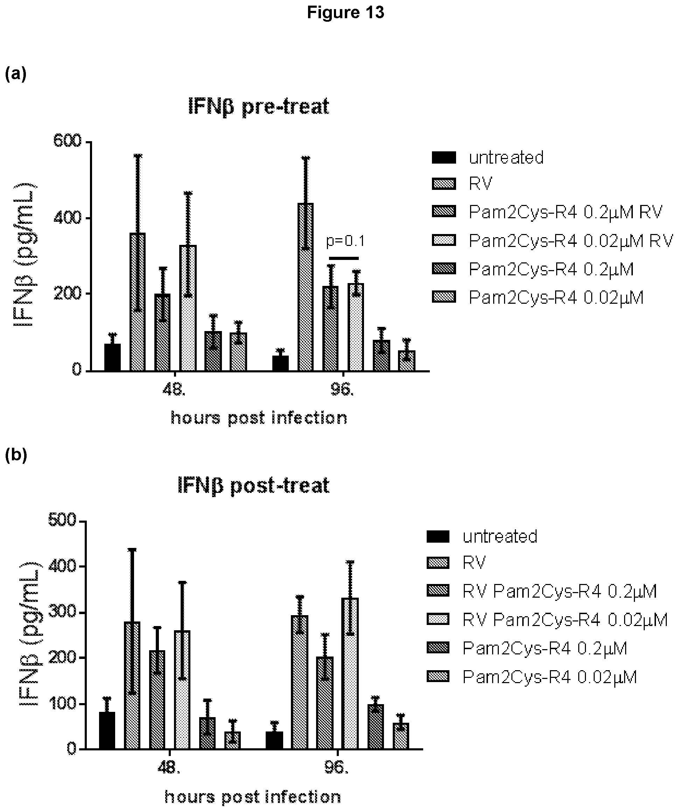

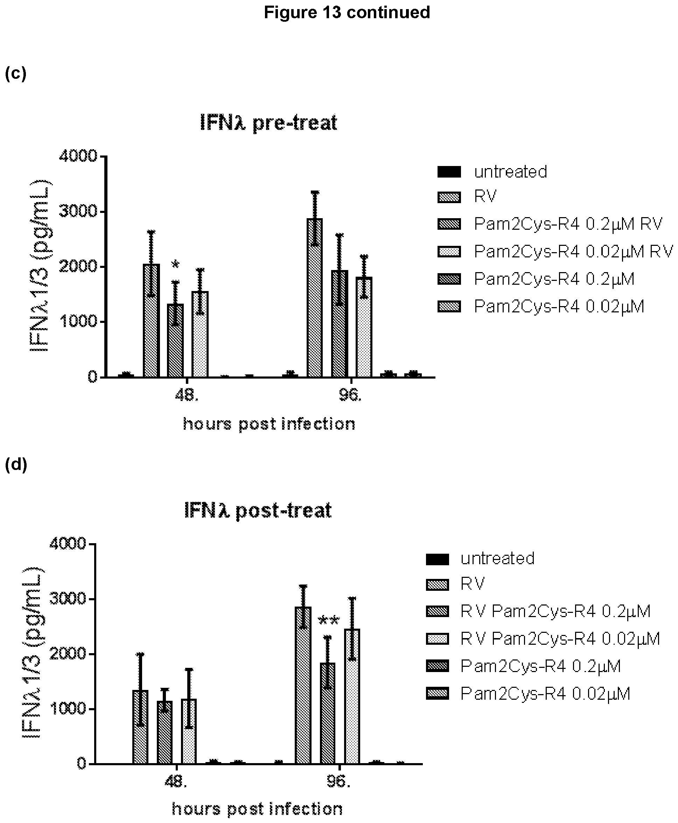

**Figure 13****: Reduced viral replication is associated with decreased production of interferon.** The level of IFNβ and IFNλ1/3 protein in apical media was measured by ELISA in (**a**, **b**) n=5 (IFNβ) or (**c**, **d**) n=6 (IFNλ) asthmatic epithelial air liquid interface (ALI) cultures infected with rhinovirus (RV) and treated with Pam2Cys-R4 either before (pre-treat) or after (post-treat) at the indicated concentrations. Data mean +/- SEM. All p values are compared to RV infection alone for indicated time point. *p<0.05, **p<0.01 , as assessed by the Friedman test.

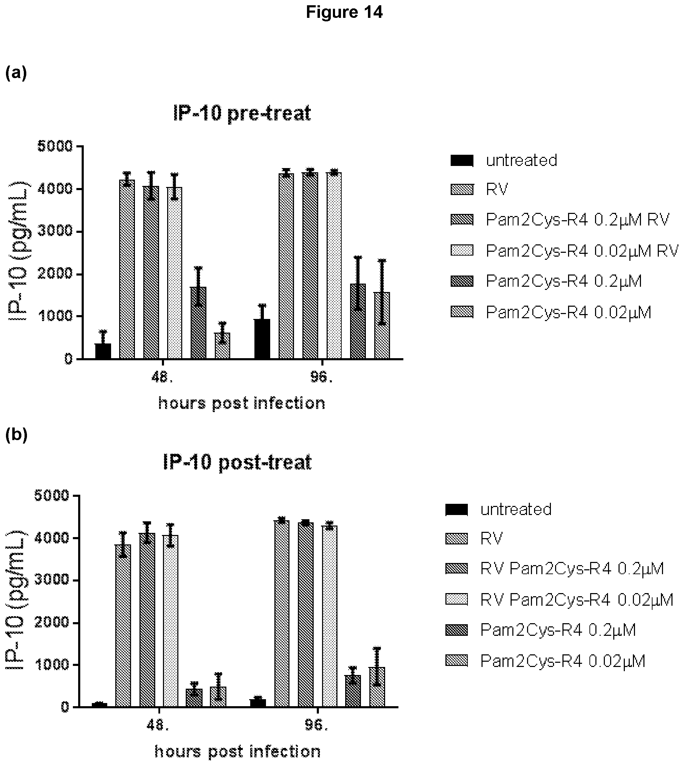

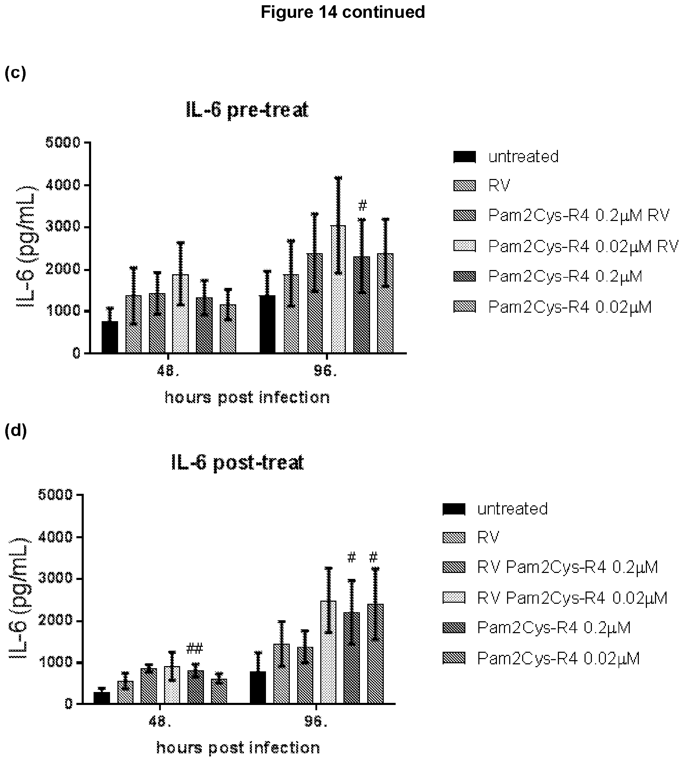

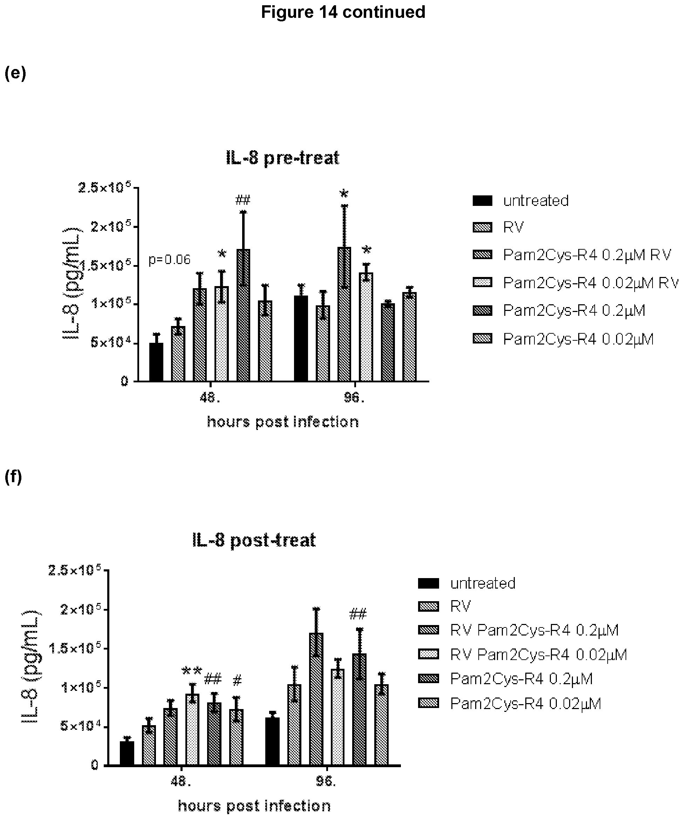

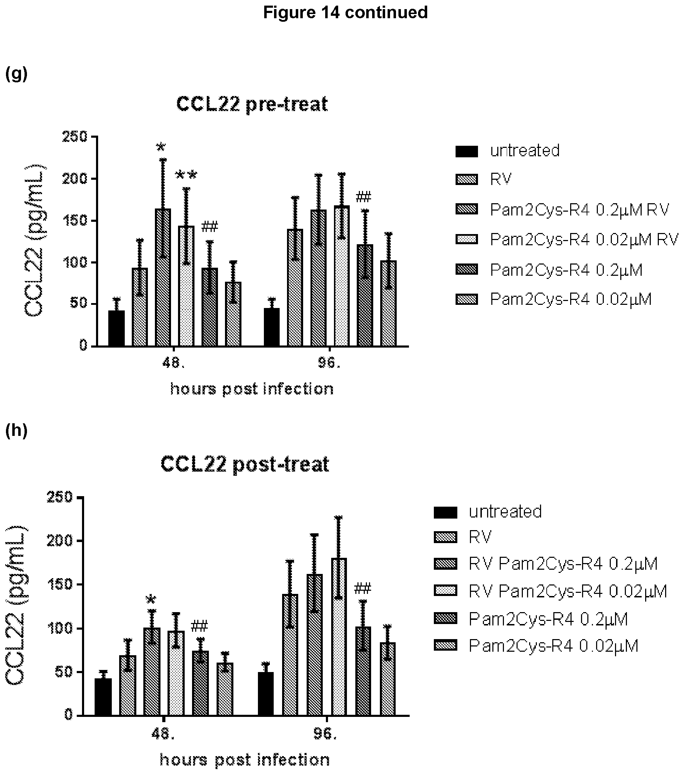

**Figure 14****: TLR-2 agonist can increase expression of pro-inflammatory mediators.** The level of (**a**, **b**) IP-10 (CXCL10), (**c**, **d**) IL-6, (**e**, **f**) IL-8 and (**g**, **h**) CCL22 protein expressed as mean +/- SEM of n=6 asthmatic epithelial cultures, measured by ELISA. *p<0.05, **p<0.01 increased mediator expression by Pam2Cys-R4 treated RV infected cells compared to untreated RV infected cells; ^{#}p<0.05, ^{##}p<0.01 increased mediator expression in Pam2Cys-R4 treated cells compared to untreated cells, as assessed using the Friedman test.

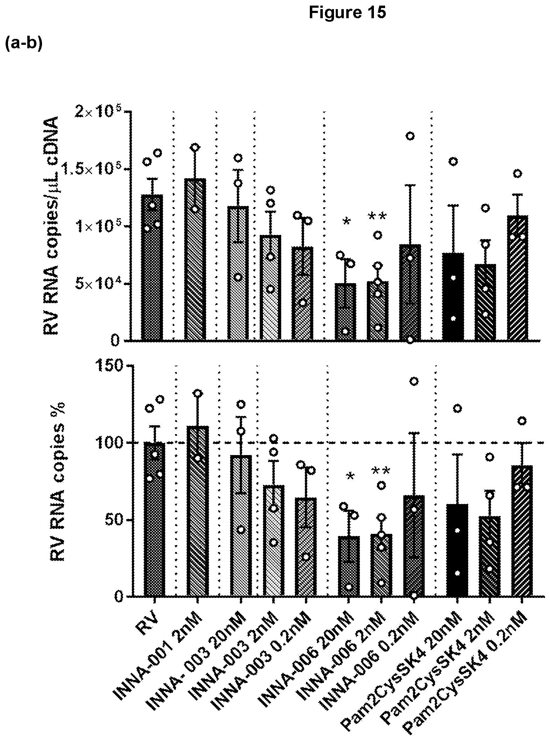

**Figure 15****: Anti-viral activity of TLR2 agonists and Pam2CSK4 (a-b)** BCi-NS1 cells were cultured at ALI to achieve differentiation. Cells were then pre-treated with Pam2Cys-R4 (INNA-001), Peg-SS-Pam2Cys (INNA-003), Peg-S-Pam2Cys (INNA-006) or Pam2CSK4 at concentrations ranging from 20 nM to 0.2 nM. At 24 h post-treatment cells were infected with RV1B at moi 0.1. and harvested at 96 h post-infection. Total RNA was extracted and reverse transcribed to cDNA using random hexamer primers. Viral load was assessed by qPCR and expressed as copy number and percentage of viral RNA in RV (untreated) wells. *p<0.05, **p<0.01 reduced viral RNA compared to RV (untreated) group. n=2-5 replicate wells.

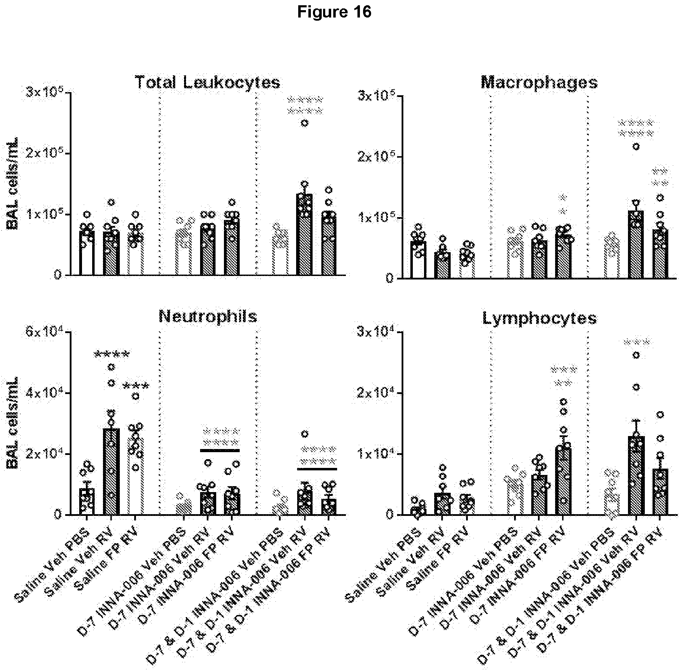

**Figure 16****: INNA-006 prevented RV-induced and steroid resistant neutrophilic inflammation.** Cells were differentially stained and counted by light microscopy. Mean +/- SEM *p<0.05, **p<0.01, ***p<0.001, ****p<0.0001 increased BAL cells compared to compared to Saline Veh PBS (single line asterisks), Saline Veh RV (bottom of double layer asterisks) or Saline FP RV (top of double layer asterisks) RV. 1 way ANOVA.

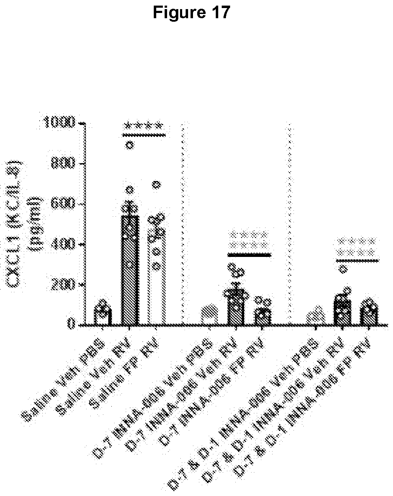

**Figure 17****: RV-induced, steroid resistant neutrophil chemokine production supressed by INNA-006.** CXCL1 protein levels in BAL were measured by ELISA at day 2 p.i. ****p<0.0001 increased mediator compared to Saline Veh PBS (black asterisks), Saline Veh RV **(red asterisks),** Saline FP RV **(blue asterisks).** 1 way ANOVA.

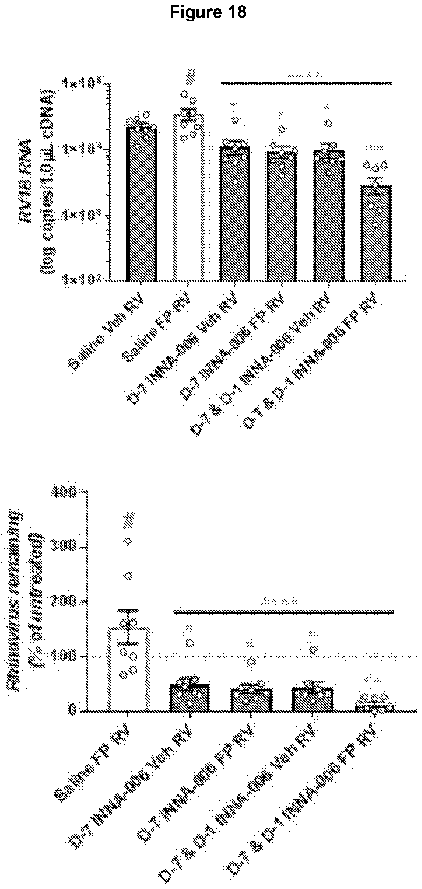

**Figure 18****: Viral lung load was increased by FP treatment in vehicle control mice, but antiviral efficacy of repeated INNA-006 treatment was enhanced by FP.** Lungs were collected at day 2 p.i., total RNA extracted and viral RNA measured by qPCR. Mean +/- SEM *p<0.05, **p<0.01, ****p<0.0001 increased BAL cells compared to Saline Veh RV (single or double asterisks) or Saline FP RV (above line asterisks). #p<0.05 increased viral load compared to Saline Veh RV group. 1 way ANOVA.

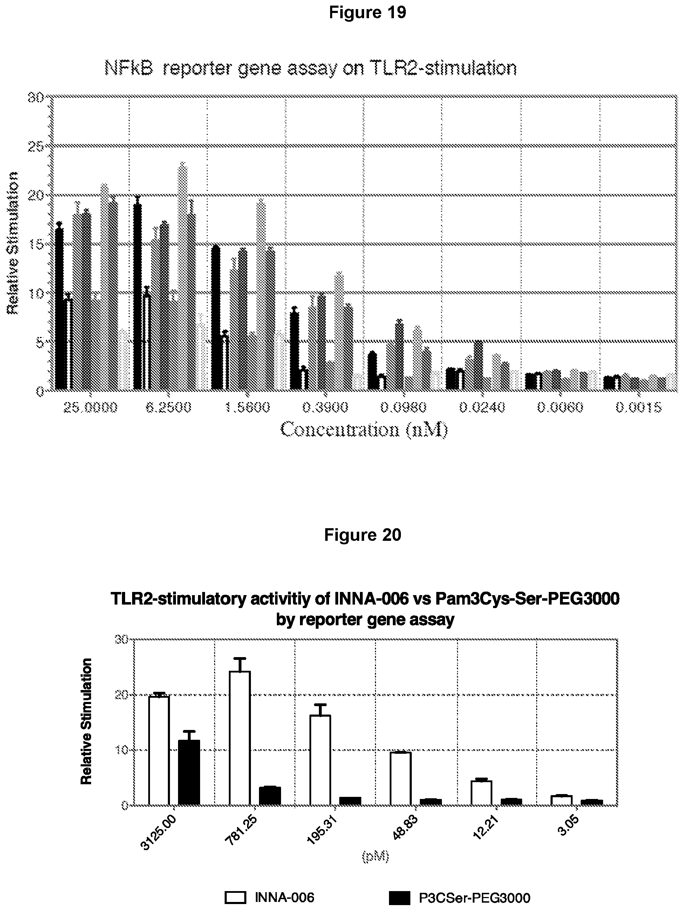

**Figure 19****. Comparison of the abilities of various compounds to stimulate luciferase activity in an NF-κB cell-based reporter system.** Columns left to right are: INNA-006 (or compound (1)); INNA-013 (or compound (4)); INNA-014 (or compound (3)); INNA-015 (or compound (2)); INNA-010; INNA-011 (or compound (5)); INNA-012 (or compound (6)); and INNA-009.
**Figure 20****: Comparison of the abilities of INNA-006 or Pam3Cys-Ser-PEG3000 to stimulate luciferase activity in an NF-κB cell-based reporter system.**

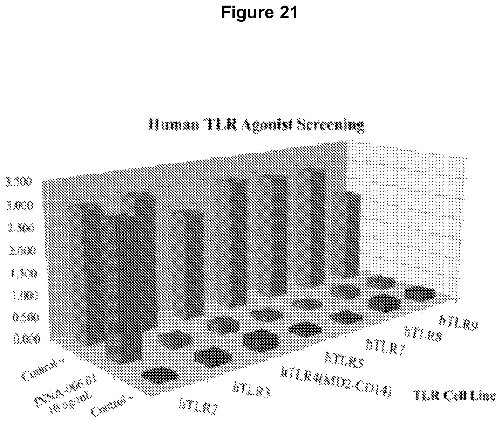

**Figure 21****: Representative data indicating specific TLR-2 activation by INNA-006.**

### Detailed description of the embodiments

Reference will now be made in detail to certain embodiments of the invention. While the invention will be described in conjunction with the embodiments, it will be understood that the intention is not to limit the invention to those embodiments. The scope of the present invention is defined by the claims.

For purposes of interpreting this specification, terms used in the singular will also include the plural and vice versa.

Viral respiratory infections are the most important trigger for respiratory exacerbations, for example asthma exacerbations. Asthmatics are susceptible to the more serious effects of viruses that usually cause the common cold such as rhinovirus (RV). Viral replication in the airway epithelium leads to production of inflammatory mediators that can trigger the immune cascade that underpins an asthma exacerbation. The inventors hypothesised that activation of innate epithelial immunity and/or other intracellular cellular signalling mechanisms by the administration of an effective amount of a TLR2 agonist will suppress RV replication and associated production of inflammatory mediators. The inventors firstly tested this hypothesis by administering a number of different doses of TLR2 agonists prior to treatment with RV in an *in vivo* model of RV infection. This was assessed by measuring parameters including weight loss, viral load and the expression of inflammatory mediators. In this study, the inventors found that the administration of TLR2 agonists did not induce weight loss, but reduced lung viral load and reduced viral-induced inflammation.

The inventors further tested the above hypothesis in a therapeutic model of ex *vivo* air liquid interface (ALI) cultures from bronchial epithelium of asthma sufferers. In this model, administration of TLR2 agonists occurred either before or after infection of the epithelium with RV. The inventors found that stimulation with TLR2 agonists reduced viral load in the asthmatic bronchial epithelium.

An advantage of an aspect of the invention is the surprising finding that treatment with a TLR2 agonist at the time of established RV infection leads to an inhibition of RV infection. The invention therefore finds particular application for subjects that are diagnosed with a respiratory infection and whom have been clinically diagnosed with a respiratory condition, such as asthma, and/or are prone to respiratory exacerbations. Another advantage of an aspect of the invention is the surprising finding that treatment with lower doses of TLR2 agonists were at least as effective, if not more so, than the tested higher doses of the TLR2 agonists. The invention therefore finds particular application where low levels of activation of the innate immune system are required or desirable. A further advantage of an aspect of the invention is the surprising finding that the TLR2 agonist PEG-Pam2Cys-R4 displayed superior anti-viral and anti-inflammatory effects in models of RV-mediated infection. Agonists with analogous functional features are therefore likely to display similar properties in inhibiting RV-mediated infection and therefore in preventing and/or treating asthma exacerbation. Yet another advantage of an aspect of the invention is the surprising finding that the anti-viral response described herein does not rely on IFN-mediated responses. This is significant because interferon expression is extremely variable, particularly in more severe forms of asthma, and a therapeutic mechanism that relies upon the regulation of IFN could be unreliable and thus problematic with no therapeutic effect or induction of excess inflammation.

Toll-Like Receptors (TLRs) are pattern recognition receptors (PRRs) expressed by diverse cell types that play an important role in both innate and adaptive immunity. Cells of the innate immune system respond to TLR activation by producing pro-inflammatory cytokines and chemokines that signal for the clearance of the pathogens and damaged-self. Upon engagement with specific ligands, TLR activation leads to the activation of transcription factors such as nuclear factor kappa B (NF)-kB, activating protein-1 (AP-1) and interferon regulatory factors (IRFs) through several adaptor molecules including myeloid differentiation primary response gene 88 (MyD88), Toll-interleukin 1 receptor (TIR) domain containing adaptor protein TIRAP and TIR-domain containing adaptor inducing interferon-beta TRIF, to regulate cytokine expression.

There are a number of TLRs that belong to this membrane receptor protein family including TLR1, TLR3, TLR4, TLR5, TLR6, TLR7, TLR8 and TLR9.

As used herein, the term "TLR2" is intended to mean Toll-Like Receptor 2 protein. In humans, TLR2 is encoded by the *TLR2* gene. TLR2 is expressed on the surface of certain cells and plays a fundamental role in pathogen recognition and activation of innate immunity.

A TLR2 agonist is an agent that binds Toll-like receptor 2. The TLR2 agonist may bind to, and activate, TLR2 as a homodimer or heterodimer.

In any embodiment of the invention, the TLR2 agonist comprises a lipopeptide or a lipid moiety selected from palmitoyl, myristoyl, and stearoyl. The TLR2 agonist may be selected from the group consisting of: Pam2Cys, Pam3Cys, and Ste2Cys. In a preferred embodiment, the TLR2 agonist comprises Pam2Cys.

An exemplary lipopeptide in accordance with any embodiment of the present invention is the lipopeptide "Pam2Cys". One of skill in the art would understand that the term "lipopeptide" means any composition of matter comprising one or more lipid moieties and one or more amino acid sequences that are conjugated. "Pam2Cys" (also known as dipalmitoyl-S-glyceryl-cysteine or S-[2, 3 bis(palmitoyloxy) propyl] cysteine has been synthesised and corresponds to the lipid moiety of MALP-2, a macrophage-activating lipopeptide isolated from *Mycoplasma fermentans.* Pam2Cys is known to be a ligand of TLR2.

Pam2Cys has the structure:

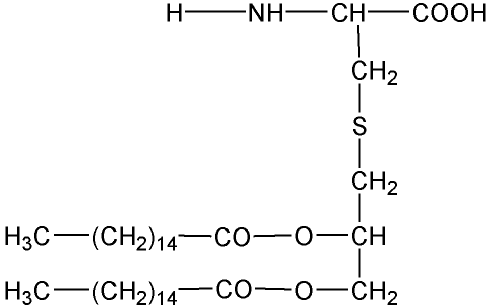

As used herein, reference to "S" as denoted in the above chemical structure defines a sulfur atom.

Another exemplary lipopeptide is the lipoamino acid N-palmitoyl-S-[2, 3-bis (palmitoyloxy) propyl] cysteine, also known as Pam3Cys or Pam3Cys-OH is a synthetic version of the N-terminal moiety of Braun's lipoprotein that spans the inner and outer membranes of Gram negative bacteria Pam3Cys has the following structure:

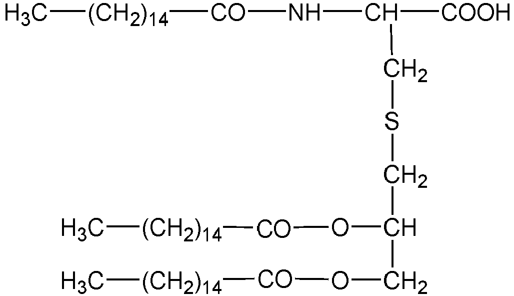

United States Patent No. 5,700,910 describes several N-acyl-S- (2-hydroxyalkyl) cysteines for use as intermediates in the preparation of lipopeptides that are used as synthetic adjuvants, B lymphocyte stimulants, macrophage stimulants, or synthetic vaccines. US 5,700,910 also teaches the use of such compounds as intermediates in the synthesis of Pam3Cys-OH and of lipopeptides that comprise this lipoamino acid or an analog thereof at the N-terminus.

Other lipid moieites which may be used to target cell surface TLRs are palmitoyl, myristoyl,or stearoyl.

In addition to Pam2Cys and Pam3Cys, the present invention also contemplates the use of Ste2Cys according to the present invention. Lau2Cys and Oct2Cys are described but not claimed. Those skilled in the art will be aware that Ste2Cys is also known as S-[2, 3-bis (stearoyloxy) propyl] cysteine or distearoyl-S-glyceryl-cysteine; that Lau2Cys is also known as S-[2, 3-bis (lauroyloxy) propyl] cysteine or dilauroyl-S-glyceryl-cysteine); and that Oct2Cys is also known as S-[2,3- bis (octanoyloxy) propyl] cysteine or dioctanoyl-S-glyceryl-cysteine).

Other suitable TLR2 agonists include, but are not limited to, synthetic triacylated and diacylated lipopeptides, FSL-1 (a synthetic lipoprotein derived from *Mycoplasma salivarium* 1), Pam3Cys (tripalmitoyl-S-glyceryl cysteine) and S-[2,3- bis(palmitoyloxy)-(2RS)-propyl]-N-palmitoyl-(R)-cysteine, where "Pam3" is "tripalmitoyl-S-glyceryl". Derivatives of Pam3Cys are also suitable TLR2 agonists, where derivatives include, but are not limited to: S-[2,3-bis(palmitoyloxy)-(2-R,S)-propyl]-N- palmitoyl-(R)-Cys-(S)-Ser-(Lys)4 -hydroxytrihydrochloride; Pam3Cys-Ser-Ser-Asn-Ala; Pam3Cys-Ser-(Lys)4; Pam3Cys-Ala-Gly; Pam3Cys-Ser-Gly; Pam3Cys-Ser; Pam3Cys-OMe; Pam3Cys-OH; PamCAG, palmitoyl-Cys((RS)-2,3-di(palmitoyloxy)-propyl)-Ala-Gly-OH, and the like.

Other non-limiting examples of suitable TLR2 agonists are Pam2CSK4 Pam2CysSK4 (dipalmitoyl-S-glyceryl cysteine-serine-(lysine)4; or Pam2Cys-Ser-(Lys)4) is a synthetic diacylated lipopeptide. Other synthetic TLRs agonists include those described, e.g., in Kellner et al. (1992) Biol. Chem. 373:1:51-5; Seifer et al. (1990) Biochem. J, 26:795-802; and Lee et al. (2003) J. Lipid Res., 44:479-486.

A TLR2 agonist for use according to the invention is linked to a solubilising moiety comprising a PEG to increase the solubility of the TLR2 agonist. As will be understood by persons skilled in the art, TLR2 agonists are typically non-polar and, accordingly, while being soluble in non-polar solvents, are only less soluble in polar and aqueous solvents.

Optionally, the solubilising agent further comprises a linear or branched peptide. Typically, the linear or branched peptide contains positively or negatively charged amino acids. Positively charged amino acids may be lysine, arginine, histidine, ornithine or combinations thereof. The branched or linear peptide may contain at least one lysine or arginine residue. Preferably, the charged amino acids are terminal, for example N-terminal. The branched peptides may have one of the following structures. 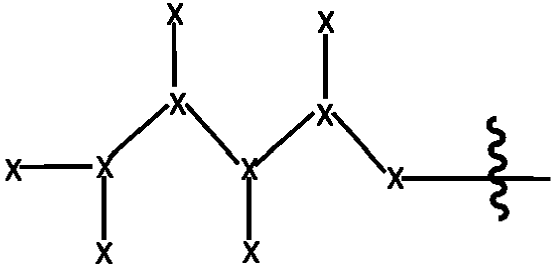 or

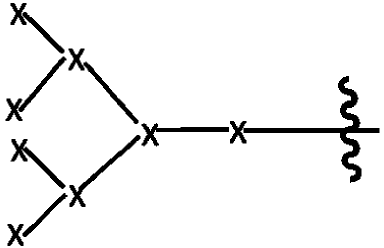

In the above structures, X may independently be a charged residue, either a positively or negatively charged residue. Preferably, the positively charged amino acids are lysine, arginine, histidine or ornithine. Preferably, the negatively charged amino acids are glutamate or aspartate.

As used herein, `PEG' refers to the polymer compound polyethylene glycol. Unless otherwise defined, reference to `PEG' includes any length polymer of ethylene oxide. Reference to PEG also includes substituted PEG.

The compound or functional group which can act as a solubilising agent comprises "PEG" (or polyethyleneglycol) and optionally one or more of a polar polypeptide such as "R4", a hyper-branched tetra arginine complex; "H4", a hyper-branched tetra histidine complex; "H8", a linear peptide containing histidine residues; and 'E8" a linear peptide containing glutamate residues. Other linear and branched lipid solubilising agents are also envisaged, including a hyper-branched peptide containing glutamate residues (see, e.g., "branched E8", below). R4, H4, H8 and E8 have been previously described in PCT/AU2009/000469 (WO/2010/115230) and have the following structures:

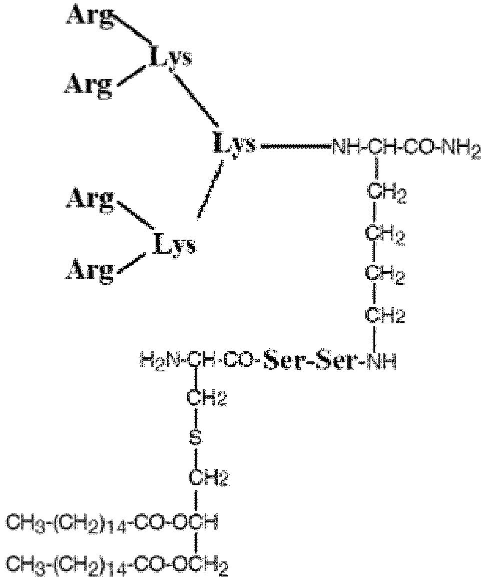

### Exemplary R4

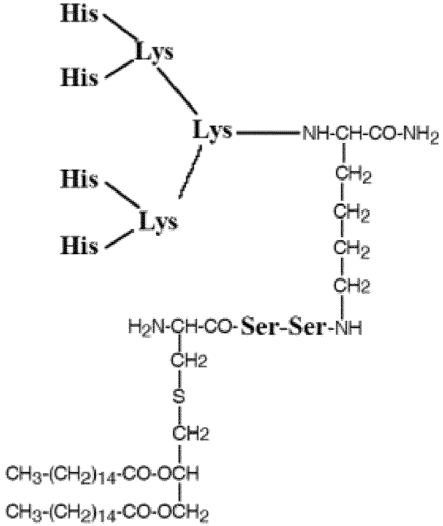

### Exemplary H4

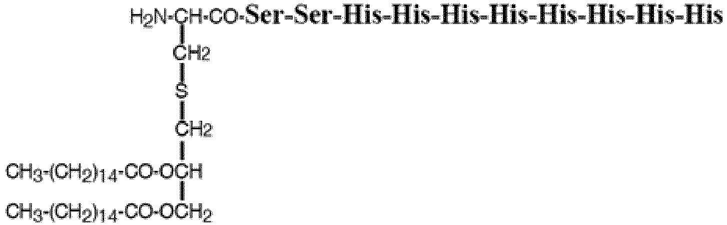

### Exemplary H8

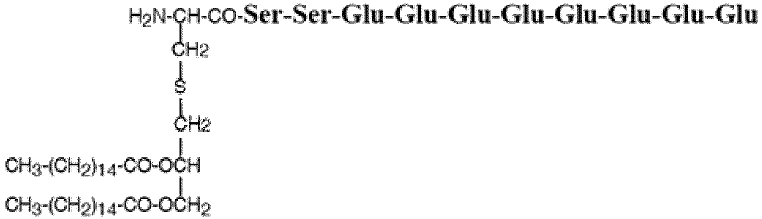

### Exemplary E8

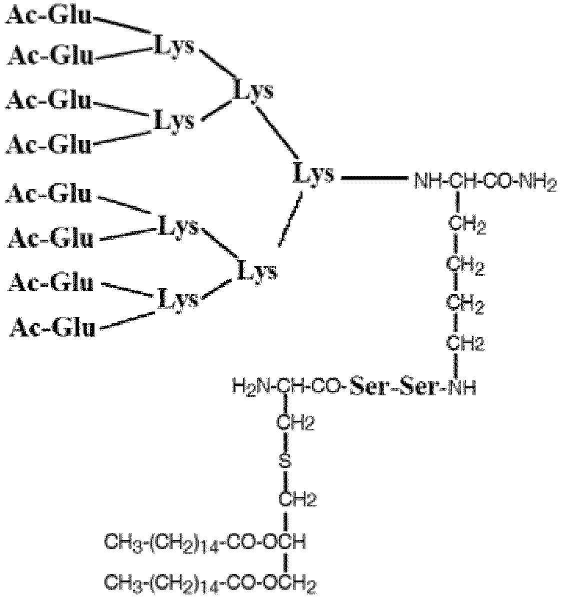

### Exemplary branched E8

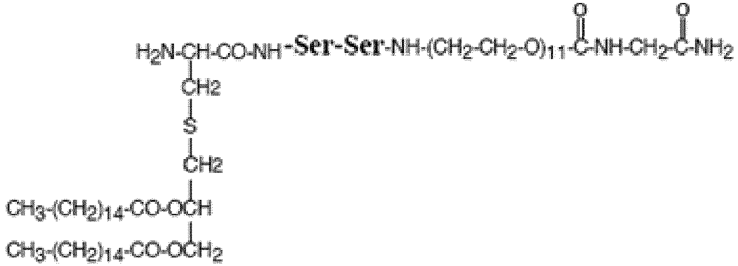

### Exemplary PEG

Following are schematic representations of some examples of branched (structures 1- 5) and linear (structures 6-8) immunogenic compositions comprising of positively charged (Arginine, R; Lysine, K) or negatively charged (Aspartic acid, D; Glutamic acid, E) amino acids in terminal positions such that their respective electrostatic charges are displayed to the environment. Each immunogenic composition also contains dipalmitoyl-S-glyceryl cysteine (Pam2Cys) which is a ligand for Toll-Like Receptor 2. Two serine residues (Ser) are also incorporated. In the case of construct 2 the peptide structure was assembled in the direction N→C, all other structures shown in the figure were assembled C→N. Positive and negative electrostatic charges are shown as 2-, 2+, 1- , 1+ etc. depending on the size of charge. Ac = acetyl group used to suppress the positive charge of alpha amino groups in the case of N-terminally situated Glutamic acid.

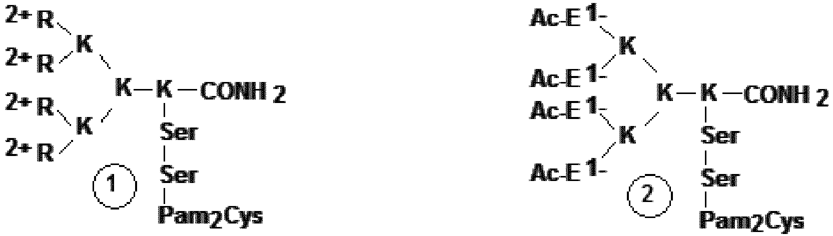

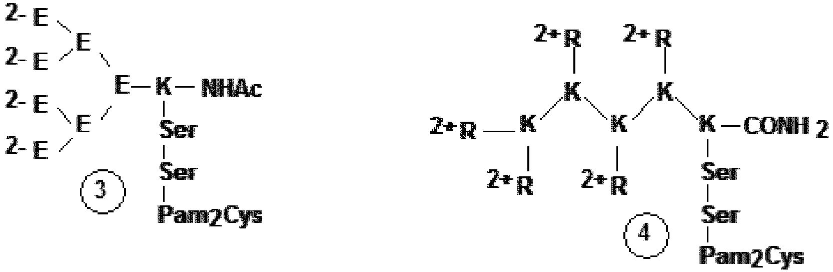

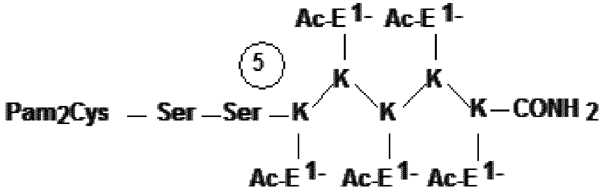

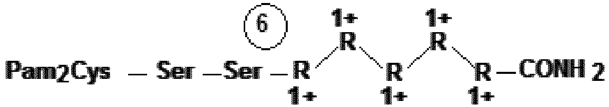

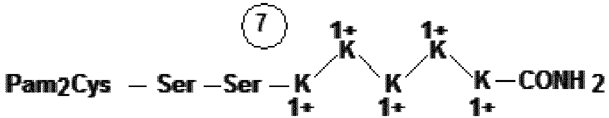

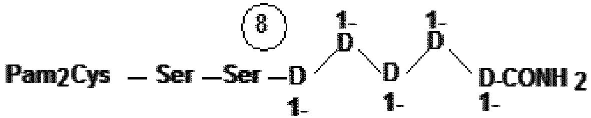

A person skilled in the art will appreciate that the present invention is not limited to the particular exemplified compounds or functional groups that can act as solubilising agents, and that other suitable compounds or functional groups including those that can act as solubilising agents known in the art may be used in accordance with the present invention, such as carbohydrates, provided that the solubilising agent also comprises PEG.

The way in which the solubilising agents may be conjugated to a lipid according to the present invention would be well known to a person skilled in the art. For example, conjugation via Fmoc chemistry, through a disulfide or a thioether bridge, or via oxime chemistry is envisaged. In a particular embodiment of the present invention, a soluble form of Pam2Cys was prepared by addition of O-(N-Fmoc-2-aminoethyl)-O'-(2-carboxyethyl)-undecaethyleneglycol (Fmoc-PEOn-OH, Merck Ltd) to Pam2Cys. This resulted in the formation of a PEGylated form of the lipid, Pam2Cys-PEG₁₁ which is then suitable for administration to a subject.

According to the present invention, the TLR2 moiety comprises a conjugate comprising Pam2Cys conjugated to PEG. In a preferred form according to any embodiment of the present invention, the TLR2 moiety comprises a conjugate comprising Pam2Cys conjugated to PEG₁₁ or PEG_{12.} Preferably, the Pam2Cys and PEG₁₁ or PEG₁₂ molecules are separated by at least two serines (PEG₁₁-SS-Pam2Cys or PEG₁₂-SS-Pam2Cys).

As used herein, reference to a TLR2 agonist also includes a pharmaceutically acceptable salt, solvate, polymorph or prodrug thereof.

Additional compounds that comprise a TLR2 agonist that are useful in any aspect of the present invention are described below.

In any aspect of the present invention, the compound comprising a TLR2 agonist comprises the structure:

A - Y - B

wherein A comprises or consists of: 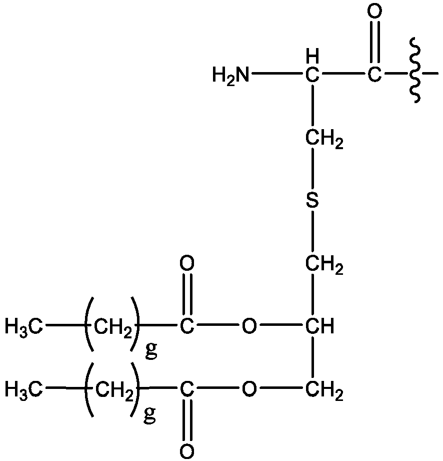 wherein each g is independently 12, 14, or 16;
Y is 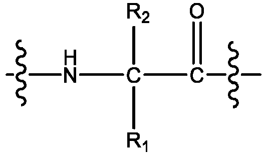
wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
   and
B comprises or consists of Polyethylene Glycol (PEG),
or a pharmaceutically acceptable salt or prodrug thereof.

In any aspect of the present invention, the compound comprising a TLR2 agonist comprises Pam2Cys and PEG, wherein the Pam2Cys and PEG are linked by a serine, homoserine, threonine or phosphoserine residue,
wherein
Pam2Cys in the compound has the structure:

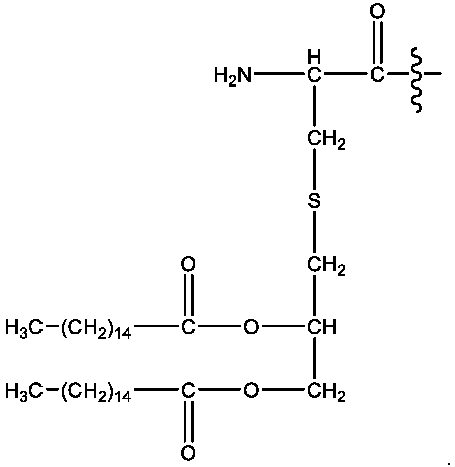

In one aspect, the present invention provides a compound comprising: 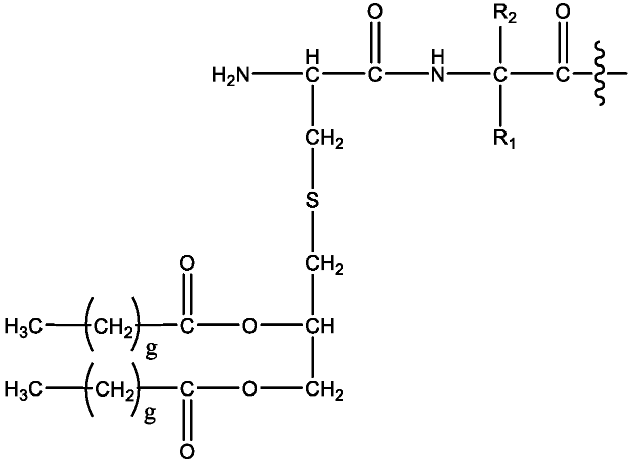 wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
covalently linked to polyethylene glycol (PEG),
or a pharmaceutically acceptable salt or prodrug thereof.

In any aspect of the present invention, the compound comprising a TLR2 agonist is a compound of formula (I): 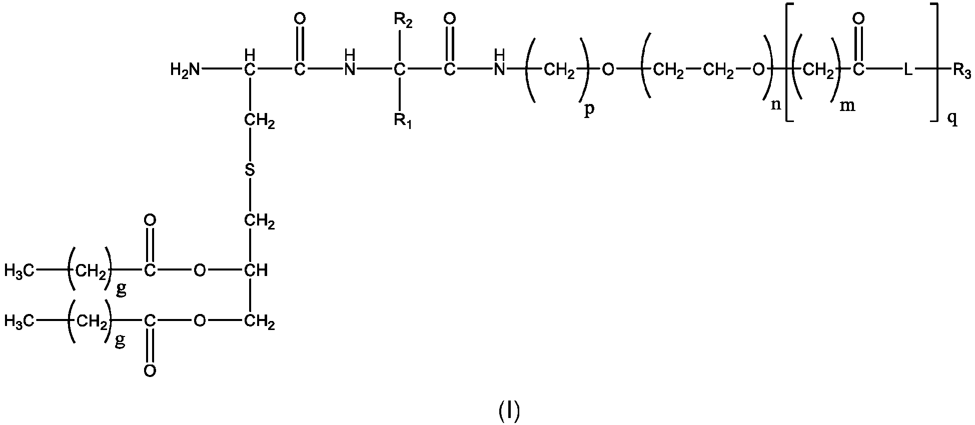 wherein
n is 3 to 100;
m is 1, 2, 3 or 4;
each g is independently 12, 14, or 16;
p is 2, 3 or 4;
q is null or 1;
R₁ and R₂ are independently selected from the group consisting of H, -CH₂OH, - CH₂CH₂OH, -CH(CH₃)OH and -CH₂OPO(OH)₂, wherein any one of the alkyl hydrogens can be replaced with a halogen, and wherein R₁ and R₂ are not both H;
wherein when q= 1, R₃ is -NH₂ or -OH;
wherein when q=0, R₃ is H;
L is null or consists of 1 to 10 units, wherein each unit is a natural alpha amino acid or derived from a natural alpha amino acid, and has the formula: 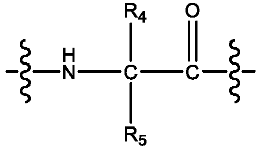
wherein R₄ is H; and
Rs is the side chain, or second hydrogen of the amino acid
or a pharmaceutically acceptable salt or prodrug thereof.

In any aspect of the present invention, the compound comprising a TLR2 agonist is a compound of formula (II):

A-Y-NH-(CH₂)ₚ-O-(CH₂-CH₂-O)ₙ-[(CH₂)ₘ-CO-L-]_{q}R₃ (II)

wherein
A has the structure: 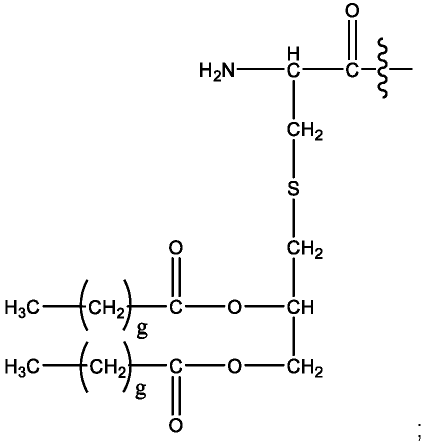
Y is 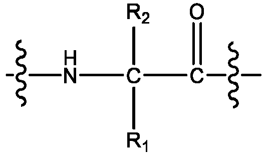
wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
n is 3 to 100;
m is 1, 2, 3 or 4;
each g is independently 12, 14, or 16;
p is 2, 3 or 4;
q is null or 1;
wherein when q= 1, Rs is -NH₂ or -OH;
wherein when q=0, R₃ is H;
L is null or consists of 1 to 10 units, wherein each unit is a natural alpha amino acid or derived from a natural alpha amino acid, and has the formula: 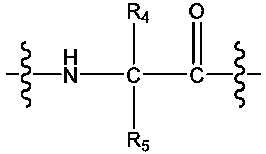
wherein R₄ is H; and
R₅ is the side chain, or second hydrogen of the amino acid,
or a pharmaceutically acceptable salt or prodrug thereof.

In any aspect of the present invention, the compound comprising a TLR2 agonist is a compound of formula (III):

Pam2Cys-Y-NH-(CH₂)ₚ-O-(CH₂-CH₂-O)ₙ-[(CH₂)ₘ-CO-L-]_{q}R₃ (III)

wherein
Pam2Cys has the structure: 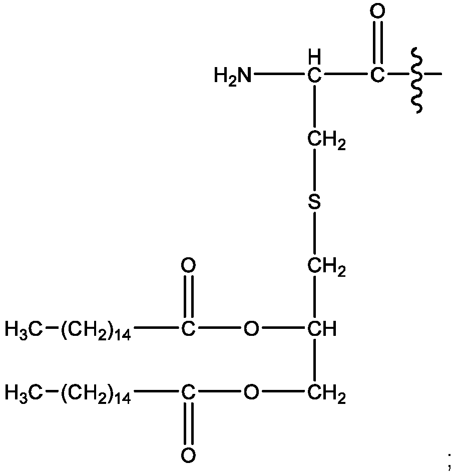
Y is: 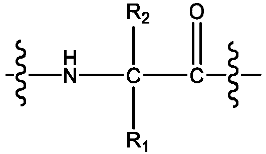
wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
n is 3 to 100;
m is 1, 2, 3 or 4;
p is 2, 3 or 4;
q is null or 1;
wherein when q= 1, R₃ is H, -NH₂ or -OH;
wherein when q=0, R₃ is H;
L is null or consists of 1 to 10 units, wherein each unit is a natural alpha amino acid or derived from a natural alpha amino acid, and has the formula: 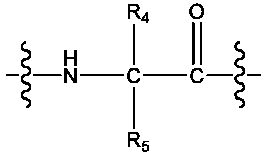
wherein R₄ is H; and
R₅ is the side chain, or second hydrogen of the amino acid,
or a pharmaceutically acceptable salt or prodrug thereof.

In any aspect of the present invention, the compound comprising a TLR2 agonist is a compound of formula (IV):

Pam2Cys-Ser-NH-(CH₂)ₚ-O-(CH₂-CH₂-O)ₙ-[(CH₂)ₘ-CO-L-]_{q}R₃ (IV)

wherein
Pam2Cys-Ser has the structure: 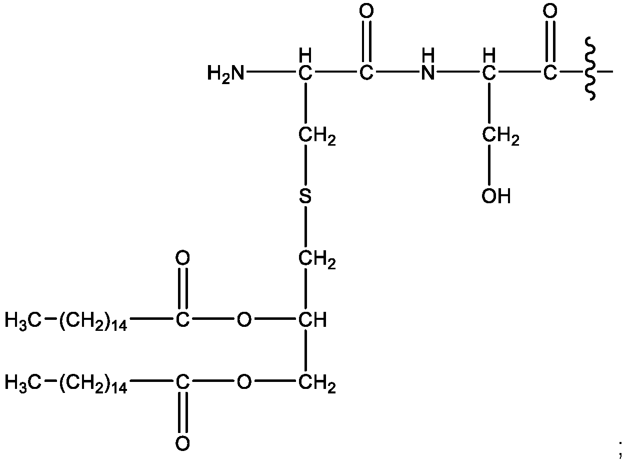
n is 3 to 100;
m is 1, 2, 3 or 4;
p is 2, 3 or 4;
q is null or 1;
wherein when q= 1, R₃ is -NH₂ or -OH;
wherein when q=0, R₃ is H;
L is null or consists of 1 to 10 units, wherein each unit is a natural alpha amino acid or derived from a natural alpha amino acid, and has the formula: 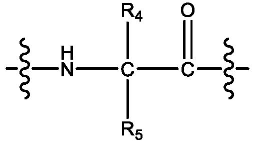
wherein R₄ is H; and
R₅ is the side chain, or second hydrogen of the amino acid,
or a pharmaceutically acceptable salt or prodrug thereof.

In one embodiment, the compound has the formula (V): 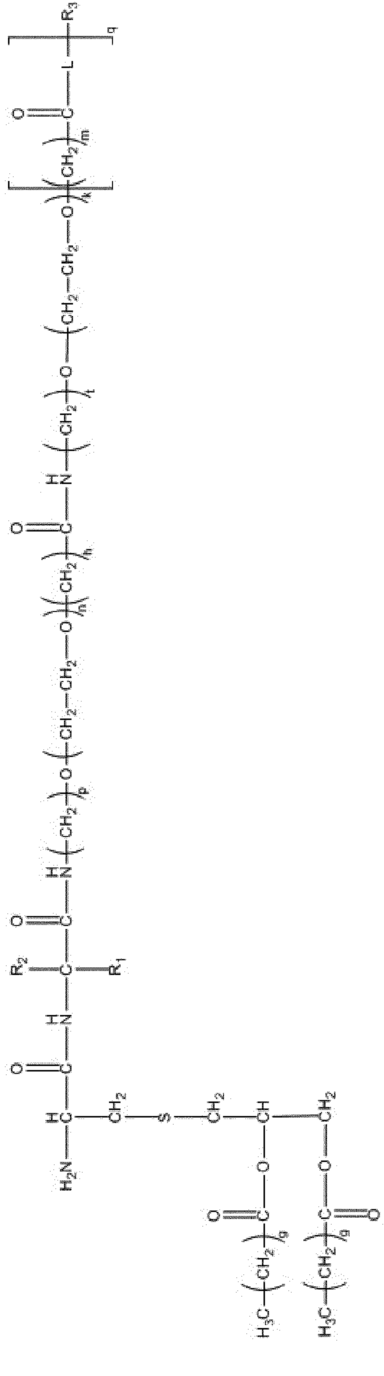 wherein
n is 3 to 100;
k is 3 to 100;
m is 1, 2, 3 or 4;
each g is independently 12, 14, or 16;
p is 2, 3 or 4;
t is 2, 3 or 4;
h is 1, 2, 3 or 4;
q is null or 1;
R₁ and R₂ are independently selected from the group consisting of H, -CH₂OH, - CH₂CH₂OH, -CH(CH₃)OH and -CH₂OPO(OH)₂, wherein any one of the alkyl hydrogens can be replaced with a halogen, and wherein R₁ and R₂ are not both H;
wherein when q= 1, R₃ is -NH₂ or -OH;
wherein when q=0, R₃ is H;
L is null or consists of 1 to 10 units, wherein each unit is a natural alpha amino acid or derived from a natural alpha amino acid, and has the formula: 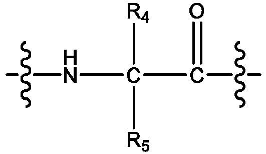
wherein R₄ is H; and
R₅ is the side chain, or second hydrogen of the amino acid,
or a pharmaceutically acceptable salt or prodrug thereof.

In one preferred embodiment, the compound has the structure of compound (1): 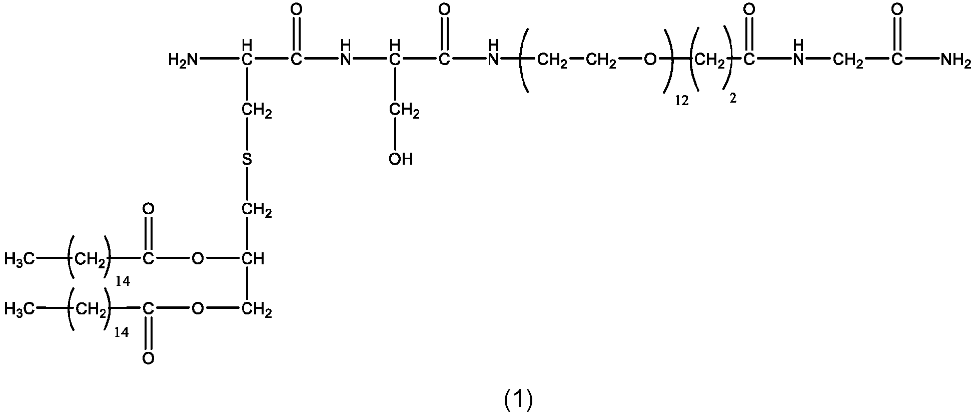 or a pharmaceutically acceptable salt or prodrug thereof.

This compound may also be referred to herein as 'Pam₂Cys-Ser-PEG', or `INNA-006'.

In other preferred embodiments, the compound is selected from the group consisting of: 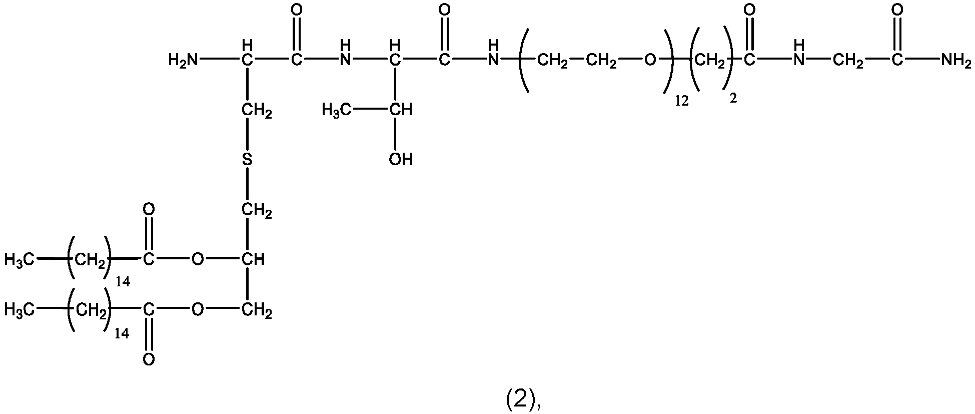 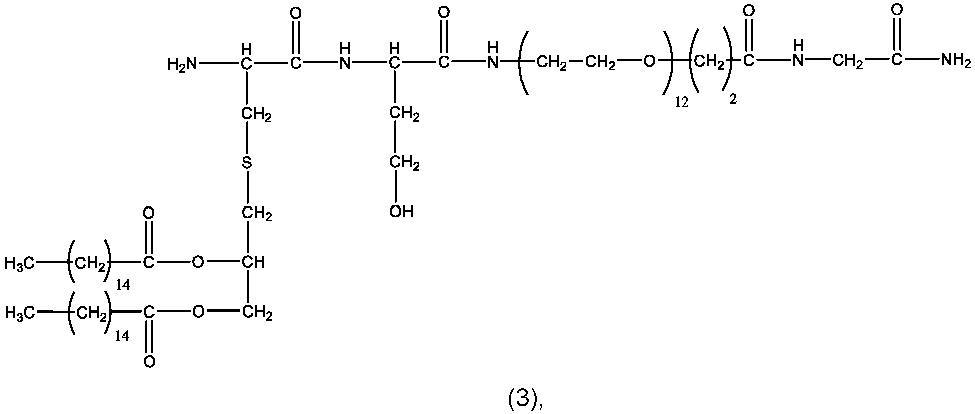 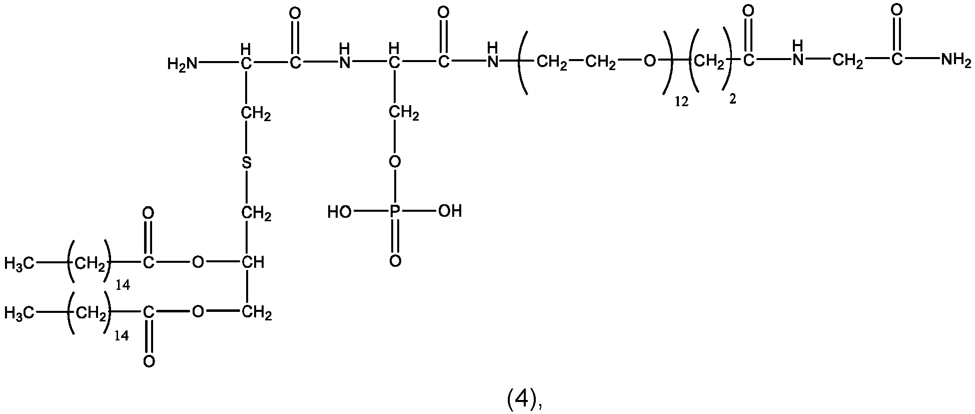 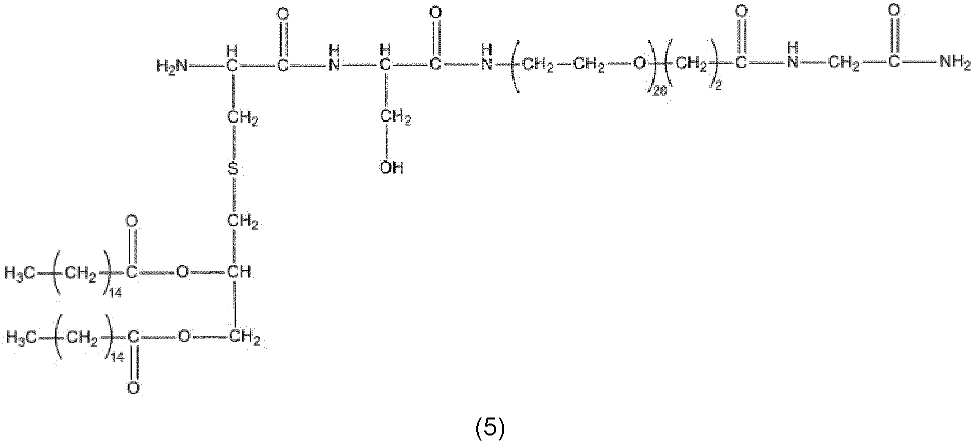 and

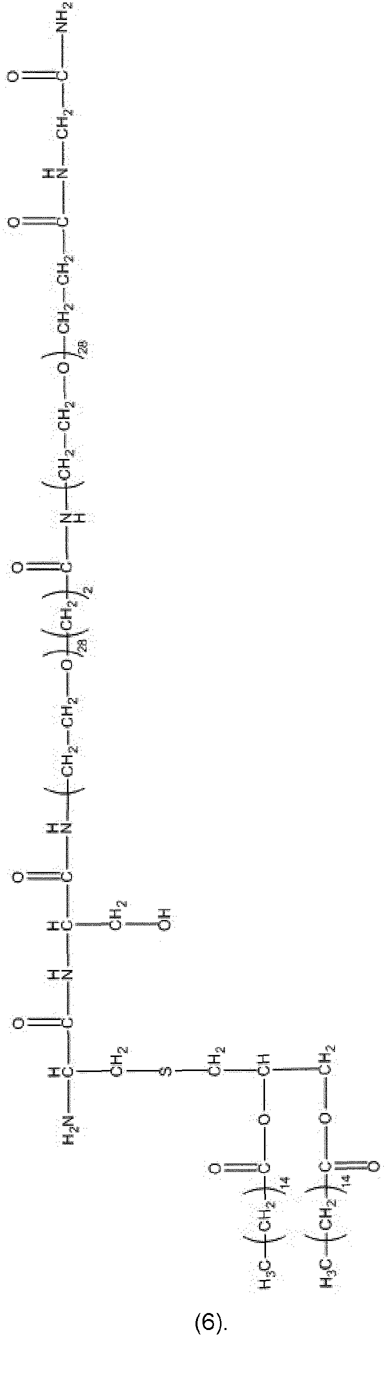

In one particularly preferred embodiment, the compound is:

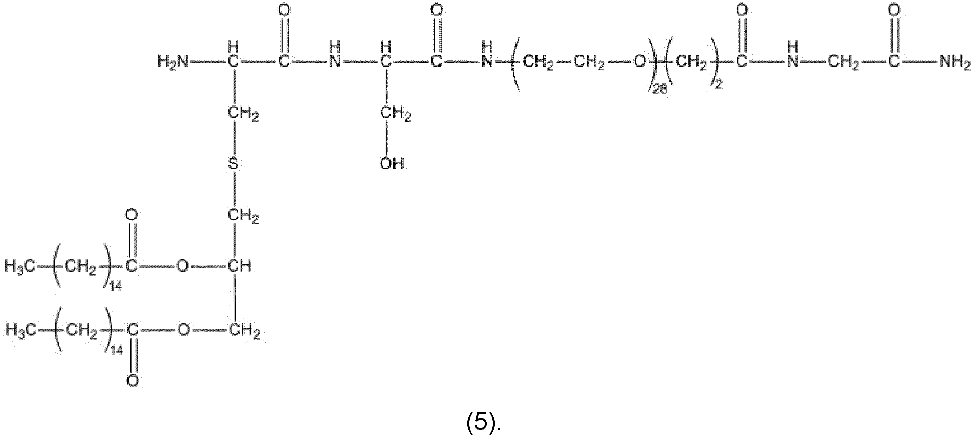

In any aspect of the present invention, the compound comprising a TLR2 agonist is a compound of formula (Ia): 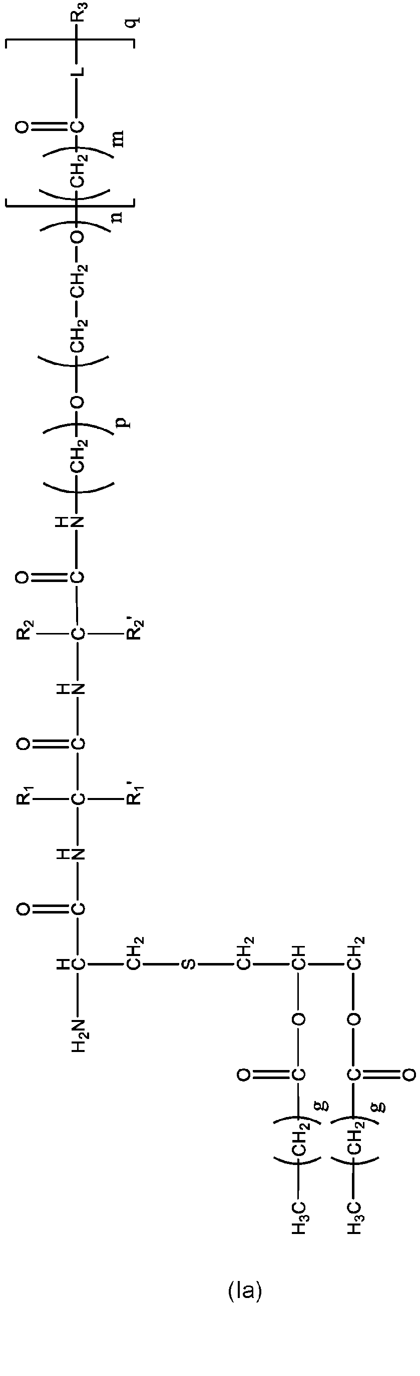 wherein
n is 3 to 100;
m is 1, 2, 3 or 4;
each g is independently 12, 14, or 16;
p is 2, 3 or 4;
q is null or 1;
R₁, R₁', R₂ and R₂' are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₁' are not both H, and R₂ and R₂' are not both H;
wherein when q is null, R₃ is H;
wherein when q is 1, R₃ is -NH₂ or -OH;
L is null or consists of 1 to 10 units, wherein each unit is a natural alpha amino acid or derived from a natural alpha amino acid, and has the formula: 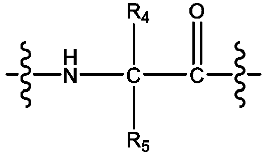
wherein R₄ is H; and
R₅ is the side chain, or second hydrogen of the amino acid,
or a pharmaceutically acceptable salt or prodrug thereof.

In any aspect of the present invention, the compound comprising a TLR2 agonist is a compound of formula (IIa):

A-Y-NH-(CH₂)ₚ-O-(CH₂-CH₂-O)ₙ-[(CH₂)ₘ-CO-L-]_{q}R₃ (IIa)

wherein
A has the structure: 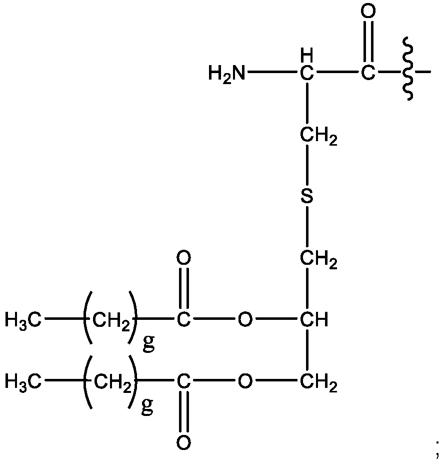
Y is 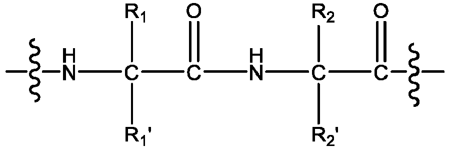
wherein R₁, R₁', R₂ and R₂' are independently selected from the group consisting of H, -CH₂OH, -CH₂CH₂OH, -CH(CH₃)OH and -CH₂OPO(OH)₂, wherein any one of the alkyl hydrogens can be replaced with a halogen, and wherein R₁ and R₁' are not both H, and R₂ and R₂' are not both H;
n is 3 to 100;
m is 1, 2, 3 or 4;
each g is independently 12, 14, or 16;
p is 2, 3 or 4;
q is null or 1;
wherein when q is null, R₃ is H;
wherein when q is 1, R₃ is -NH₂ or -OH;
L is null or consists of 1 to 10 units, wherein each unit is a natural alpha amino acid or derived from a natural alpha amino acid, and has the formula: 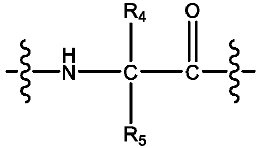
wherein R₄ is H; and
Rs is the side chain, or second hydrogen of the amino acid,
or a pharmaceutically acceptable salt or prodrug thereof.

In any aspect of the present invention, the compound comprising a TLR2 agonist is a compound of formula (IIIa):

Pam2Cys-Y-NH-(CH₂)ₚ-O-(CH₂-CH₂-O)ₙ-[(CH₂)ₘ-CO-L-]_{q}R₃ (IIIa)

wherein
Pam2Cys has the structure: 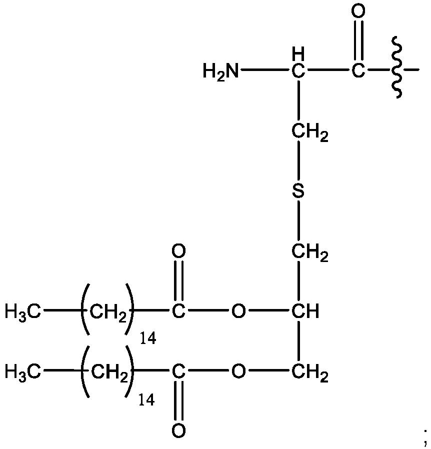
Y is 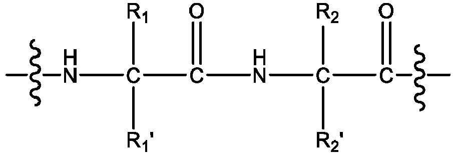
wherein R₁, R₁', R₂ and R₂' are independently selected from the group consisting of H, -CH₂OH, -CH₂CH₂OH, -CH(CH₃)OH and -CH₂OPO(OH)₂, wherein any one of the alkyl hydrogens can be replaced with a halogen, and wherein R₁ and R₁' are not both H, and R₂ and R₂' are not both H;
n is 3 to 100;
m is 1, 2, 3 or 4;
p is 2, 3 or 4;
q is null or 1;
wherein when q is null, R₃ is H;
wherein when q is 1, R₃ is -NH₂ or -OH;
L is null or consists of 1 to 10 units, wherein each unit is a natural alpha amino acid or derived from a natural alpha amino acid, and has the formula: 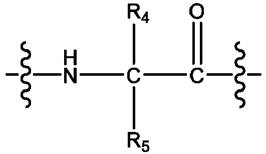
wherein R₄ is H; and
R₅ is the side chain, or second hydrogen of the amino acid,
or a pharmaceutically acceptable salt or prodrug thereof.

In any aspect of the present invention, the compound comprising a TLR2 agonist is a compound of formula (IVa):

Pam2Cys-Ser-Ser-NH-(CH₂)ₚ-O-(CH₂-CH₂-O)ₙ-[(CH₂)ₘ₋CO-L-]_{q}R₃ (IVa)

wherein
Pam2Cys has the structure: 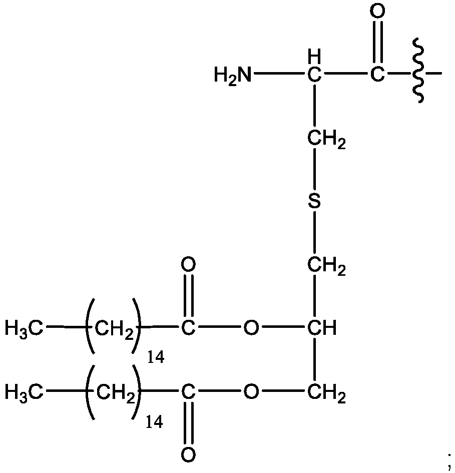
n is 3 to 100;
m is 1, 2, 3 or 4;
p is 2, 3 or 4;
q is null or 1;
R₁, R₁', R₂ and R₂' are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₁' are not both H, and R₂ and R₂' are not both H;
wherein when q is null, R₃ is H;
wherein when q is 1, R₃ is -NH₂ or -OH;
L is null or consists of 1 to 10 units, wherein each unit is a natural alpha amino acid or derived from a natural alpha amino acid, and has the formula: 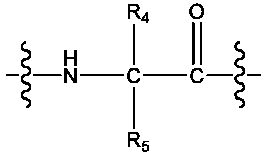
wherein R₄ is H; and
Rs is the side chain, or second hydrogen of the amino acid,
or a pharmaceutically acceptable salt or prodrug thereof.

In any aspect of the present invention, the compound comprising a TLR2 agonist is a compound of formula (Va): 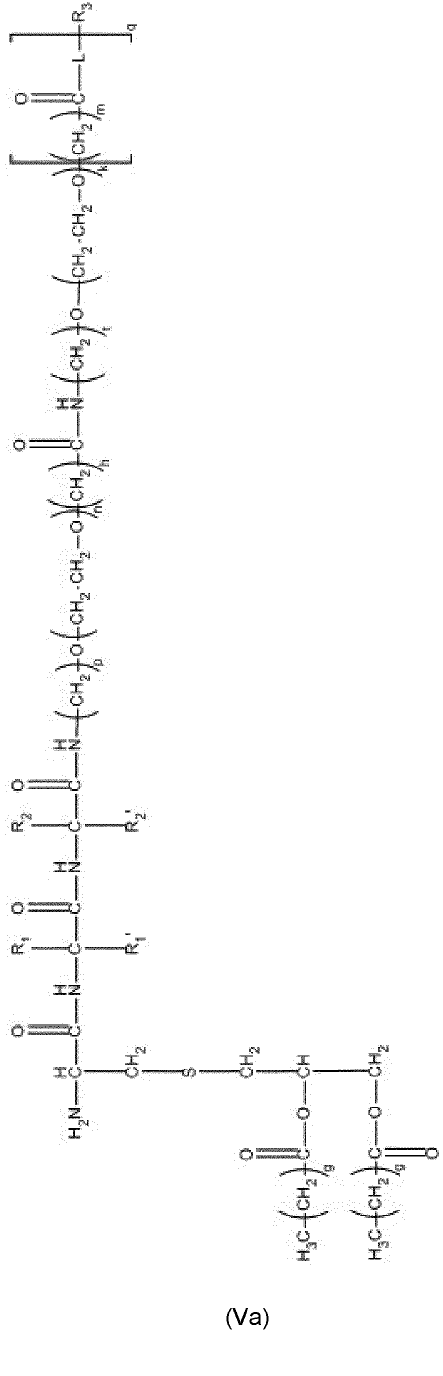 wherein
n is 3 to 100;
k is 3 to 100;
h is 1, 2, 3 or 4;
m is 1, 2, 3 or 4;
each g is independently 12, 14, or 16;
p is 2, 3 or 4;
t is 2, 3 or 4;
q is null or 1;
R₁, R₁', R₂ and R₂' are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₁' are not both H, and R₂ and R₂' are not both H;
wherein when q is null, Rs is H;
wherein when q is 1, Rs is -NH₂ or -OH;
L is null or consists of 1 to 10 units, wherein each unit is a natural alpha amino acid or derived from a natural alpha amino acid, and has the formula: 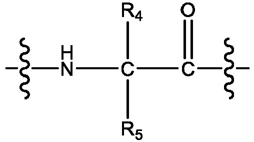
wherein R₄ is H; and
Rs is the side chain, or second hydrogen of the amino acid,
or a pharmaceutically acceptable salt or prodrug thereof.

In one embodiment, the compound has the structure:

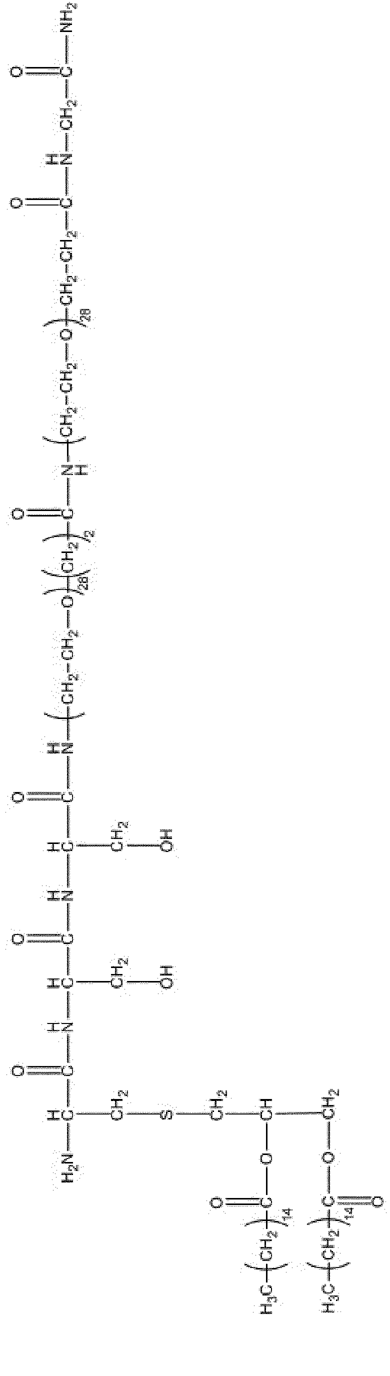

In a particularly preferred embodiment of the invention, the compound has the structure of compound (1a): 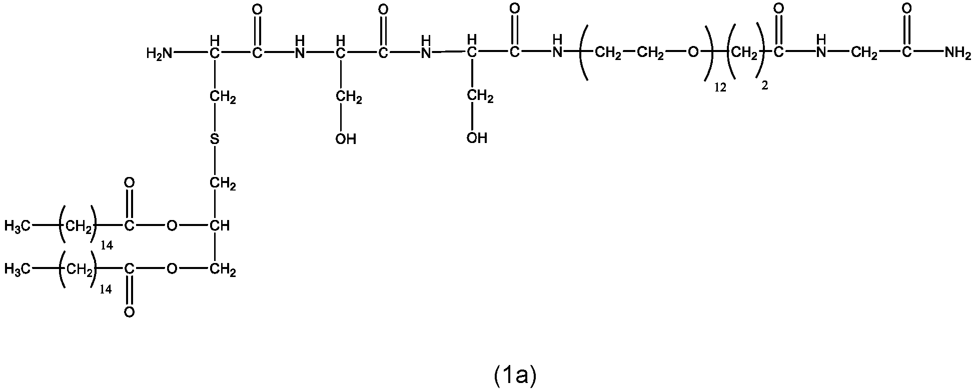 or a pharmaceutically acceptable salt or prodrug thereof.

In other preferred embodiments, the compound is selected from the group consisting of:

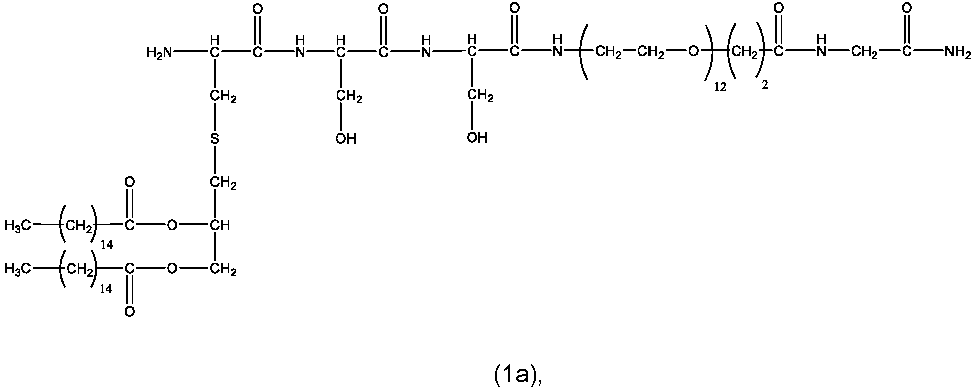

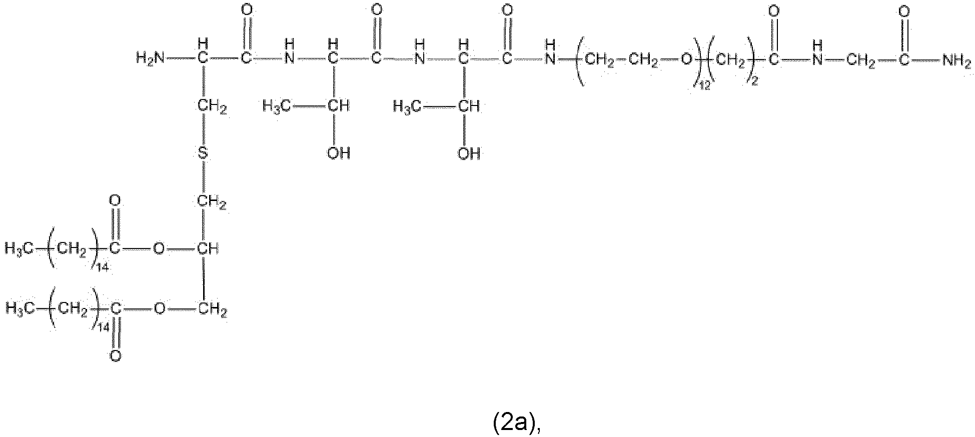

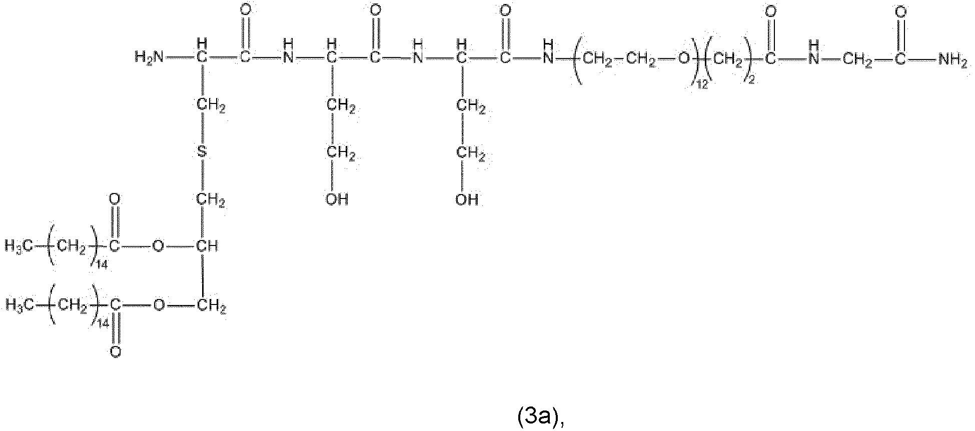

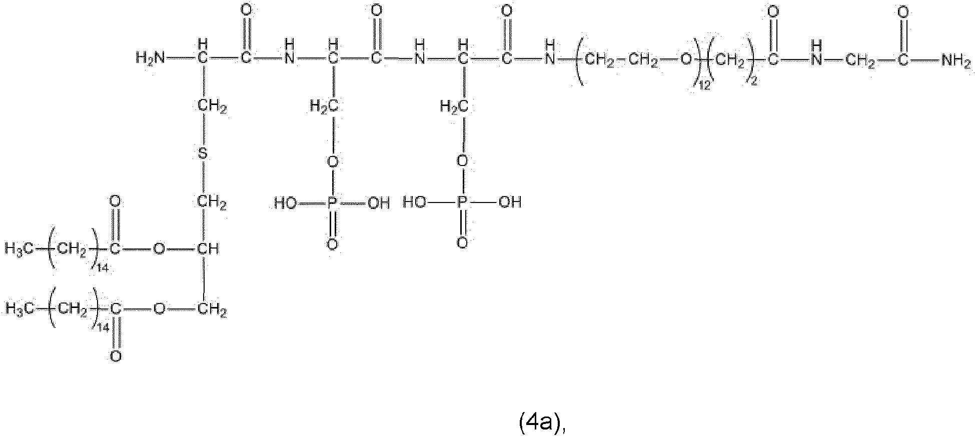

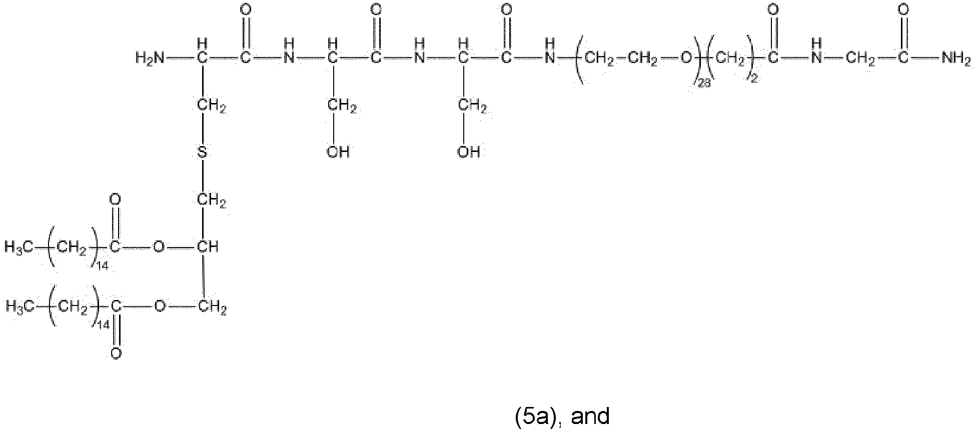

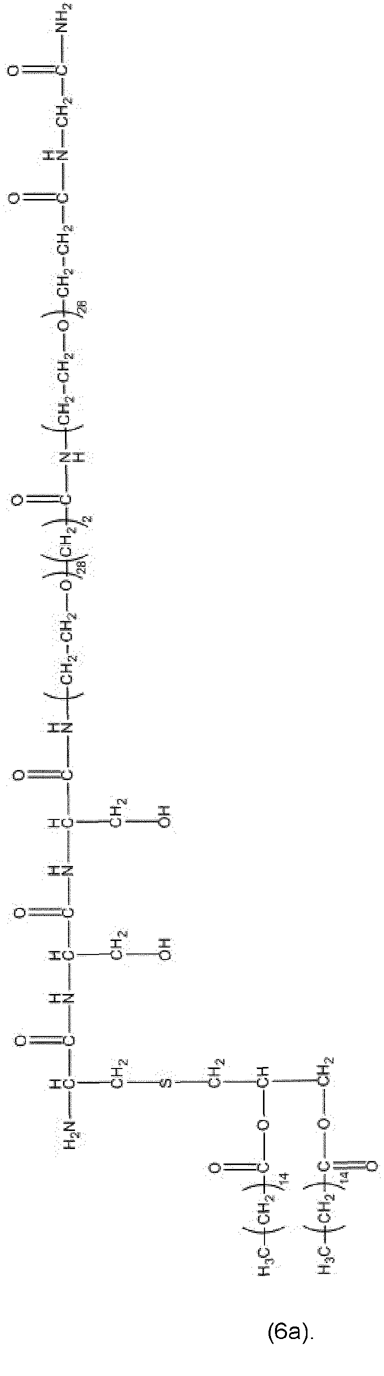

Also included as compounds of the invention are pharmaceutically acceptable salts or prodrugs of compounds (1) to (6), or (1a) to (6a) above.

For all the above structures, where present, one or more of the following features are preferable:
n is between 10-14, even more preferably, n is 11.
n is 3 or 5.
n is between 24-30, even more preferably, n is 27.
k is between 24-30, even more preferably, k is 27.
m is 1-3, even more preferably, m is 2.
h is 1-3, even more preferably, h is 2.
g is 12, 14, or 16, most preferably, g is 14.
one of R₁ and R₂ is hydrogen.
p is 2.
t is 2.

The term "pharmaceutically acceptable" may be used to describe any pharmaceutically acceptable salt, hydrate or prodrug, or any other compound which upon administration to a subject, is capable of providing (directly or indirectly) a compound of the invention as described herein, or a pharmaceutically acceptable salt, prodrug or ester thereof, or an active metabolite or residue thereof.

Suitable pharmaceutically acceptable salts may include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, malic, citric, lactic, mucic, gluconic, benzoic, succinic, oxalic, phenylacetic, methanesulphonic, toluenesulphonic, benzenesulphonic, salicylic, sulphanilic, aspartic, glutamic, edetic, stearic, palmitic, oleic, lauric, pantothenic, tannic, ascorbic and valeric acids.

Base salts may include, but are not limited to, those formed with pharmaceutically acceptable cations, such as sodium, potassium, lithium, calcium, magnesium, zinc, ammonium, alkylammonium such as salts formed from triethylamine, alkoxyammonium such as those formed with ethanolamine and salts formed from ethylenediamine, choline or amino acids such as arginine, lysine or histidine. General information on types of pharmaceutically acceptable salts and their formation is known to those skilled in the art and is as described in general texts such as *"*Handbook of Pharmaceutical salts" P.H.Stahl, C.G.Wermuth, 1st edition, 2002, Wiley-VCH.

In the case of compounds that are solids, it will be understood by those skilled in the art that the inventive compounds, agents and salts may exist in different crystalline or polymorphic forms, all of which are intended to be within the scope of the present invention and specified formulae.

The term "polymorph" includes any crystalline form of compounds of the invention as described herein, such as anhydrous forms, hydrous forms, solvate forms and mixed solvate forms.

Compounds described herein are intended to cover, where applicable, solvated as well as unsolvated forms of the compounds. Thus compounds described herein include compounds having the indicated structures, including the hydrated or solvated forms, as well as the non-hydrated and non-solvated forms.

As used herein, the term "solvate" refers to a complex of variable stoichiometry formed by a solute (in this invention, a compound of the invention described herein, or a pharmaceutically acceptable salt, prodrug or ester thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include, without limitation, water, ethanol and acetic acid. Most preferably the solvent used is water.

Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

The compounds as described herein are to also include isotope variations, such as the replacement of hydrogen for deuterium.

Compounds described herein may exist in and be isolated in optically active and racemic forms. As would be understood by a person skilled in the art, the present invention is intended to encompass any racemic, optically active or stereoisomeric form, or mixtures thereof, of compounds of Formula (I), (II), (III), (IV), (V), (Ia), (IIa), (IIIa), (IVa) and/or (Va) which possess the useful properties described herein. It is well known in the art how to prepare such forms (for example, by resolution of racemic mixtures by recrystallization, by synthesis from optically-active starting materials, by chiral synthesis, or by chiral chromatographic separation). In one preferred embodiment, with regard to the carbons shown with a * below, the compound is provided in a racemic mixture. In another preferred aspect, the compound is provided with excess of, or only, the L-configuration or naturally occurring amino acid: 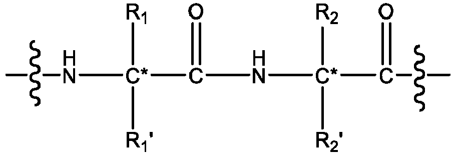 or

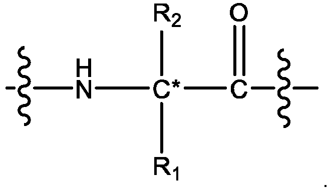

A "prodrug" is a compound that may not fully satisfy the structural requirements of the compounds provided herein, but is modified in vivo, following administration to a subject or patient, to produce a compound of the invention as described herein. For example, a prodrug may be an acylated derivative of a compound as provided herein. Prodrugs include compounds wherein hydroxy, carboxy, amine or sulfhydryl groups are bonded to any group that, when administered to a mammalian subject, cleaves to form a free hydroxy, carboxy, amino, or sulfhydryl group, respectively. Examples of prodrugs include, but are not limited to, acetate, formate, phosphate and benzoate derivatives of alcohol and amine functional groups within the compounds provided herein. Prodrugs of the compounds provided herein may be prepared by modifying functional groups present in the compounds in such a way that the modifications are cleaved *in vivo* to generate the parent compounds.

Prodrugs include compounds wherein an amino acid residue, or a polypeptide chain of two or more (eg, two, three or four) amino acid residues which are covalently joined to free amino, and amido groups of compounds of Formula (I), (II), (III), (IV), (V), (Ia), (IIa), (IIIa), (IVa) and/or (Va). The amino acid residues include the 20 naturally occurring amino acids commonly designated by three letter symbols and also include, 4-hydroxyproline, hydroxylysine, demosine, isodemosine, 3-methylhistidine, norvlin, beta-alanine, gamma-aminobutyric acid, citrulline, homocysteine, homoserine, ornithine and methionine sulfone. Prodrugs also include compounds wherein carbonates, carbamates, amides and alkyl esters which are covalently bonded to the above substituents of Formula (I), (II), (III), (IV), (V), (Ia), (IIa), (IIIa), (IVa) and/or (Va), or other structure as depicted herein.

The term 'respiratory' refers to the process by which oxygen is taken into the body and carbon dioxide is discharged, through the bodily system including the nose, throat, larynx, trachea, bronchi and lungs.

As used herein, respiratory tract includes the upper and lower respiratory tracts. Typically, the upper respiratory tract includes the nose and nasal passages, paranasal sinuses, the pharynx, and the portion of the larynx above the vocal folds (cords). Typically, the lower respiratory tract includes the portion of the larynx below the vocal folds, trachea, bronchi and bronchioles. The lungs can be included in the lower respiratory tract or as separate entity and include the respiratory bronchioles, alveolar ducts, alveolar sacs, and alveoli.

The term 'respiratory disease' or 'respiratory condition' refers to any one of several ailments that involve inflammation and affect a component of the respiratory system including the upper (including the nasal cavity, pharynx and larynx) and lower respiratory tract (including trachea, bronchi and lungs). Preferably, the respiratory disease is an obstructive airway disease, such ailments include asthmatic conditions including hay fever, allergen-induced asthma, exercise-induced asthma, pollution- induced asthma, cold-induced asthma, stress-induced asthma and viral-induced- asthma, chronic obstructive pulmonary diseases including chronic bronchitis with normal airflow, chronic bronchitis with airway obstruction (chronic obstructive bronchitis), emphysema, asthmatic bronchitis, and bullous disease, and other pulmonary diseases involving inflammation including cystic fibrosis, pigeon fancier's disease, farmer's lung, acute respiratory distress syndrome, pneumonia, aspiration or inhalation injury, fat embolism in the lung, acidosis inflammation of the lung, acute pulmonary edema, acute mountain sickness, post-cardiac surgery, acute pulmonary hypertension, persistent pulmonary hypertension of the newborn, hyaline membrane disease, acute pulmonary thromboembolism, sepsis, status asthmaticus and hypoxia. The inflammation in the upper and lower respiratory tract may be associated with or caused by viral infection or an allergen. It is expected that the anti-inflammatory activity of the compounds either alone or when co-administered with a glucocorticoid would make them particularly suitable for treatment of these disease or conditions.

A symptom of respiratory disease may include cough, excess sputum production, a sense of breathlessness or chest tightness with audible wheeze. Exercise capacity may be quite limited. In asthma the FEV1 .0 (forced expiratory volume in one second) as a percentage of that predicted nomographically based on weight, height and age, may be decreased as may the peak expiratory flow rate in a forced expiration. In COPD the FEV1.0 as a ratio of the FVC is typically reduced to less than 0.7. The impact of each of these conditions may also be measured by days of lost work/school, disturbed sleep, requirement for bronchodilator drugs, requirement for glucocorticoids including oral glucocorticoids.

The existence of, improvement in, treatment of or prevention of a respiratory disease may be determined by any clinically or biochemically relevant method of the subject or a biopsy therefrom. For example, a parameter measured may be the presence or degree of lung function, signs and symptoms of obstruction; exercise tolerance; night time awakenings; days lost to school or work; bronchodilator usage; ICS dose; oral GC usage; need for other medications; need for medical treatment; hospital admission.

As used herein, the term respiratory infection means an infection anywhere in the respiratory tract. Examples of respiratory infection include but are not limited to colds, sinusitis, throat infection, tonsillitis, laryngitis, bronchitis, pneumonia or bronchiolitis. Preferably, in any embodiment of the invention the respiratory infection is a cold. An individual may be identified as having a respiratory tract infection by viral testing and may exhibit symptoms of itchy watery eyes, nasal discharge, nasal congestion, sneezing, sore throat, cough, headache, fever, malaise, fatigue and weakness. In one aspect, a subject having a respiratory infection may not have any other respiratory condition. Detection of the presence or amount of virus, preferably rhinovirus, may be by PCR/sequencing of RNA isolated from clinical samples (nasal wash, sputum, BAL) or serology.

The respiratory condition associated with a rhinovirus may be a condition caused by rhinovirus. Preferably, the condition is associated with or caused by a rhinovirus infection. A rhinovirus infection may be determined by the presence of rhinovirus in a sample taken from the respiratory tract of a subject. An individual may be identified as having an RV infection by serological viral testing or PCR/sequencing of RNA isolated from clinical samples (nasal wash, sputum, BAL). Symptoms of RV infection include but are not limited to sore throat, runny nose, nasal congestion, sneezing and cough; sometimes accompanied by muscle aches, fatigue, malaise, headache, muscle weakness, or loss of appetite.

According to the invention the respiratory infection is caused by a rhinovirus (RV) or exacerbated by a RV. As used herein, the term RV refers to a picornavirus comprising any single-stranded positive sense RNA with a genome virus-encoded protein at the 5' region and a 3' poly-A tail. It will be understood that the viral particles themselves are not enveloped and are icosahedral in structure. It will also be understood that human rhinoviruses are composed of a capsid that contains four viral proteins VP1, VP2, VP3 and VP4. VP1, VP2, and VP3 form the major part of the protein capsid. Examples of human rhinoviruses may include any of the following: HRV-A1, HRV-A2, HRV-A7, HRV-A8, HRV-A9, HRV-A10, HRV-A11, HRV-A12, HRV-A13, HRV-A15, HRV-A16, HRV-A18, HRV-A19, HRV-A20, HRV-A21, HRV-A22, HRV-A23, HRV-A24, HRV-A25, HRV-A28, HRV-A29, HRV-A30, HRV-A31, HRV-A32, HRV-A33, HRV-A34, HRV-A36, HRV-A38, HRV-A39, HRV-A40, HRV-A41, HRV-A43, HRV-A44, HRV-A45, HRV-A46, HRV-A47, HRV-A49, HRV-A50, HRV-A51, HRV-A53, HRV-A54, HRV-A55, HRV-A56, HRV-A57, HRV-A58, HRV-A59, HRV-A60, HRV-A61, HRV-A62, HRV-A63, HRV-A64, HRV-A65, HRV-A66, HRV-A67, HRV-A68, HRV-A71, HRV-A73, HRV-A74, HRV-A75, HRV-A76, HRV-A77, HRV-A78, HRV-A80, HRV-A81, HRV-A82, HRV-A85, HRV-A88, HRV-A89, HRV-A90, HRV-A94, HRV-A95, HRV-A96, HRV-A98, HRV-A100, HRV-A101, HRV-A102 and HRV-A103, which are collectively known as rhinovirus A viruses; HRV-B3, HRV-B4, HRV-B5, HRV-B6, HRV-B14, HRV-B17, HRV-B26, HRV-B27, HRV-B35, HRV-B37, HRV-B42, HRV-B48, HRV-B52, HRV-B69, HRV-B70, HRV-B72, HRV-B79, HRV-B83, HRV-B84, HRV-B86, HRV-B91, HRV-B92, HRV-B93, HRV-B97, and HRV-B99 which are collectively known as rhinovirus B viruses; and HRV-C1, HRV-C2, HRV-C3, HRV-C4, HRV-C5, HRV-C6, HRV-C7, HRV-C8, HRV-C9, HRV-C10, HRV-C11, HRV-C12, HRV-C13, HRV-C14, HRV-C15, HRV-C16, HRV-C17, HRV-C18, HRV-C19, HRV-C20, HRV-C21, HRV-C22, HRV-C23, HRV-C24, HRV-C25, HRV-C26, HRV-C27, HRV-C28, HRV-C29, HRV-C30, HRV-C31, HRV-C32, HRV-C33, HRV-C34, HRV-C35, HRV-C36, HRV-C37, HRV-C38, HRV-C39, HRV-C40, HRV-C41, HRV-C42, HRV-C43, HRV-C44, HRV-C45, HRV-C46, HRV-C47, HRV-C48, HRV-C49, HRV-C50 & HRV-C51 which are collectively known as rhinovirus C viruses.

In any aspect of the invention, administration of the TLR2 agonist may raise an innate immune response.

In asthma, human rhinoviruses have been associated with the majority of asthma exacerbations for which current therapy is inadequate. In any embodiment of the invention there is therefore provided a compound for the use in treating or preventing a viral mediated exacerbation of asthma as defined in claim 3, wherein the viral mediated exacerbation is caused by a rhinovirus infection.

As used herein, the term 'asthma' refers to a respiratory disorder characterized by episodic difficulty in breathing brought on by any one or a combination of three primary factors including: 1) bronchospasm (i.e., variable and reversible airway obstruction due to airway muscle contraction), 2) inflammation of the airway lining, and 3) bronchial hyper-responsiveness resulting in excessive mucous in the airways, which may be triggered by exposure to an allergen or combination of allergens (i.e., dust mites and mold), viral or bacterial infection (i.e., common cold virus), environmental pollutants (i.e., chemical fumes or smoke), physical over exertion (i.e., during exercise), stress, or inhalation of cold air. An individual may be characterized as suffering from, for example, allergen-induced asthma, exercise-induced asthma, pollution-induced asthma, viral-induced asthma, or cold-induced asthma. It will be understood that asthma causes recurring periods of wheezing (a whistling sound when you breathe), chest tightness, shortness of breath, and coughing.

As used herein, the term asthma exacerbation refers to acute or subacute episodes of progressively worsening shortness of breath, coughing, wheezing, and chest tightness or any combination thereof and may be accompanied by a decrease in expiratory flow. The intensity of exacerbations is variable. Sometimes the symptoms are mild and cannot be detected by the patient and other times they are very severe episodes that are life threatening. According to the invention, the asthma exacerbation is caused by rhinovirus infection.

An individual may be identified as having asthma exacerbation by extent of airflow obstruction by determining FEV1 or PEF and its repercussion on gaseous exchange. It will be understood that FEV1 and PEF are measurements used to assess expiratory flow. Depending on the values obtained, an exacerbation will be considered mild if the FEV1 or PEF value is equivalent to or higher than 70% its theoretical or best previous personal value respectively, moderate if the FEV1 or PEF measurement is between 70% to 50% and serious if these values are lower than 50%. It is estimated that the functional response to treatment is satisfactory when FEV1 or PEF values are higher than 45% of the predetermined value and PEF increases at least 50 l/min 30 minutes after treatment is initiated. The initial therapeutic airflow obstruction response is the key prognostic factor for assessing an attack. Table 1 (J Investig Allergol Clin Immunol Vol. 20, Suppl. 1: 27-31(2010) outlines those diagnostic indicators used to determine whether someone is having a mild or moderate-severe asthma exacerbation.

**Table 1: Classification of severity of asthma exacerbation**

| | **Mild Crisis** | **Moderate-severe Crisis** | **Imminent Respiratory Failure** |
|---|---|---|---|
| Dyspnea | Mild | Moderate-intense | Very intense |
| Speech | Paragraphs | Sentences-words | |
| Respiratory rate (x') | Increased | > 20-30 | |
| Heart rate (x') | <100 | >100-120 | Bradycardia |
| Use of accessory muscles | Absent | Present | Paradoxical thoracoabdominal movement |
| Wheezing | Present | Present | Ausculatory silence |
| Consciousness | Normal | Normal | Impaired |
| Paradoxical pulse | Absent | >10-25mmHg | Absence (muscular fatigue) |
| FEV1 or PEF (reference values) | >70% | <70% | |
| SaO2 (%) | >95% | 90-95% | <90% |
| PaO2 mmHg | Normal | 80-60 | <60 |
| PaCO2 mmHg | <40 | >40 | >40 |

| | | | |
|---|---|---|---|
| Abbreviations: FEV₁: forced expiratory volume during the first second; PEF: peak expiratory flow; x': per minute; SaO₂: oxyhaemoglobin saturation; PaO₂: arterial oxygen pressure; PaCO₂: arterial carbon dioxide pressure. | | | |

In many cases, asthma exacerbation caused by rhinovirus infection can lead to chronic obstructive pulmonary disease (COPD). Human rhinoviruses are therefore associated with COPD for which current therapy is inadequate. In any embodiment of claim 2 there is therefore provided a compound for the use in treating or preventing a viral mediated COPD comprising administering a TLR2 agonist to a subject, wherein the viral mediated COPD is caused by rhinovirus.

The terms 'chronic obstructive pulmonary disease' and 'COPD' as used interchangeably herein refer to a chronic disorder or combination of disorders characterized by reduced maximal expiratory flow and slow forced emptying of the lungs that does not change markedly over several months and is not, or is only minimally, reversible with traditional bronchodilators. Most commonly, COPD is a combination of chronic bronchitis, i.e. the presence of cough and sputum for more than three months for about two consecutive years, and emphysema, i.e. alveolar damage. However, COPD can involve chronic bronchitis with normal airflow, chronic bronchitis with airway obstruction (chronic obstructive bronchitis), emphysema, asthmatic bronchitis, and bullous disease, and combinations thereof. Chronic obstructive pulmonary disease is a condition usually but not exclusively resulting from chronic lung damage induced by exposure to tobacco smoke. Other noxious airborne pollutants, such as indoor cooking exhaust and car exhaust may over the long-term cause or increase the risk of COPD. COPD will therefore be understood to be interchangeable with terms such as "chronic bronchitis" and "emphysema."

The symptoms of COPD are progressively worsening and persistent breathlessness on exertion, eventually leading to breathlessness at rest. The most common symptoms of COPD are breathlessness (or a "need for air"), chronic cough, and sputum (mucous) production. Daily activities, such as walking up a short flight of stairs and even daily routine activities can become very difficult as the condition gradually worsens. Sufferers also frequently experience exacerbations, that is, serious episodes of increased breathlessness, cough and sputum production that last from several days to a few weeks. These episodes can be seriously disabling and result in need for urgent medical care (including hospitalisation) and sometimes death.

Chronic obstructive pulmonary disease is usually suspected in people who experience the symptoms described above and can be confirmed by a breathing test called "spirometry" that measures how much and how quickly a person can forcibly exhale air.

Respiratory viruses can also exacerbate disease in cystic fibrosis. For instance, viral infection of a subject diagnosed with cystic fibrosis can increase susceptibility to bacterial infections. In any embodiment of claim 2 there is therefore provided a compound for use in treating or preventing a viral mediated exacerbation of cystic fibrosis comprising administering a TLR2 agonist to a subject, wherein the viral mediated exacerbation is caused by a rhinovirus infection.

It will be understood that cystic fibrosis is a genetic disorder that affects the respiratory, digestive and reproductive systems involving the production of abnormally thick mucus linings in the lungs and can lead to fatal lung infections. It will be understood that a subject with cystic fibrosis may display a variety of symptoms including very salty-tasting skin; persistent coughing, possibly with phlegm, wheezing or shortness of breath, an excessive appetite but poor weight gain, and greasy, bulky stools. It will also be understood that the sweat test is the standard diagnostic test for cystic fibrosis. This procedure measures the amount of salt in the sweat. A high salt level indicates cystic fibrosis.

Respiratory viruses can also exacerbate disease in transplant recipient patients. For instance, viral infection in a lung transplant recipient can increase susceptibility to pneumonia, acute rejection and chronic allograft dysfunction. In any embodiment of claim 2 there is therefore provided a compound for use in treating or preventing a viral infection in a lung transplant recipient comprising administering a TLR2 agonist to a subject, wherein the viral infection is a rhinovirus infection.

The present invention also finds application in the restoration of anti-viral immunity in the context of chronic glucocorticosteroid use. It will be understood that a glucocorticoid is an agent having a cortisol-like agonist action on glucocorticoid receptors resulting in a range of endocrine and anti-inflammatory effects. The majority of patients with severe asthma and COPD take steroids or glucocorticosteroids. The use of steroids increases during a viral exacerbation which can prolong virus infection and increase susceptibility to secondary bacterial infection.

In any embodiment of the invention there is therefore provided a compound for use in treating or preventing a viral infection in a subject medicated with glucocorticosteroids comprising administering a TLR2 agonist to a subject, wherein the viral infection is a rhinovirus infection. Preferably the glucocorticosteroid administration is chronic.

As used herein, "preventing" or "prevention" is intended to refer to at least the reduction of likelihood of the risk of (or susceptibility to) acquiring a disease or disorder (i.e., causing at least one of the clinical symptoms of the disease not to develop in a patient that may be exposed to or predisposed to the disease but does not yet experience or display symptoms of the disease). Biological and physiological parameters for identifying such patients are provided herein and are also well known by physicians. For example, prevention of RV-induced respiratory infection or RV induced exacerbation of asthma may be characterised by a reduction or absence in viral load, or a reduction in an increase in inflammatory cell mediators or cytokines. In some embodiments, the administration of the compound may minimise development of the infection to minimise viral load. Preferably, this reduces viral load.

In any preventative or prophylactic aspect of the invention, the subject may not have any detectable symptoms of a rhinovirus infection, at the time of administration of the compound.

The terms "treatment" or "treating" of a subject includes the application or administration of a compound of the invention to a subject (or application or administration of a compound of the invention to a cell or tissue from a subject) with the purpose of delaying, slowing, stabilizing, curing, healing, alleviating, relieving, altering, remedying, less worsening, ameliorating, improving, or affecting the disease or condition, the symptom of the disease or condition, or the risk of (or susceptibility to) the disease or condition. The term "treating" refers to any indication of success in the treatment or amelioration of an injury, pathology or condition, including any objective or subjective parameter such as abatement; remission; lessening of the rate of worsening; lessening severity of the disease; stabilization, diminishing of symptoms or making the injury, pathology or condition more tolerable to the subject; slowing in the rate of degeneration or decline; making the final point of degeneration less debilitating; or improving a subject's physical or mental well-being. The method of treatment itself is not encompassed by the scope of the invention.

The existence of, improvement in, treatment of, or prevention of a respiratory infection or exacerbation (e.g. asthma exacerbation) may be determined by any clinically or biochemically relevant method as described herein or known in the art. A relevant method may be measurement of viral load, interferon expression or inflammatory cell numbers via bronchoalveolar lavage (BAL) in which in which a bronchoscope is passed through the mouth or nose into the lungs and fluid is squirted into a small part of the lung and then collected for examination. The improvement or treatment or prevention may be determined directly from the subject, or a sample or biopsy therefrom. The sample or biopsy may be of the upper or lower respiratory tract. Further, in a respiratory infection or asthma exacerbation, a positive response to therapy may be determined by measuring levels of chemokines and cytokines through known assays such as ELISA as shown herein.

Further, for example, in a respiratory infection or asthma exacerbation, a positive response to therapy would be to prevent a further decline in lung function as measured by spirometry, body plethysmography, and lung diffusion capacity. In particular to asthma exacerbation, a positive response to therapy would be an improvement from the initially diagnosed severity (as outlined in Table 1). For example, a subject diagnosed with a moderate exacerbation (FEV1 or PEF measurement is between 70% to 50%) will show a positive response to therapy when FEV1 or PEF values are higher than 45% of the predetermined value and PEF increases at least 50l/min 30 minutes after treatment is initiated.

A positive response to therapy may also be prevention or attenuation of worsening of respiratory symptoms, e.g. asthma symptoms (exacerbation), following a respiratory virus infection. This could be assessed by comparison of the mean change in disease score from baseline to end of study period based on Juniper Asthma Control Questionnaire (ACQ-6), and could also assess lower respiratory symptom score (LRSS - symptoms of chest tightness, wheeze, shortness or breath and cough) daily following infection/onset of cold symptoms. Change from baseline lung function (peak expiratory flow PEF) could also be assessed and a positive response to therapy could be a significant attenuation in reduced PEF. For example, a placebo treated group would show a significant reduction in morning PEF of 15% at the peak of exacerbation whilst the treatment group would show a non-significant reduction in PEF less than 15% change from baseline.

The present disclosure also describes a method of improving or maintaining the ability of a subject to control a respiratory disease during a respiratory RV infection, the method comprising administering a compound comprising a TLR2 agonist to the subject, thereby improving the ability of the subject to control the respiratory disease a respiratory viral infection. Improving or maintaining the ability to control a respiratory disease may be that the subject does not require any additional intervention to the treatment generally administered for the existing respiratory disease. In other words, the only treatments necessary for the subject is the treatment taken normally (i.e. when the subject does not have a viral infection) for the underlying respiratory condition and the compound comprising a TLR2 agonist as described herein.

Typically, a therapeutically effective dosage is formulated to contain a concentration (by weight) of at least about 0.1% up to about 50% or more, and all combinations and sub-combinations of ranges therein. The compositions can be formulated to contain one or more compounds, or a pharmaceutically acceptable salt, polymorph or prodrug thereof in a concentration of from about 0.1 to less than about 50%, for example, about 49, 48, 47, 46, 45, 44, 43, 42, 41 or 40%, with concentrations of from greater than about 0.1%, for example, about 0.2, 0.3, 0.4 or 0.5%, to less than about 40%, for example, about 39, 38, 37, 36, 35, 34, 33, 32, 31 or 30%. Exemplary compositions may contain from about 0.5% to less than about 30%, for example, about 29, 28, 27, 26, 25, 25, 24, 23, 22, 21 or 20%, with concentrations of from greater than about 0.5%, for example, about 0.6, 0.7, 0.8, 0.9 or 1%, to less than about 20%, for example, about 19, 18, 17, 1 6, 1 5, 14, 13, 12, 11 or 10%. The compositions can contain from greater than about 1% for example, about 2%, to less than about 10%, for example about 9 or 8%, including concentrations of greater than about 2%, for example, about 3 or 4%, to less than about 8%, for example, about 7 or 6%. The active agent can, for example, be present in a concentration of about 5%. In all cases, amounts may be adjusted to compensate for differences in amounts of active ingredients actually delivered to the treated cells or tissue.

Although the invention finds application in humans, the invention is also useful for therapeutic veterinary purposes. The invention is useful for domestic or farm animals such as cattle, sheep, horses and poultry; for companion animals such as cats and dogs; and for zoo animals.

The composition according to the present invention is to be administered in an effective amount. The phrase 'therapeutically effective amount' or `effective amount' generally refers to an amount of a TLR2 agonist, a pharmaceutically acceptable salt, polymorph or prodrug thereof of the present invention that (i) treats the particular disease, condition, or disorder, (ii) attenuates, ameliorates, or eliminates one or more symptoms of the particular disease, condition, or disorder, or (iii) delays the onset of one or more symptoms of the particular disease, condition, or disorder described herein. Undesirable effects, e.g. side effects, are sometimes manifested along with the desired therapeutic effect; hence, a practitioner balances the potential benefits against the potential risks in determining what is an appropriate "effective amount".

The exact amount required will vary from subject to subject, depending on the species, age and general condition of the subject, mode of administration and the like. Thus, it may not be possible to specify an exact "effective amount". However, an appropriate "effective amount" in any individual case may be determined by one of ordinary skill in the art using only routine experimentation. In one aspect, the dose administered to a subject is any dose that reduces viral load. Preferably, the dose does not significantly increase inflammation, for example does not significantly increase absolute neutrophil numbers or the proportion of neutrophils of total BAL cells in the lung.

In some embodiments, an effective amount for a human subject lies in the range of about 250 nmoles/kg body weight/dose to 0.005 nmoles/kg body weight/dose. Preferably, the range is about 250 nmoles/kg body weight/dose to 0.05 nmoles/kg body weight/dose. In some embodiments, the body weight/dose range is about 250 nmoles/kg, to 0.1 nmoles/kg, about 50 nmoles/kg to 0.1 nmoles/kg, about 5 nmoles/kg to 0.1 nmol/kg, about 2.5 nmoles/kg to 0.25 nmoles/kg, or about 0.5 nmoles/kg to 0.1 nmoles/kg body weight/dose. In some embodiments, the amount is at, or about, 250 nmoles, 50 nmoles, 5 nmoles, 2.5 nmoles, 0.5 nmoles, 0.25 nmoles, 0.1 nmoles or 0.05nmoles/kg body weight/dose of the compound. Dosage regimes are adjusted to suit the exigencies of the situation and may be adjusted to produce the optimum therapeutic dose.

The TLR2 agonists as described herein may be compositions formulated as inhaled formulations, including dry powder, sprays, mists, or aerosols. This may be particularly preferred for treatment of a respiratory infection. For inhalation formulations, the composition or combination provided herein may be delivered via any inhalation methods known to a person skilled in the art. Such inhalation methods and devices include, but are not limited to, metered dose inhalers with propellants such as CFC or HFA or propellants that are physiologically and environmentally acceptable. Other suitable devices are breath operated inhalers, multidose dry powder inhalers and aerosol nebulizers. Aerosol formulations for use in the subject method typically include propellants, surfactants and co-solvents and may be filled into conventional aerosol containers that are closed by a suitable metering valve.

Inhalant compositions may comprise liquid or powdered compositions containing the active ingredient that are suitable for nebulization and intrabronchial use, or aerosol compositions administered via an aerosol unit dispensing metered doses. Suitable liquid compositions comprise the active ingredient in an aqueous, pharmaceutically acceptable inhalant solvent such as isotonic saline or bacteriostatic water. The solutions are administered by means of a pump or squeeze-actuated nebulized spray dispenser, or by any other conventional means for causing or enabling the requisite dosage amount of the liquid composition to be inhaled into the patient's lungs. Suitable formulations, wherein the carrier is a liquid, for administration, as for example, a nasal spray or as nasal drops, include aqueous or oily solutions of the active ingredient. Alternatively, the composition may be a dry powder and administered to the respiratory tract as defined herein.

It will be understood, that the specific dose level for any particular patient will depend upon a variety of factors including the activity of the specific compound employed, the age, body weight, general health, sex, diet, time of administration, route of administration, and rate of excretion, drug combination (i.e. other drugs being used to treat the patient), and the severity of the particular disorder undergoing therapy.

A kit for use in a therapeutic and/or prophylactic application mentioned above may comprise:
- a container holding a therapeutic composition in the form of one or more TLR2 agonists as described herein, or a pharmaceutically acceptable salt, diluent or excipient or pharmaceutical composition;
- a label or package insert with instructions for use.

The kit may contain one or more further active principles or ingredients for treatment of a respiratory condition.

The kit or "article of manufacture" may comprise a container and a label or package insert on or associated with the container. Suitable containers include, for example, bottles, vials, syringes, blister pack, etc. The containers may be formed from a variety of materials such as glass or plastic. The container holds a therapeutic composition which is effective for treating the condition and may have a sterile access port (for example the container may be an intravenous solution bag or a vial having a stopper pierceable by a hypodermic injection needle). The label or package insert indicates that the therapeutic composition is used for treating the condition of choice. In one embodiment, the label or package insert includes instructions for use and indicates that the therapeutic or prophylactic composition can be used to treat a respiratory condition described herein.

The kit may comprise (a) a therapeutic or prophylactic composition; and (b) a second container with a second active principle or ingredient contained therein. The kit may further comprise a package insert indicating the composition and other active principle can be used to treat a disorder or prevent a complication stemming from a respiratory condition described herein.

### EXAMPLES

As used herein, including the following Examples, the following compounds are described in the table below and specific structures shown elsewhere herein. Compounds outside the scope of the appended claims are described and examined for illustrative purposes only.

| **Compound Structure** | **Compound name** |
|---|---|
| 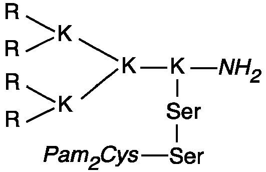 | INNA-001 |
| 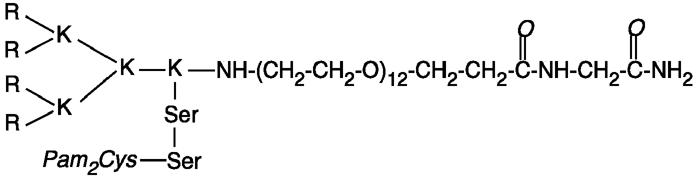 | INNA-002 |
| 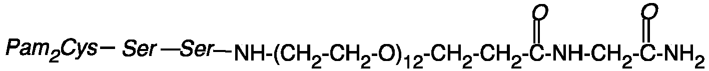 | INNA-003 |
| ***Pam₂Cys-Ser-Ser-Lys-Lys-Lys-Lys*** | INNA-004 |
| ***Pam₂Cys-Ser-Lys-Lys-Lys-Lys*** | INNA-005 |
| 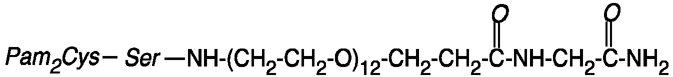 | INNA-006 (also shown herein as compound (1)) |
| 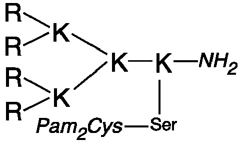 | INNA-007 |
| 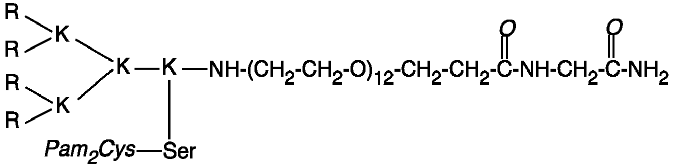 | INNA-008 |
| 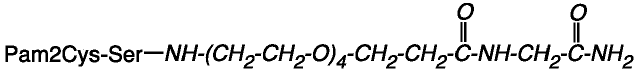 | INNA-009 |
| 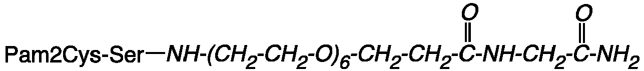 | INNA-010 |
| 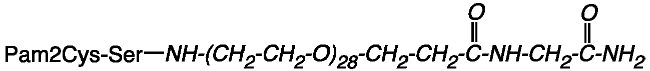 | INNA-011 (also shown herein as compound (5)) |
| 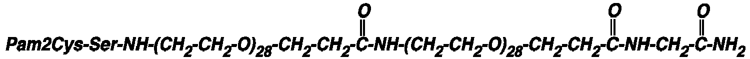 | INNA-012 (also shown herein as compound (6)) |
| 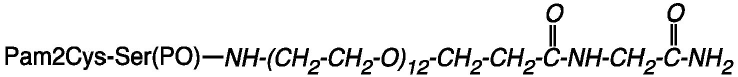 | INNA-013 (also shown herein as compound (4)) |
| 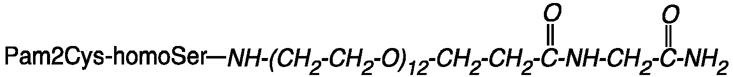 | INNA-014 (also shown herein as compound (3)) |
| 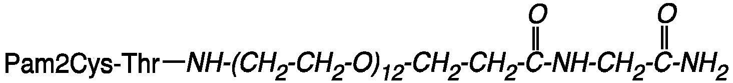 | INNA-015 (also shown herein as compound (2)) |

### Example 1

### Inhibition of rhinovirus infection in a mouse model

This study was conducted to determine if activation of the innate immune system by a TLR2 agonist reduces viral load and virus-induced inflammation during rhinovirus infection in mice.

### Animals

Female 6-8 week old BALB/c mice were used for all studies. Each group contained 5 mice. After treatment or challenge procedures, mice were monitored daily for weight changes, and behavioural or physical changes as stipulated in animal ethics approval for project A-2016-605. At the time of sample collection, all mice were sacrificed with intraperitoneal administration with sodium pentobarbital. All mice were housed in HMRI Bioresources facility in individually ventilated cages with not more than four mice per cage. Mice were observed daily from the beginning of each study and a health checklist maintained.

### Mouse surgical procedures and treatments

Rhinovirus serotype 1B was originally purified from a clinical isolate, was grown in RD-ICAM cells and purified as previously described (Bartlett et al., Nat Med (2008) 14, 199-204; Bartlett et al., Methods Mol Biol (2015) 1221, 181-188). Mice were dosed with 50 µl of agonist molecules intranasally under light isofluorane anaesthesia in an induction chamber within a class II biosafety cabinet. At indicated times post TLR-2 agonist PEG-Pam2Cys-R4 and Pam2Cys-R4 was administered to mice intranasally with 50 µl containing 5 × 106 TCID50 of RV1B using the same procedure. At day 2 post-infection, bronchoalveolar lavage (BAL) was performed to count inflammatory cell infiltrate and measure immune mediator protein expression. Lungs were harvested for total RNA to assess viral loads. The mouse RV infection model and associated techniques have been previously developed (Bartlett et al., Nat Med (2008) 14, 199-204; Bartlett et al., Methods Mol Biol (2015) 1221, 181-188). Experimental groups are shown in Table 2.

**Table 2: Experimental groups**

| **Total Mice 5 per group** | **Treatment groups** | **Administration protocol (intranasal** | **Time of challenge** | **Time of harvest** |
|---|---|---|---|---|
| 1A 35 (N=5 per group) | Saline control | Lung prophylaxis with three dose levels (day -1) | RV1B at day 0 | Harvest day 2. |
| | PEG-Pam2Cys-R4 5nmol | | | Analysis of viral titres in lung tissue by qPCR and / or inflammatory responses in BAL and lung tissue |
| | PEG-Pam2Cys-R4 1nmol | | | |
| | PEG-Pam2Cys-R4 0.1nmol | | | |
| | Pam2Cys-R4 5nmol | | | |
| | Pam2Cys-R4 1nmol | | | |
| | Pam2Cys-R4 0.1nmol | | | |
| 1B 35 (N=5 per group) | Saline control | Lung prophylaxis with three dose levels (day -7) | RV1B at day 0 | Harvest day 2. |
| | PEG-Pam2Cys-R4 0.1nmol | | | Analysis of viral titres in lung tissue by qPCR and inflammatory responses in BAL and lung tissue |
| | PEG-Pam2Cys-R4 0.05nmol | | | |
| | PEG-Pam2Cys-R4 0.01nmol | | | |
| | Pam2Cys-R4 0.1nmol | | | |
| | Pam2Cys-R4 0.05nmol | | | |
| | Pam2Cys-R4 0.01nmol | | | |
| 1C 35 (N=5 per group) | Saline control | Lung prophylaxis with three dose levels (day -7) | RV1B at day 0 | Harvest day 2. |
| | PEG-Pam2Cys-R4 5nmol | | | Analysis of viral titres in lung tissue by qPCR and inflammatory responses in BAL and lung tissue |
| | PEG-Pam2Cys-R4 1nmol | | | |
| | PEG-Pam2Cys-R4 0.1nmol | | | |
| | Pam2Cys-R4 5nmol | | | |
| | Pam2Cys-R4 1nmol | | | |
| | Pam2Cys-R4 0.1nmol | | | |
| 1D 50 (N=5 per group) | Saline control | Lung prophylaxis with three dose levels (day -7) | RV1B at day 0 | Harvest day 2. |
| | PEG-Pam2Cys-R4 10pmol | | | Analysis of viral titres in lung tissue by qPCR and inflammatory responses in BAL and lung tissue |
| | PEG-Pam2Cys-R4 5pmol | | | |
| | PEG-Pam2Cys-R4 2pmol | | | |
| | PEG-Pam2Cys-R4 1pmol | | | |
| | Pam2Cys-R4 10pmol | | | |
| | Pam2Cys-R4 5pmol | | | |
| | Pam2Cys-R4 2pmol | | | |
| | Pam2Cys-R4 1pmol | | | |
| IE | Saline/uninfected control | day -7, | PBS | Harvest day 2. |
| 70 (N=5/ group) | Saline/RV | day -7, | RVIB | Analysis of viral titres in lung tissue by qPCR and inflammatory responses in BAL |
| | Peg-SS-Pam2Cys/RV | day -7 | RV1B | and lung tissue |
| (Indicative dose shown only - dose regimen will be determined from results of studies 1C and ID) | Peg-S-Pam2Cys/RV | day -7 | RV1B | |
| | Pam2CysSK4/RV | day -7 | RV1B | |
| | 10pmol | | | |
| | Peg-SS-Pam2Cys/RV | day -7 | RV1B | |
| | Peg-S-Pam2Cys/RV | day -7 | RV1B | |
| | Pam2CysSK4/RV | day -7 | RV1B | |
| | 5pmol | | | |
| | Peg-SS-Pam2Cys/RV | day -7 | RV1B | |
| | Peg-S-Pam2Cys/RV | day -7 | RV1B | |
| | Pam2CysSK4/RV | day -7 | RV1B | |
| | 2pmol | | | |
| | Peg-SS-Pam2Cys/RV | day -7 | RV1B | |
| | Peg-S-Pam2Cys/RV | day -7 | RV1B | |
| | Pam2CysSK4/RV | day -7 | RV1B | |
| | 1pmol | | | |
| 1F | Saline/uninfected control | day -7, -1 | PBS | Harvest day 2. |
| 70 (N=5/ group) | Saline/RV | day -7, -1 | RV1B | Analysis of viral titres in lung tissue by qPCR and inflammatory responses in BAL and lung tissue |
| All drug treatments with the minimal effective dose (90% inhibition of | Peg-SS-Pam2Cys /PBS | day -7, -1 | PBS | |
| | Peg-SS-Pam2Cys /PBS | day -7 | PBS | |
| | Peg-SS-Pam2Cys /PBS | day -1 | PBS | |
| viral load in the lungs of treated animals as determined by study 1D and 1E | | | | |
| | Peg-SS-Pam2Cys /RV | day -7, -1 | RV1B | |
| | Peg-SS-Pam2Cys /RV | day -7 | RV1B | |
| | Peg-SS-Pam2Cys /RV | day -1 | RV1B | |
| Dose: 2pmol | | | | |
| | Peg-S-Pam2Cys /PBS | day -7, -1 | PBS | |
| | Peg-S-Pam2Cys /PBS | day -7 | PBS | |
| | Peg-S-Pam2Cys /PBS | day -1 | PBS | |
| | Peg-S-Pam2Cys /RV | day -7, -1 | RV1B | |
| | Peg-S-Pam2Cys /RV | day -7 | RV1B | |
| | Peg-S-Pam2Cys /RV | day -1 | RV1B | |
| 1G | Saline/uninfected control | day +1, | PBS | Harvest day 2. |
| 50 (N=5/ group) | Saline/RV | day +1, | RV1B | Analysis of viral titres in lung tissue by qPCR and inflammatory responses in BAL and lung tissue |
| | PEG-PAM2CYS-R4/RV | day +1 | RV1B | |
| | PAM2CYS-R4/RV 0.01 nmol | day +1 | RV1B | |
| | PEG-PAM2CYS-R4/RV | day +1 | RV1B | |
| | PAM2CYS-R4/RV 5pmol | day +1 | RV1B | |
| | PEG-PAM2CYS-R4/RV | day +1 | RV1B | |
| | PAM2CYS-R4/RV 2pmol | day +1 | RV1B | |
| | PEG-PAM2CYS-R4/RV | day +1 | RV1B | |
| | PAM2CYS-R4/RV | day +1 | RV1B | |
| | 1pmol | | | |

### Bronchoalveolar lavage (BAL) cell analysis

Following sacrifice, mice were tracheally cannulated and 1ml of Hanks buffered saline solution (HycloneTM, GE Life Sciences) flushed through the airways, 3-5 times. BAL cells were pelleted by centrifugation and supernatant was collected and stored at - 80°C for ELISAs. The pelleted cells were red blood cells lysed and the remaining cells were counted on a heamocytometer by trypan blue exclusion. Cell suspensions were then cytocentrifuged onto slides and fixed and stained with Diff Quick (POCD) solutions as per manufacturer's recommendations. A minimum of 200 total cells were counted per slide and numbers of neutrophils, lymphocytes, and macrophages were determined.

### RNA extraction and qRT-PCR

The apical lung lobe from each mouse was collected into RNA-later (Ambion). For processing, lung lobes were transfer into RLT (Qiagen)/2ME buffer for tissue dissociation by TissueLyser II (Qiagen) at 25Hz, twice for 2 minutes (with sample rotation). Cell debris was pelleted by centrifugation and RNA was manually extracted using a miRNeasy kit (Qiagen) following the recommended suppliers protocol for extracting total RNA, including miRNA from animal and human cells and tissues. Following extraction, RNA concentration was determined using spectrophotometry (Nanodrop) and 200ng of RNA used for reverse transcription with random primers and RNase-inhibtor (AB, Applied Biosystems). cDNA was then subsequently used for qPCR analyses on an ABI700 using TaqMan, FAM-TAMRA chemistry (Life Technologies) with mastermix containing ROX (Qiagen), primers and probes outlined in Table 3. Ct values for genes of interest where referenced to a seven standards of known concentration, starting with 107 copies and proceeding in 1:10 dilution series. Copy numbers for all genes of interest were normalised to the reference gene 18s.

### Quantification of cytokines by ELISA

Remaining lung lobes we snap frozen in liquid nitrogen, homogenised in 600µl PBS containing protease inhibitors (Roche) by TissueLyser II run twice at 30Hz for 4 minutes. Cell debris was pelleted by centrifugation, samples were diluted 1:2 with PBS and stored at -80°C. BAL fluid was then analysed for production of KC/IL-8 (CXCL1) and TNF-α by Duoset ELISA (R&D Systems) as per manufacturer's instructions.

**Table 3. Primer and probe sequences for qPCR**

| **Gene** | | **Sequence (5'-3')** | | |
|---|---|---|---|---|
| | | **Forward** | **Reverse** | **Probe** |
| ***18s*** | CGCCGCTAGAGGT GAAATTCT | | CATTCTTGGCAAATGCTTTC G | FAM- ACCGGCGCAAGACGGACCA GA-TAMRA |
| ***Rhinovirus*** | GTGAAGAGCCsCrT GTGCT | | GCTsCAGGGTTAAGGTTAG CC | FAM- TGAGTCCTCCGGCCCCTGA ATG-TAMRA |

qPCR analyses conducted using TaqMan chemistry in a total volume of 12.5ul per reaction with optimal, custom forward/reverse primer ratios on cDNA generated from RNA extracted from the apical lung lobe of each mouse.

### Statistical analysis

For comparisons between cohorts of mice treated with either saline RV control, Pam2Cys-R4 or PEG-Pam2Cys-R4, one-way ANOVA analysis was conducted. A P value of <0.05 was considered significant.

### Results

Mice were dosed intranasally to the total respiratory tract (50µl) with a range of doses of PEG-Pam2Cys-R4 and Pam2Cys-R4 as indicated (see Table 2). Following treatment mice were infected intranasally with RV1B. Viral loads in the respiratory tract were determined by qPCR analysis of viral RNA and lung inflammation determined by differential staining of BAL inflammatory cells and measurement of protein immune mediators in BAL-fluid.

### Study 1A Treatment 1 day before infection

Mice were treated with indicated amount of PEG-Pam2Cys-R4 and Pam2Cys-R4 one day before intranasally infection with RV. Controls not dosed with TLR agonists were treated with saline (Figure 1a). Lung viral load was assessed by qPCR. We observed a significant reduction in viral load for all doses tested (Figure 1b).

### Study 1C Treatment 7 days before infection

Study 1C was completed at the same time as 1A. Mice were treated with the indicated amount of PEG-Pam2Cys-R4 and Pam2Cys-R4 seven days before intranasally infection with RV (Figure 2a). Controls not dosed with TLR-2 agonist were treated with saline. Agonist treatment with all doses resulted in highly significant reduction in viral load compared to saline treated, RV infected controls (Figure 2b).

Analysis of BAL cells at day 2 p.i. indicated that all treatments significantly increased the total number of inflammatory cells, the majority of which were macrophages, although increased numbers of lymphocytes were observed at lower agonist treatment doses (Figure 3a-b). Inflammatory cytokines in BAL were measured by ELISA. Significantly reduced production of the neutrophil recruiting chemokine CXCL1 were observed for all treatment groups compared to saline treated infected mice (Figure 4a). Reduced expression of TNFα was also observed for the higher doses of agonist treatment groups compared to saline treated infected controls (Figure 4b).

### Study 1B and 1D Low dose treatment 7 days before infection

Having demonstrated a potent, long lasting anti-viral effect associated with reduced expression of inflammatory cytokines the design of study 1C was modified to determine if anti-viral efficacy could be maintained at lower doses. Starting at the lowest dose from previous studies (0.1mnoles/mouse) additional groups were treated with 0.05nmoles/mouse and 0.01nmoles/mouse of Pam2Cys-R4 or PEG- Pam2Cys-R4 at 7 days before viral treatment (Study 1B) or with 10pmoles/mouse, 5pmoles/mouse, 2pmoles/mouse or 1pmol/mouse of Pam2Cys-R4 or PEG- Pam2Cys-R4 at 7 days before viral treatment (Study 1D). No weight loss was observed by the end of Study 1B or 1D. We measured lung tissue RV RNA to determine if reduced inflammation was associated with lower viral load (Figure 5a-b). All doses of PEG-Pam2Cys-R4 inhibited RV replication. Pam2Cys-R4 also caused a significant reduction in viral RNA at the indicated doses.

For immune cells, Pam2Cys-R4 and PEG-Pam2Cys-R4 caused significant increases in the recruitment of cells following treatment with the indicated doses (Fig 6a, d). Increased BAL cells were primarily driven by increased macrophage numbers (Fig 6b,e). Significantly increased numbers of lymphocytes were also observed with the indicated doses (Figure 6c,f). As shown in Figure 6c, lymphocytes constituted approximately 10% of the total BAL cells in response to the indicated doses. Neutrophilic inflammation is a hallmark feature of viral asthma exacerbations and related to disease severity. A clinically significant reduction in viral load would be expected to be associated with reduced viral airway neutrophilic inflammation. Compared to saline treated RV infected mice there was a significant reduction in neutrophils when expressed as a percentage of total BAL cells, or absolute numbers of total BAL cells, at the indicated doses (Figure 7a-c). To provide further evidence of TLR-2 agonist-mediated suppression of virus induced inflammation we measured the level of neutrophil recruiting chemokine (CXCL1) and inflammatory cytokine TNFα in BAL for both Study 1B and 1D. Viral replication drives CXCL1 expression so this data supports virus replication being suppressed with TLR-2 agonist treatment. For all doses of TLR-2 agonist, a highly significant reduction in CXCL1 expression was observed when compared to untreated RV infected controls (Figure 8a, b). Treatment had no effect on TNFα production which confirms that treatment was not causing activation of inflammatory pathways (Figure 8c, d).

### Study 1E Comparison of treatment of (i) Peg-SS-Pam2Cys, Peg-S-Pam2Cys and Pam2CysSK4; and (ii) INNA-011 and Peg-S-Pam2Cys 7 days before infection (dose range 1pmol- 10pmol).

Additional TLR2-agonists (Peg-SS-Pam2Cys and Peg-S-Pam2Cys) were next assessed. Having demonstrated potent, long lasting anti-viral effect associated with reduced expression of inflammatory cytokines with the lowest doses of Pam2Cys-R4 and Peg-Pam2Cys-R4, we sought to assess Peg-SS-Pam2Cys, Peg-S-Pam2Cys and INNA-011 at the similar doses. Accordingly, we treated mouse groups with 10pmoles/mouse, 5pmoles/mouse, 2pmoles/mouse and/or 1pmole/mouse 7 days prior to infection (or 2pmol in the case of INNA-011). A comparison with the commercially available Pam2CysSk4 molecule was also conducted using the same doses.

Mouse weights over time were then assessed. Mouse weight data over time was noticeably clustered between the various groups. At baseline (day -7) there was a significant different between saline RV controls and the 1pmol Peg-SS-Pam2Cys group (p=0.046 One-way ANOVA) and there was a trend towards lower weight within the 1pmol Peg-S-Pam2Cys group (p=0.091 One way ANOVA) at the time of treatment. Except for the 1pmol dose of Pam2CysSK4, all mouse groups displayed a trend to weight gain or no changes to weight were observed (data not shown). There were significant differences between 1pmol Peg-S-Pam2Cys and saline RV control mice at day -4 and day 1 post-infection, however, this is likely due to tight clustering of mouse weight and not due to weight loss induced by drug treatment (data not shown).

To assess the antiviral efficacy of the defined TLR-agonists, RV copy number was quantified in lung lysates from the apical lobe of the tri-lobe lung by Taqman qPCR. Every dose of TLR-agonist resulted in significant reduction in RV infection. Peg-S-Pam2Cys suppression appeared to be dose dependant. Peg-SS-Pam2Cys and Peg-S-Pam2Cys have better anti-viral efficacy compared to Pam2CysSK4 at a 10pmol dose. Peg-SS-Pam2Cys and Peg-S-Pam2Cys reduced RV-infection by -85% (84.82% with 10pmol Peg-SS-Pam2Cys and 86.76% with Peg-S-Pam2Cys) compared to only a 58% reduction with the same dose of Pam2CysSK4 (p=0.0246 by one way ANOVA) (Figure 9a). Notably, treatment with INNA-011 reduces RV lung RNA to the same extent as Peg-SPam2Cys (INNA-006) (9(a)(ii)).

Lung inflammation as assessed by total leukocytes in BAL showed that there was no significant difference between any of the drug treatments, compared to Saline RV controls (Figure 9b). In this experiment, differential cell counts were not possible due to loss of cells either at the point of cytospin preparation (cells not attaching to slides) or at the point of staining (cells detaching from the slides when submerged in fixing or staining solutions). Because of this, the fixing and staining solutions were discarded and replaced. The cytocentrifuge settings were also changed to increase the relative centrifugal force (300rpm to 500rpm) to ensure successful differential BAL leukocytes in future experiments. An assessment of neutrophils in BAL cells at 2 days post-infection demonstrated that INNA-011 and Peg-S-Pam2Cys (INNA-006) reduced RV-induced neutrohillic inflammation (Figure 9c).

Treatment with Peg-SS-Pam2Cys, Peg-S-Pam2Cys or Pam2CysSK4 reduced the levels of CXCL1, the key neutrophil chemokine produced in response to RV infection (Figure 9d-e). Every dose of Peg-SS-Pam2Cys, as well as the 10pmol and 5pmol dose of Peg-S-Pam2Cys and Pam2CysSK4 compounds effectively reduced CXCL1 levels. Further, INNA-011 and Peg-S-Pam2Cys (INNA-006) reduced RV-induced expression of CXCL1. TNF-α was not increased by any of the compounds at this, providing evidence the defined TLR-agonists do not promote inflammation.

### Study 1F Combined drug timing interaction and effect upon infection.

To determine if there is synergistic effect on anti-viral response and inflammation, mice were prophylactically dosed either 7 days and/or 1 day prior to infection with 2pmol of either Peg-SS-Pam2Cys or Peg-S-Pam2Cys. One group was specifically dosed 7 days and 1 day prior to infection. Following treatment/s mice were infected intranasally with RV1B (or mock treated) and we recorded mouse weights (measurements in grams or percentage change from baseline), assessed inflammation in BAL and quantified viral loads in the respiratory tract. Mouse weight measurements and weight change compared to baseline (day -7) indicated that as observed previously, 2pmol dose of either compound did not induce weight loss at either time point (data not shown). More importantly, there was no weight loss observed when mice were treated with a second dose of the TLR-agonists (6 days after first dose on the day prior to infection).

In order to test the effect of the timing of administration of these TLR-agonists, separate groups of mice were intranasally treated with 2pmol of Peg-SS-Pam2Cys or Peg-S-Pam2Cys either 7 days post infection, 1 day post infection, or a combination of 7 and 1 days prior to infection. Mice were then intranasally inoculated with mock or RV1B. Lung Inflammation in BAL was assessed 2 days after infection.

To test whether the increase in lung inflammation was attributed to increased viral load, or was caused by the drug, RV copy numbers in the lung were assessed by qPCR. Every drug treated group had very significant reduction in viral copy numbers and the combination of day-7 and day-1 treatment with Peg-S-Pam2Cys enhanced viral clearance compared to mice dose only on day 7 (Figure 10a-b).

BAL neutrophils were only significantly increased in mice with Peg-SS-Pam2Cys treatment 1 day prior to RV or mock infection. However, lymphocytes numbers were induced by Peg-SS-Pam2Cys at all treatment times (except day -7 treatment with RV1B infection). The combination of treatment with Peg-S-Pam2Cys 7 days prior, with 1 day prior to infection also promoted lymphocyte recruitment in the BAL (Figure 10c-d).

Total leukocytes were increased in mock control mice given a day -1 dose of Peg-SS-Pam2Cys, in mock mice given Peg-SS-Pam2Cys at both -7 and -1 time points and in mice given a day -7 Peg-S-Pam2Cys treatment compared to Saline mock controls (Figure 10e-f). Interestingly, the same dose regimes in mice infected with RV1B did not induce significantly higher leukocyte recruitment when compared to Saline RV controls. Contrary to previous experiments, the increase in total BAL leukocytes is not due to macrophage recruitment. Only day -7 Peg-S-Pam2Cys mock group has higher numbers of macrophages compared to its saline mock control group.

Interestingly, despite pronounced neutrophilic inflammation in the Peg-SS-Pam2Cys d-7/Mock group, CXCL1 production was only increased in Peg-SS-Pam2Cys d-1/RV1B and Peg-S-Pam2Cys d-1/RV1B groups. It is also important to note that mice already treated on day -7 were protected from lung inflammation induced by administration of either Peg-SS-Pam2Cys or Peg-S-Pam2Cys on day -1 (Figure 10g-h). Consistent with previous experiments, Peg-SS-Pam2Cys or Peg-S-Pam2Cys did not induce TNF-α in any of the groups.

### Study 1G Treatment during RV infection

Mice were infected intranasally with RV1B and at 1 day post-infection mice were treated with a 10, 5, 2 or 1 pmol dose of Peg-SS-Pam2Cys or Peg-S-Pam2Cys to assess therapeutic antiviral efficacy and the interaction with established RV infection on pulmonary inflammation. After RV infection, mouse weights were recorded and viral loads and inflammation in the respiratory tract were determined.

Administration of TLR-agonists during infection significantly reduced RV copy numbers in the lung (Figure 11a). Study 1G and 1F (TLR-agonist dosing at day-1 and day 1 post infection) therefore delineate inflammation from viral load.

There were no significant differences between total leucocyte numbers in mice treated with Peg-SS-Pam2Cys or Peg-S-Pam2Cys during active infection compared to infected mice treated with saline (Saline RV) or RV and mock controls. However, Peg-SS-Pam2Cys and Peg-S-Pam2Cys both changed the profile of BAL leukocytes, significantly reducing macrophage numbers and increasing neutrophil recruitment (Figure 11b-e). Unlike previous studies, there were no changes to lymphocyte numbers.

Neutrophilic inflammation in the BAL was also associated with production of neutrophil chemokine, CXCL1 and the inflammatory cytokine, TNF-α, both of which were dose dependent upon drug treatment (Figure 11f-g). Importantly, CXCL1 and TNF- α are not increased by the lowest doses of Peg-SS-Pam2Cys or Peg-S-Pam2Cys.

### Discussion

This *in vivo* program of work was carried out in parallel with *in vitro* experiments in RV-infected primary bronchial epithelial cells. *In vitro* data provide evidence of anti-viral effect of the defined TLR2 agonists. The aim of the mouse studies was to determine if the candidate TLR2 agonists (Pam2Cys-R4, Peg-Pam2Cys-R4, Peg-SS-Pam2Cys and PegS-Pam2Cys) are anti-viral against RV *in vivo* when administrated to the lower respiratory tract and if suppression of viral infection provided evidence of clinical benefit by reducing virus-induced airway inflammation.

In study 1A mice and 1B mice were dosed with 0.1nmoles, 1.0nmoles and 5nmoles per mouse. Dosing 7 days before infection (study 1B) the lowest dose (0.1nmoles) of Peg-Pam2Cys-R4 did not cause significant weight loss identifying a potential positive effect of pegylation on the systemic effects of Pam2Cys. For both dosing regimens (day -1 and day -7) there was evidence of agonist induced cellular inflammation however this was associated with reduced viral loads in both studies. Treatment at 7 days before infection provided impressive reduction in viral load (>90% reduced viral RNA).

All doses for study 1B caused immune activation. This consisted predominantly of macrophages likely involved in the resolution of neutrophilic inflammation. For lower doses (1 and 0.1nmoles) there was evidence of increased lymphocyte recruitment indicative of lower numbers of neutrophils and resolution of neutrophilic inflammation in these groups. Cytokine data supported this. TLR2 agonist treatment 7 days before infection reduced the level of virus induced neutrophil chemokine CXCL1. Higher doses also reduced expression of TNFα. This study showed for the first time that prophylactic treatment (7 days before infection) with a TLR2 agonist could inhibit infection and this was associated with a reduction in virus induced inflammatory mediators.

Studies 1A (dosing 1 day before infection) and 1B (dosing 7 days before infection) were conducted with the same group of mice. Based on the weight loss and inflammatory profile we decided to reduce the dosing range for study 1C (0.1 to 0.01nmoles/mouse), 7 days before infection with rhinovirus. Neither drug caused weight loss when 0.01nmoles/mouse were given. The data indicated that the pegylated form was better tolerated in terms of impact on weight loss.

Assessment of inflammatory cells in study 1C revealed modest two-fold or less increase in total BAL cells, which were predominantly macrophages. Macrophages are important in the resolution of neutrophilic inflammation and may be mechanistically involved in agonist-mediated control of virus-induced inflammation in this model. Again Peg-Pam2Cys-R4 was less inflammatory with no increase in total BAL cells following treatment with 0.05nmoles/mouse and 0.01nmoles/mouse. A low level, but significant lymphocyte signal was apparent except in the lowest dose of the Peg-Pam2Cys-R4 treated group. Neutrophils are a key readout of viral inflammation. We observed near significant reduction in BAL neutrophils with agonist treatment. Given the consistency of the trend for reduced neutrophils we are confident that a repeat study to increase dataset size will show this effect (>50% reduction) to be statistically significant. The peak of BAL neutrophils in the mouse RV infection model is 1 day post-infection so future studies focusing on control of viral neutrophilic inflammation by the TLR2 agonist are likely to obtain a clearer signal if assessed a day earlier.

Consistent with reduced neutrophils, agonist treatment was highly effective at suppressing CXCL1 expression. There was no effect on TNFα which confirmed that treatment was not causing significant activation of inflammatory pathways. Viral replication drives innate immune activation and CXCL1 expression so this data supports virus replication and infection induced inflammation being suppressed with TLR2 agonist treatment. Analysis of viral RNA confirmed this with Peg-Pam2Cys-R4 treatment inducing a significant reduction in viral load at all doses. The highest dose only (0.1nmoles/mouse) of Pam2Cys-R4 provided a significant reduction in virus load.

Having completed proof of concept studies with Pam2Cys-R4 and Peg-Pam2Cys-R4 we next focused on Peg-SS-Pam2Cys,Peg-S-Pam2Cys and INNA-011. Apart from a transient reduction in weight gain with the highest treatment dose (10pmoles per mouse), Peg-SS-Pam2Cys and Peg-S-Pam2Cys did not affect mouse weights. The lack of clinically deleterious inflammation was consistent with lack of induction of TNFα and significantly reduced neutrophilic inflammation - caused by highly significant reduction (>80%) in viral load. The potency of Peg-SS-Pam2Cys and Peg-S-Pam2Cys in terms of anti-viral effect were similar and superior to Pam2CSK4 which reduced lung vial load by 50%. These data confirmed that Peg-SS-Pam2Cys and Peg-S-Pam2Cys potently suppressed viral inflammation when administered seven days prior to infection with RV. Further, INNA-011 also yielded a significant inhibitory effect on viral inflammation.

We next investigated the interaction of multiple doses of Peg-SS-Pam2Cys and - Peg-S-Pam2Cys and proximity of dosing to RV infection. In terms of lung inflammation (examining response to drug treatment only) in uninfected mice three days after dosing (d-1 groups) there was a neutrophilic response to Peg-SS-Pam2Cys (not Peg-SPam2Cys). This response was reduced if preceded by treatment six days earlier. The experiment therefore identified an unexpected effect of multiple doses whereby the primary dose reduced the inflammatory response to the secondary dose. One possible explanation for this is the induction of inflammation resolving pathways such as anti-inflammatory macrophages phagocytosing apoptotic neutrophils operating when the second agonist is given. This same experiment also identified a difference between Peg-SS-Pam2Cys and Peg-S-Pam2Cys in terms of inflammatory potency (Peg-SS-Pam2Cys more inflammatory). CXCL1 levels were significantly increased in mice treated one day prior to infection but this effect was completely abolished if the mice had received prior treatment on day -7. Despite suppressed inflammation following multiple treatments there was no loss of anti-viral immunity. In fact, treatment at both days -7 and -1 was more effective (approx. 90% reduction in viral RNA) than a single treatment at day -7 (70% reduced viral RNA).

In the final study we examined the therapeutic efficacy of Peg-SS-Pam2Cys and Peg-S-Pam2Cys given one day after RV1B infection (treatment protocol). There was clinical evidence of enhanced inflammation in terms of weight loss at the higher doses. This was mirrored by dose-dependent expression of inflammatory mediators and neutrophil recruitment. For the lower doses (2pmole and 1pmole) there was no significant increase in KC or TNFα above that induced by RV infection without treatment. There was a significant reduction in lung viral load for doses 5pmole per mouse and lower. The highest dose (10pmole per mouse) was less effective and not significant for Peg-SPam2Cys. This data is consistent with study 1A viral load data (Fig 4) which also indicated that the highest dose loses anti-viral effect. This is typical of a bell-shaped dose response curve and is often seen with mixed agonist-antagonists

In summary, this study shows for the first time that prophylactic treatment with a representative TLR-2 agonist can inhibit viral-mediated infection which is associated with a reduction in viral load, and viral-induced inflammatory mediators including the chemokine CXCL1. These studies demonstrate potent ant-viral activity against RV infection of the structurally diverse compounds comprising TLR2 agonists. Further, the anti-viral activity against rhinovirus infection can be achieved at agonist doses that do not cause clinical or immunopathological signals. These data also demonstrate that multiple doses of TLR agonists protect against the acute inflammatory effect of primary response to agonist treatment without compromising anti-viral activity. Further, post-infection treatment suppresses viral replication with as low as 1pmol per mouse, induces neutrophilia, but at low doses does not increase inflammatory cytokines.

Without being bound by any theory or mode of action, it appears that the mechanism of protection against infection potentially involves both non-immune (airway epithelium) and low level macrophage and lymphocyte activation.

### Example 2

### Protective and therapeutic effect of TLR2 agonist against rhinovirus infection in primary asthmatic bronchial epithelial cells

This study was conducted to determine if TLR2 agonist treatment or prevention reduces viral load and virus-induced immune mediators during rhinovirus infection in air-liquid interface (ALI)-differentiated human asthmatic bronchial epithelial cells.

### Air-liquid interface-differentiation of COPD patient primary bronchial epithelial cells

Primary bronchial epithelial cells obtained from 6 mild to moderate persistent asthmatic patients (Figure 12a) were grown until confluent (passage 3) in a T75 flask and differentiated at air liquid-interface (ALI). Briefly, primary cells were grown in complete BEGM (Lonza) with growth factor supplements in submerged monolayer culture and then seeded at 2 × 105 cells in transwells (Corning Cat# 3460) in a 12-well plate with ALI-initial media comprised of 50%BEBM / 50%DMEM containing 0.1% hydrocortisone, 0.1% bovine insulin, 0.1% epinephrine, 0.1% transferrin, 0.4% bovine pituitary extract (all from Lonza singlequots, Cat# CC-3171) and ethanolamine (final concentration 80 µM), MgCl2 (final concentration 0.3mM), MgSO4 (final concentration 0.4mM), bovine serum albumin (final concentration 0.5 mg/ml), aphotericin B (final concentration 250 ug/ml), all-trans retinoic acid (30 ng/ml) and 2% penicillin streptomycin with 10 ng/ml recombinant human epithelial growth factor (rhEGF) until confluent (at least three days in both apical and basal compartments). Once confluent, rhEGF concentration is changed to 0.5ng/ml during the ALI phase for differentiation in the basal compartment (beneath the transwell insert) without apical media until day 21 after initial seeding.

### Trans-epithelial electrical resistance readings

Trans-epithelial electrical resistance was measured using WPI EVOM - Epithelial Voltohmmeter with AC current through an STX2 chopstick electrode set, simultaneously placed in the apical media and the basal media. An average of three readings were recorded for each time point, starting at day zero (when seeded cells become confluent), continued weekly throughout growth and differentiation (at day 7, day 14 and 21) and then following differentiation, relative to the time of infection (-2 hrs, 0 hrs, 24 hrs, 48 hrs, 72 hrs and 96 hrs post infection), and resistance was expressed as Ohms (Ω) / cm2

### Sample collection from ALI cultures

ALI culture samples were collected at 48 and 96 hrs post-infection. At each time point, apical media was removed from the cultures and stored at -80°C for protein expression analyses and half of the transwell membrane was carefully cut from the inserts and collected into 350µl RLT buffer (Qiagen) containing 1% 2-Mercaptoethanol (2ME) for downstream molecular analyses by RT-qPCR while the remaining transwell membrane was reserved for protein analyses.

### RNA extraction and qRT-PCR

Transwell membranes in RLT/2ME buffer were pulse vortexed to remove and lyse cells from the membrane. Membranes were removed and RNA was extracted using a miRNeasy kit (Qiagen) following the recommended suppliers protocol for extracting total RNA, including miRNA from animal and human cells and tissues on the semi-automated Qiacube platform. Following extraction, RNA concentration was determined using spectrophotometry (Nanodrop) and 200ng of RNA used for reverse transcription with random primers and RNase-inhibitor (AB, Applied Biosystems). cDNA was then subsequently used for qPCR analyses on an ABI700 using TaqMan, FAM-TAMRA chemistry (Life Technologies) with mastermix containing ROX (Qiagen), primers and probes outlined in Table 3. Ct values for genes of interest where referenced to a seven standards of known concentration, starting with 107 copies and proceeding in 1:10 dilution series. Copy numbers for all genes of interest were normalised to the reference gene 18s.

qPCR analyses conducted using TaqMan chemistry in a total volume of 12.5ul per reaction with optimal, custom forward/reverse primer ratios on cDNA generated from RNA extracted from cell lysates from half of an air-liquid interface transwell membrane.
Virus stocks
   Virus infection at MOI 0.1 2hr adsorption
   RV1-B (May 2010 stock 1.55 × 108 TCID/ml)
   MOI 1 = 6.45ul RV1B + 243.55ul Minimal
   MOI 0.1 = 1/10 of MOI 1
   Prepare 7W (250ul each) = 175ul MOI 1 RV1B + 1575ul Minimal
Infection harvest times
   At time point:
   Re-read TEER (ensuring not to cross-contaminate virus treated samples)
   Remove apical supernatant
   Collect 500ul and store at -80°C
   Remove transwells into a collection plate containing 1ml PBS.
   Protein: follow GLP855 and AR method and extract protein from half membrane in 200ul protein lysis buffer (store at -80°C)
   RNA: follow GLP855 and extract RNA from half membrane in 350ul RLT lysis buffer (store at -80°C)

### Quantification of cytokine and interferon production

Apical supernatant from ALI cultures was analysed for production of IL-6 and IP-10 (CXCL10) by multiplexed cytometric bead array (CBA) as per manufacturer's instructions with BD CBA Flex sets (BD). Briefly, samples were brought to room temperature and 50ul of sample were mixed with multiplexed beads coated with either anti-human IL-6 or IP-10 and subsequently incubated with Phycoerythrin (PE) conjugated detection antibodies. Samples were run on a 96-well plate format, FACS Canto-II and, IL-6 and IP-10 coated beads were differentiated based APC and APC-Cy7 clustering and PE intensity of unknowns referenced to a standard curve of known concentrations using FCAP-Array (version 3) software. ELISA was used for quantification of IFN-λ, IL-8 and CCL22 (R&D systems Duoset) and IFN-β (PBL Assays) as per manufacturer's instructions.

### Statistical analysis

Unpaired T test non parametric (Mann Whitney) was used for all stats comparing a treatment with saline RV control. A P value of <0.05 was considered significant. The Friedman test was used for assessment of interferon expression and inflammatory mediator expression between saline RV control groups and treatment with Pam2Cys-R4 at the indicated doses.

### Results

To determine if Pam2Cys-R4 could induce an anti-viral response in asthmatic airway epithelial cells we generated fully differentiated epithelial cultures from five asthma patients. These cultures were treated with two doses of Pam2Cys-R4 (0.2µM or 0.02µM) in starvation media either 24 hours before infection (pre-treatment; preventative model), or 2 hours after RV infection (post-treatment; therapeutic model). Media was added to untreated cells. A consistent trend was observed for reduced viral RNA in treated cultures and this reached statistical significance for 0.02µM pre-treat and 0.2µM post-treat group at 96 hours post-infection (Figure 12b-e). These data suggest that the kinetics of the anti-viral response can be manipulated with dose.

RV replication generates viral RNA which activates pathogen pattern recognition receptors (PRRs), innate immunity and production of type I/III interferons (IFNβ/IFNλ). This process has been shown to be impaired in asthma, particularly in more severe forms of disease. To determine whether Pam2Cys-R4 treatment was inhibiting viral replication we measured virus-induced IFN production. We measured type I (IFNβ) and type III (IFNλ) protein levels in the apical media (Figure 13a-d). For all treatment conditions there was a trend that TLR-2 agonist treatment reduced the expression of IFN and this corresponded with the significant reduction in viral replication for the post-treat 0.2µM 96h group shown in Figure 12.

The agonist did induce production of inflammatory mediators IP-10, IL-6, IL-8 and CCL22 by uninfected cells and infected cells (Figure 14a-h). IL-6 exhibits both pro- and anti-inflammatory properties and its role in asthma is somewhat controversial. It is generally accepted that high levels are linked to asthma severity. IL-8 is a neutrophil chemokine and is another biomarker of severe acute asthma. CCL22 is a chemokine that binds to the CCR4 receptor on the surface of Th2 cells and type 2 innate lymphoid cells and is associated with type-2 inflammation in asthma. These data demonstrate that TLR-2 agonist treatment can reduce the peak and duration of infection without causing a major increase in inflammation, as demonstrated by the measurement of the defined inflammatory markers. Clinical studies have shown that peak and duration of viral load are associated with disease severity in asthma supporting the idea that decreasing viral replication will reduce disease severity.

We next conducted experiments to assess the anti-viral activity of the TLR2 agonist variants of Pam2Cys-R4, Peg-SS-Pam2Cys and Peg-S-Pam2Cys, in comparison to commercially available Pam2CSK4. Initial experiments were conducted using the human bronchial epithelial BCi-NS1 cell line. This is a minimally immortalised human bronchial epithelial cell line that constitutively expresses human telomerase reverse transcriptase. This cell line was derived from brushing airway epithelium of a healthy volunteer and retains characteristics of original primary cells for over 40 passages. A key characteristic retained by these cells is that they can be differentiated at the ALI. Cells were either untreated or pre-treated with Pam2Cys-R4, Peg-SS-Pam2Cys and Peg-S-Pam2Cys, or Pam2CysSK4 (CSK4) at the indicated doses. Cells were then infected with RV1B and viral RNA levels measured at 96 hr p.i. (Figure 15a-b). Compared to the control, treatment with Peg-S-Pam2Cys (20nM and 2nM) significantly reduced viral RNA levels by approximately 50% at 96 h post infection.

In summary, this study demonstrates that TLR-2 agonist treatment of fully differentiated asthmatic epithelium taken from human patients with differing severities of asthma inhibits viral replication and associated production of innate anti-viral mediators induced by viral replication. These results are significant because they show for the first time that a TLR2 agonist can suppress rhinovirus replication without the requirement to first trigger IFN production and IFN-mediated anti-viral responses.

The epithelial cells for this study have been acquired for patients with mild to moderate persistent asthma. Compared to cells from patients with severe disease these cells are more likely to have a fully functional anti-viral response with 'normal' interferon expression. Even so we were able to observe enhanced control of infection with agonist treatment.

Significantly, the anti-viral response does not rely on IFN-mediated responses. This is important because interferon expression is extremely variable, particularly in more severe forms of asthma. Thus controlling the therapeutic response to a TLR agonist that induces IFN (eg TLR3- or TLR7 agonist) could be problematic and could lead to no therapeutic effect or excess inflammation and associated side effects in clinical trials. Variability in response to recombinant IFN in asthma has been observed in previous clinical trials. A reduction in exacerbations was only observed in those trials in the severe sub-group, limiting the application of this treatment in asthma. As reduced viral loads in cells from mild and moderate persistent asthmatic patients were observed in the present experiments this suggests that targeting an anti-viral pathway not dependent on interferon may have wider application across asthma phenotypes.

Pam2Cys-R4 induced production of inflammatory mediators IL-6, IL-8 and CCL22 but not IP-10 in both infected and uninfected cells. Consistent with our results, TLR2 activation has been reported to induce epithelial expression of inflammatory cytokines and chemokines in other systems. IL-6 exhibits both pro- and anti-inflammatory properties and its role in asthma is controversial. It is generally accepted that high levels of IL-6 are linked to asthma severity. IL-8 is a neutrophil chemokine and is another biomarker of severe acute asthma. CCL22 is a chemokine that binds to the CCR4 receptor on the surface of Th2 cells and type 2 innate lymphoid cells and is associated with type-2 inflammation in asthma. Following treatment with Pam2Cys-R4, increased expression of these mediators was modest (generally less than 2 fold).

Alveolar macrophages are the predominant resident immune cell in the airways and BAL cells from healthy lungs are typically 85% macrophages. We assessed the response of BAL macrophages to concurrent stimulation with RV and Pam2Cys-R4 or Peg-Pam2Cys-R4 to identify the type and magnitude of inflammatory cytokines induced. We measured IL-6, IL-8 and TNFα. CXCL10 (IP10) was undetectable. For the majority of experiments RV challenge alone did not induce IL-6, IL-8 and TNFα. Given that macrophages are not permissive for RV infection, this was not unexpected.

Both BECs and macrophages expressed IL-6, IL-8 and TNFα in response to TLR2 activation. CXCL10 was expressed by epithelium but not by BAL macrophages in response to RV infection and/or TLR2 stimulation. This observation helps to understand the responses we might expect during human clinical studies - expression of IP10 will likely indicate that epithelium is being activated whereas expression of inflammatory cytokines in the absence of CXCL10 could indicate that the epithelium is not being engaged and immune cells (macrophages) are responding.

Having demonstrated the proof of concept experiments with Pam2Cys-R4 and Peg-Pam2Cys-R4 we progressed with candidate selection and assessed the anti-viral effect of structural analogues of Pam2Cys-R4 and Peg-Pam2Cys-R4, namely Peg-SS-Pam2Cys, Peg-S-Pam2Cys. Pam2Cys-R4 and commercially available TLR2 agonist Pam2CSK4 were used as controls.

The first round of experiments were carried out in the healthy human bronchial epithelial cell line BCi-NS1. These cells behave like primary cells in that they are able to from pseudostratified epithelium at the ALI. We confirmed that structurally related TLR2 agonist compounds exhibit potent anti-viral activity. In conclusion, these studies demonstrate the ability of representative TLR-2 agonists to act as an anti-viral against RV infection in asthmatic epithelial cells.

### Example 3

### Synthesis of INNA-003 and INNA-006

### Synthesis of INNA-003 and INNA-006

**Reagents:** Solid phase support: TentaGel S RAM resin (substitution factor 0.24mmol/g; Rapp Polymere, Tübingen, Germany). **Amino acid derivatives:** Fmoc-Gly-OH, Fmoc-Ser(tBu)-OH, Fmoc-homo-Ser(tBu)-OH, Fmoc-Ser(PO(OBzl)OH)-OH, Fmoc-Thr(tBu)-OH, Fmoc-NH-(PEG)₃-COOH, Fmoc-NH-(PEG)₅-COOH, Fmoc-NH-(PEG)₁₁-COOH, Fmoc-NH-(PEG)₂₇-COOH from Merck (Darmstadt, Germany).

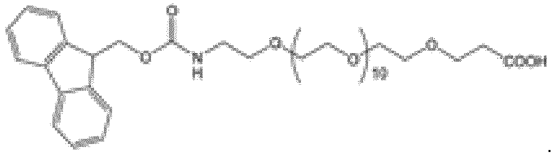

**NB** use of Merck catalogue number 851024 gives rise to the structures shown below as "INNA-003" (which may also be referred to herein as Pam2Cys-SS-PEG) and "INNA-006" (which may also be referred to herein as Pam2Cys-S-PEG).

### INNA-003:

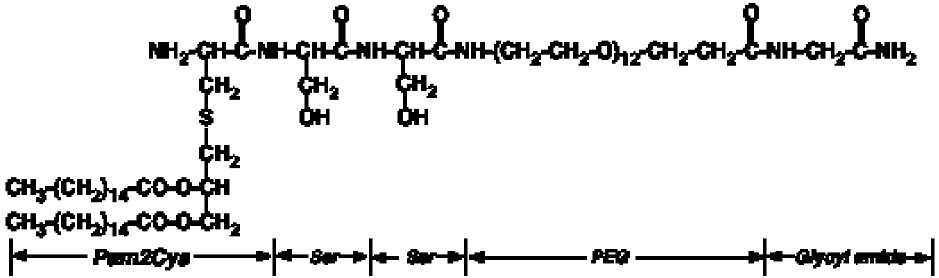

### INNA-006, or compound (1):

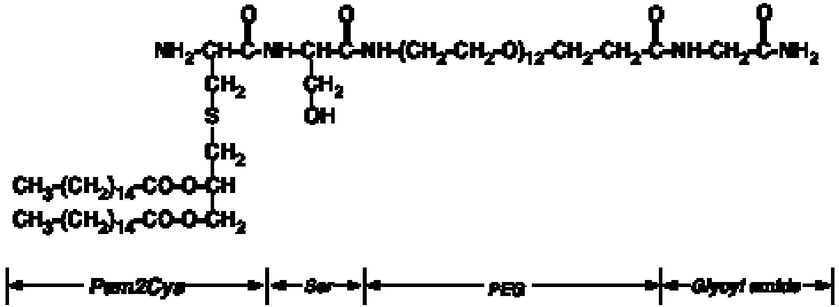

**Acylation:** A 4-fold molar excess of Fmoc amino acid, O-benzotriazole-N,N,N',N'-tetramethyl-uroniumhexafluorophosphate (HBTU) and a 6-fold molar excess of diisopropylethylamine (DIPEA) are used in all acylation steps. All acylation reactions are carried out for 60 minutes and completion of reaction confirmed by trinitrobenezene sulfonic acid (TNBSA) test. Removal of the Fmoc protective group from α-amino groups is achieved by exposing the solid phase support to 2.5% diazabicyclo[5.4.0]undec-7-ene (DBU; Sigma, Steinheim, Germany) for 2 × 5 minutes. dimethylformamide (DMF; Auspep, Melbourne, Australia) is used to wash the solid phase support between each acylation and de-protection step. The coupling of Fmoc-NH-(PEG)₁₁-COOH (Merck, Bayswater, Australia) is carried out in the same way as coupling amino acids.

**NB.** Glycine is first coupled to the TentaGel S RAM solid phase support followed by Fmoc-NH-(PEG)₁₁-COOH.

### Peptide quantitation

Quantitation of peptide-based materials was determined by amino acid analysis performed *in vacuo* by hydrolysis of samples at 110°C in sealed glass vials in the presence of 6N HCl containing 0.1% phenol. Derivatisation of amino acids was then carried out using Waters AccQTag reagents according to the manufacturer's instructions followed by analysis on a Waters Acquity UPLC System (Waters Millipore) using an AccQTag ultra column (2.1mm × 100mm; Waters Millipore).

### Preparation of INNA-003 and INNA-006

In the case of INNA-003 **two** serine residues are coupled seriatim following addition of the PEG moiety and in the case of INNA-006 a ***single*** serine is incorporated following addition of the PEG moiety.

### Lipidation (addition of Pam2Cys).

*Synthesis of S-(2,3-dihydroxypropyl)cysteine:* Triethylamine (6 g, 8.2 ml, 58 mmoles) is added to L-cysteine hydrochloride (3 g, 19 mmole) and 3-bromo-propan-1,2-diol (4.2 g, 2.36 ml, 27 mmole) in water and the homogeneous solution kept at room temperature for 3 days. The solution is reduced in vacuo at 40°C to a white residue which is then precipitated with acetone (300ml) and the precipitate isolated by centrifugation. The precipitate is washed with acetone twice more and dried to yield S-(2,3-dihydroxypropyl)cysteine as a white amorphous powder.

*Synthesis of N-Fluorenylmethoxycarbonyl-S-(2,3-dihydroxypropyl)-cysteine (Fmoc-Dhc-OH):* S-(2,3-dihydroxypropyl) cysteine (2.45 g, 12.6 mmole) is dissolved in 9% sodium carbonate (20 ml). A solution of fluorenylmethoxycarbonyl-N-hydroxysuccinimide (3.45 g, 10.5 mmole) in acetonitrile (20 ml) is then added and the mixture stirred for 2 h, diluted with water (240 ml) and extracted with diethyl ether (25 ml × 3). The aqueous phase is acidified to pH 2 with concentrated hydrochloric acid and then extracted with ethyl acetate (70 ml × 3). The extract is washed with water (50 ml × 2) and saturated sodium chloride solution (50 ml × 2). The extract is dried over anhydrous sodium sulphate and evaporated to dryness. The final product is obtained by applying high vacuum to remove residual solvent.

*Coupling of Fmoc-Dhc-OH to resin-bound peptide:* Fmoc-Dhc-OH (100mg, 0.24 mmole) is activated in DCM and DMF (1:1, v/v, 3mL)with HOBt (36 mg, 0.24 mmole) and DICI (37 uL, 0.24 mmole) at 0 °C for 5 min. The mixture is then added to a vessel containing the resin-bound peptide (0.04 mmole, 0.25g amino-peptide resin). After shaking for 2 h the solution is removed by filtration on a glass sinter funnel (porosity 3) and the resin washed with DCM and DMF (3 × 30mL each). The reaction is monitored for completion using the TNBSA test. If necessary a double coupling is performed.

*Palmitoylation of the two hydroxyl groups of the Fmoc-Dhc-peptide resin:* Palmitic acid (204 mg, 0.8 mmole), DIPCDI (154 uL, 1 mmole) and DMAP (9.76 mg, 0.08_mmole) are dissolved in 2mL of DCM and 1mL of DMF. The resin-bound Fmoc-Dhc-peptide_resin (0.04 mmole, 0.25 g) is suspended in this solution and shaken for 16 h at room temperature. The solution is removed by filtration and the resin then washed with DCM and_DMF thoroughly to remove any residue of urea. The removal of the Fmoc group is accomplished with 2.5% DBU (2 × 5min).

*Cleavage of peptide from the solid support:* Reagent B (93%TFA, 5%water and 2% triisopropylsilane) for two hours. **NB** the peptide will not precipitate in chilled ether. Most of the TFA must be removed and then the residue is dissolved in 50% acetonitrile and purified immediately or freeze-dried.

### Purification and characterisation of INNA-003 and INNA-006:

Following cleavage from the solid support, INNA-003 and INNA-006 were purified by reversed-phase high-performance liquid chromatography using a C4 VYDAC column (10 mm × 250 mm; Alltech, NSW, Australia) installed in a Waters HPLC system (Waters Millipore, Milford, MA, USA). Identity of the target materials were determined by mass spectrometry and the purified material was then characterised by analytical HPLC using a VYDAC C8 column (4.6 mm × 250 mm) and found to be greater than 95%. Mass analysis was carried out using an Agilent 1100 Series LC/ MSD ion-trap mass spectrometer (Agilent, Palo Alto, CA, USA).

Preparation of compound (2) or Pam2Cys-Thr-PEG, a single threonine is incorporated following the addition of the PEG11 moiety. The addition of Pam2Cys (lipidation) was carried out as described above.

Preparation of compound (3) or Pam2Cys-homoSer-PEG, a single homo-serine is incorporated following the addition of the PEG11 moiety. The addition of Pam2Cys (lipidation) was carried out as described above.

Preparation of compound (4) or Pam2Cys-phosphoSer-PEG, a single phosphoserine is incorporated following the addition of the PEG11 moiety. The addition of Pam2Cys (lipidation) was carried out as described above.

Preparation of Pam2Cys-Ser-PEG3, PEG3 moiety instead of PEG11 was coupled following the coupling of the first amino acid glycine. After the coupling of a single serine residue the addition of Pam2Cys (lipidation) was carried out as described above.

Preparation Pam2Cys-Ser-PEG5, PEG5 instead of PEG11 moiety was coupled following the coupling of the first amino acid glycine. After the coupling of a single serine residue the addition of Pam2Cys (lipidation) was carried out as described above.

Preparation of compound (5), PEG27 instead of PEG11 moiety was coupled following the coupling of the first amino acid glycine. After the coupling of a single serine residue the addition of Pam2Cys (lipidation) was carried out as described above

Preparation of compound (6), PEG27 moiety was coupled sequentially twice following the coupling of the first amino acid glycine. After the coupling of a single serine residue the addition of Pam2Cys (lipidation) was carried out as described above.

Preparation of compound (2a), two threonines are incorporated following the addition of the PEG11 moiety. The addition of Pam2Cys (lipidation) is carried out as described above.

Preparation of compound (3a), a two homo-serines are incorporated following the addition of the PEG11 moiety. The addition of Pam2Cys (lipidation) is carried out as described above.

Preparation of compound (4a), two phosphoserines are incorporated following the addition of the PEG11 moiety. The addition of Pam2Cys (lipidation) is carried out as described above.

Preparation of compound (5a), PEG27 instead of PEG11 moiety was coupled following the coupling of the first amino acid glycine. After the coupling of two serine residues the addition of Pam2Cys (lipidation) is carried out as described above

Preparation of compound (6a), PEG27 moiety was coupled sequentially twice following the coupling of the first amino acid glycine. After the coupling of two serine residues the addition of Pam2Cys (lipidation) is carried out as described above.

### Example 4

The primary objectives of the following study were to determine if the antiviral efficacy of a compound comprising a TLR2 agonist and suppression of subsequent virus-induced inflammation is maintained by compound treatment in the presence of FP, and if compound prophylactic treatment reverses FP suppression of innate antiviral defence during rhinovirus infection in mice.

### Experimental animals

Female 6-8 week old BALB/c mice were used for all studies. Each group contained 8 mice. After treatment or challenge procedures, mice were monitored daily for weight changes, and behavioural or physical changes as stipulated in animal ethics approval. At the time of sample collection, all mice were sacrificed with intraperitoneal administration with sodium pentobarbital.

### INNA-006 dosing and rhinovirus serotype 1B (RV1B) infection

Rhinovirus serotype 1B was originally purified from a clinical isolate and was grown in RD-ICAM cells and purified as previously described in Nat Med 14, 199-204 (2008) and Methods Mol Biol 1221, 181-188 (2015). Mice were dosed with 50 µl of agonist molecules intranasally (i.n.) under light isofluorane anaesthesia in an induction chamber within a class II biosafety cabinet. At indicated times post TLR-2 agonist dosing mice were infected i.n. with 50 µl containing 5 × 10⁶ TCID₅₀ of RV1B using the same procedure. At day 2 post-infection bronchoalveolar lavage (BAL) was performed to enumerate inflammatory cell infiltrate and measure immune mediator protein expression. Lungs were harvested for total RNA to assess viral loads. The mouse RV infection model and associated techniques are described in published Nat Med 14, 199-204 (2008) and Methods Mol Biol 1221, 181-188 (2015).

### Bronchoalveolar lavage (BAL) cell analysis

Following sacrifice, mice were tracheally cannulated and 1ml of Hanks buffered saline solution (Hyclone^{™}, GE Life Sciences) flushed through the airways, 3-5 times. BAL cells were pelleted by centrifugation and supernatant was collected and stored at -80°C for ELISAs. The pelleted cells were red blood cells lysed and the remaining cells were counted on a heamocytometer by trypan blue exclusion. Cell suspensions were then cytocentrifuged onto slides and fixed and stained with Diff Quick (POCD) solutions as per manufacturers recommendations. A minimum of 200 total cells were counted per slide and numbers of neutrophils, lymphocytes, and macrophages were determined.

### RNA extraction and qRT-PCR

The apical lung lobe from each mouse was collected into RNA-later (Ambion). For processing, lung lobes were transfer into RLT (Qiagen)/2ME buffer for tissue dissociation by TissueLyser II (Qiagen) at 25Hz, twice for 2 minutes (with sample rotation). Cell debris was pelleted by centrifugation and RNA was manually extracted using a miRNeasy kit (Qiagen) following the recommended suppliers protocol for extracting total RNA, including miRNA from animal and human cells and tissues. Following extraction, RNA concentration was determined using spectrophotometry (Nanodrop) and 200ng of RNA used for reverse transcription with random primers and RNase-inhibtor (AB, Applied Biosystems). cDNA was then subsequently used for qPCR analyses on a Quantstudio 6 using TaqMan, FAM-TAMRA chemistry (Life Technologies) with mastermix containing ROX (Qiagen), primers and probes outlined in table 1. Ct values for genes of interest where referenced to a seven standards of known concentration, starting with 10⁷ copies and proceeding in 1:10 dilution series. Copy numbers for all genes of interest were normalised to the reference gene 18s.

### Quantification of cytokines by ELISA

BAL fluid was then analysed for production of KC/IL-8 (CXCL1) and TNF-α by Duoset ELISA (R&D Systems) as per manufacturer's instructions.

**Table 1. Primer and probe sequences for qPCR**

| **Gene** | **Sequence (5'-3')** | | |
|---|---|---|---|
| | **Forward** | **Reverse** | **Probe** |
| ***18s*** | CGCCGCTAGAGGT GAAATTCT | CATTCTTGGCAAATGCTT TCG | FAM- ACCGGCGCAAGACGGAC CAGA-TAMRA |
| ***Rhinovirus*** | GTGAAGAGCCsCrT GTGCT | GCTsCAGGGTTAAGGTT AGCC | FAM- TGAGTCCTCCGGCCCCT GAATG-TAMRA |

qPCR analyses conducted using TaqMan chemistry in a total volume of 12.5ul per reaction with optimal, custom forward/reverse primer ratios on cDNA generated from RNA extracted from the apical lung lobe of each mouse.

### Study protocol

Restoration of anti-viral immunity following corticosteroid (CS) fluticisone propionate (FP) treatment. Mice were prophylactically dosed with the lead candidate using the previously determined optimal dosing protocols. Mice were treated with FP (PBS for controls) 1 hour before RV1B infection (or mock infection with PBS). Innate anti-viral immunity (type I/III IFN protein in BAL) and lung tissue viral load (viral RNA, qPCR) were assessed at 24 h post-infection.

### Results

Restoration of anti-viral immunity following CS (fluticisone propionate FP) treatment. Mice were prophylactically dosed with 2pmol of INNA-006 to the total respiratory tract seven days prior to infection, or (separately). Weight loss was monitored daily after first treatment. Mice were then treated with FP (PBS for controls) 1 hour before RV1B infection (or mock infection with PBS). Lung tissue viral load (viral RNA, qPCR) was assessed at 48 h post-infection and lung inflammation determined by differential staining of BAL inflammatory cells and measurement of protein immune mediators in BAL-fluid.

Mice were treated i.n. with 2pmol of INNA-006 and day-7 or combined day-7 & day -1 before i.n. FP administration and i.n. infection with RV (all to the total respiratory tract. Controls not dosed with TLR agonists were treated with saline. Weight loss was assessed as % change from day of first treatment (day -7). There was no significant weight loss observed upon INNA-006 or FP treatment compared to the relevant controls (data not shown).

Inflammatory cell analysis indicated that D-7 INNA-006 treatment resulted in increased macrophages and lymphocytes when given in conjunction with FP. Combined D-7 & D-1 INNA-006 treatment increased macrophages and lymphocytes in BAL. Increased Macrophages with combined D-7 & D-1 INNA-006 was also observed in FP treated mice. Astonishingly, INNA-006 treatment completely ameliorated RV-induced and steroid resistant neutrophilic inflammatory response in RV infected mice (Figure 16).

The inflammatory mediator CXCL1 in BAL were measured by ELISA (Figure 17). Steroid resistant RV-induced neutrophilic inflammation corresponded with increased CXCL1 (KC, mouse IL-8) protein production. INNA-006 supressed CXCL1 production and prevented steroid resistant inflammatory responses.

Lung viral load was assessed by qPCR. FP treatment increased viral lung load in Saline control mice only. INNA-006 reduced virus lung load in all groups, but repeated INNA-006 treatment prior to FP actually enhanced antiviral efficacy (Figure 18).

The most striking feature of the BAL data from this study was complete suppression of RV-induced steroid resistant neutrophilic inflammation by INNA-006 in all treatment protocols. Suppressed neutrophilic inflammation corresponded with drastically reduced levels of mouse neutrophil chemokine CXCL1 (KC).

Repeated treatment with 2pmol INNA-006 (day -7 & day -1) increased total leukocyte numbers, corresponding with macrophage and lymphocyte recruitment. Increased macrophage recruitment was also observed in FP treated mice with either INNA-006 dosing protocol as well as FP treated mice. Lymphocyte numbers were increased in D-7 INNA-006 FP RV and D-1 & d-1 INNA-006 Veh RV groups, by an unknown mechanism. A single dose with 2pmol of INNA-006 seven days prior to infection resulted in significant TNF-α production in BAL but this was not observed when repeated doses of INNA-006 (D-7 & D-1) were given. FP treatment also reduced TNF-α production stimulated by INNA-006.

Consistent with reduced neutrophils, agonist treatment was highly effective at suppressing CXCL1 expression. Viral replication drives innate immune activation and CXCL1 expression so this data supports virus replication and infection induced inflammation being suppressed with TLR-2 agonist treatment. Analysis of viral RNA confirmed this with INNA-006 treatment inducing a significant reduction in viral load with both treatment protocols. The best suppression of viral lung RNA was actually observed in mice with repeated INNA-006 treatment in conjunction with FP.

In conclusion these studies demonstrate that ant-viral activity against RV infection with TLR agonists, is maintained and even enhanced with FP treatment.

### Example 5 - TLR2 activation by various compounds

Comparison of the abilities of various compounds to stimulate luciferase activity in an NF-κB cell-based reporter system was determined. Compounds tested include INNA-006 (or compound (1)); INNA-013 (or compound (4)); INNA-014 (or compound (3)); INNA-015 (or compound (2)); INNA-010; INNA-011 (or compound (5)); INNA-012 (or compound (6)); and INNA-009. HEK293T cells, transiently co-transfected with a human TLR2 plasmid and a luciferase-NF-κB plasmid reporter system, were exposed to various dilutions of each compound. Successful receptor binding and subsequent signal transduction events were determined by measuring the luminescence due to luciferase activity (results shown in Fig. 19 - left to right columns for each concentration are in the following order INNA-006 (or compound (1)); INNA-013 (or compound (4)); INNA-014 (or compound (3)); INNA-015 (or compound (2)); INNA-010; INNA-011 (or compound (5)); INNA-012 (or compound (6)); and INNA-009.

The results demonstrate that the most potent compounds were those with a single serine, threonine or homoserine separating the Pam2Cys and PEG, or a length of 12, 28, or two groups of 28, ethylene oxide monomers. However, all compounds resulted in successful receptor binding and subsequent signal transduction.

### Example 6 - Comparison of INNA-006 and Pam3Cys-Ser-PEG3000 using an in vitro luciferase assay

Comparison of the *in vitro* TLR2 agonistic activity of Pam3Cys-Ser-PEG3000 and INNA-006: HEK293T cells, transiently co-transfected with a human TLR2 plasmid and a luciferase-NF-κB plasmid reporter system, were exposed to various dilutions of INNA-006 or Pam3Cys-Ser-PEG3000.

Successful receptor binding and subsequent signal transduction events were determined by measuring the luminescence due to luciferase activity (Fig. 20). The results demonstrate that Pam3Cys-Ser-PEG3000 is inferior to INNA-006 in its ability to signal NF-κB in the dose range tested (12.2pM to 3.125pM.).

### Example 7 - TLR binding and specificity

INNA-006 was assessed for its ability to activate a range of other TLR pattern recognition receptors. These assessments were conducted using both human and mouse TLR panels. These assays detect a secreted embryonic alkaline phosphatase (SEAP) reporter under the control of a promoter which is inducible by NF-κB activation in HEK293 cells.

The secreted embryonic alkaline phosphatase (SEAP) reporter is under the control of a promoter inducible by the transcription factor NF-κB. This reporter gene allows the monitoring of signaling through the TLR, based on the activation of NF-κB. In a 96-well plate (200 µL total volume) containing the appropriate cells (50,000 - 75,000 cells/well), 20 µL of the test article or the positive control ligand is added to the wells. The media added to the wells is designed for the detection of NF-κB induced SEAP expression. After a 16-24 hr incubation the optical density (OD) is read at 650 nm on a Molecular Devices SpectraMax 340PC absorbance detector.

### Control Ligands

hTLR2: HKLM (heat-killed Listeria monocytogenes) at 1x108 cells/mL
hTLR3: Poly(I:C) HMW at 1 µg/mL
hTLR4: E. coli K12 LPS at 100 ng/mL
hTLR5: S. typhimurium flagellin at 100 ng/mL
hTLR7: CL307 at 1 µg/mL
hTLR8: CL075 at 1 pg/mL
hTLR9: CpG ODN2006 at 1 µg/mL.

Under the conditions tested, it was confirmed that INNA-006 was able to activate its proposed target (TLR-2) and showed no activation of any other TLR tested in these assays (Fig. 21).

## Claims

1. A compound comprising a TLR2 agonist for use in treating or preventing a respiratory condition associated with rhinovirus in a subject, wherein the TLR2 agonist comprises a lipopeptide or a lipid moiety, wherein the lipid moiety is selected from palmitoyl, myristoyl and stearoyl, and wherein the TLR2 agonist is linked to a solubilising moiety, wherein the solubilising moiety comprises a polyethyleneglycol (PEG).

2. A compound comprising a TLR2 agonist for use in treating or preventing a viral mediated exacerbation of a respiratory condition in a subject, wherein the viral mediated exacerbation is a rhinovirus mediated exacerbation, wherein the TLR2 agonist comprises a lipopeptide or a lipid moiety, wherein the lipid moiety is selected from palmitoyl, myristoyl and stearoyl, and wherein the TLR2 agonist is linked to a solubilising moiety, wherein the solubilising moiety comprises a polyethyleneglycol (PEG).

3. A compound of claim 1 or 2, wherein the respiratory condition is asthma.

4. A compound according to any one of claims 1 to 3, wherein the compound is administered in a composition that further comprises a pharmaceutically acceptable carrier, diluent or excipient.

5. A compound according to claim 1, wherein the respiratory condition is chronic obstructive pulmonary disease (COPD), asthma, cystic fibrosis, or lung conditions associated with lung transplantation or chronic glucocorticosteroid use.

6. A compound according to any one of claims 1 to 5, wherein the respiratory condition is a rhinovirus infection.

7. A compound according to claim 1 or 2, wherein the TLR2 agonist comprises a lipopeptide selected from the group consisting of: Pam2Cys, Pam3Cys, Ste2Cys, and Oct2Cys, preferably Pam2Cys.

8. A compound according to any one of claims 1 to 7, wherein the compound comprising a TLR2 agonist comprises the structure:
A-Y-B
wherein A comprises or consists of: 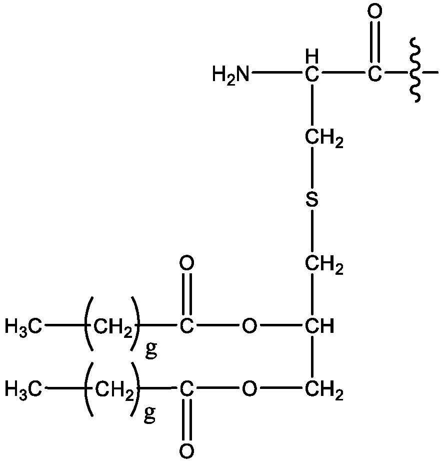 wherein each g is independently 12, 14 or 16;
Y is 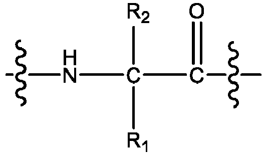
wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H;
and
B comprises or consists of Polyethylene Glycol (PEG),
or a pharmaceutically acceptable salt or prodrug thereof.

9. A compound according to any one of claims 1 to 7, wherein the compound comprising a TLR2 agonist comprises Pam2Cys and PEG, wherein the Pam2Cys and PEG are linked by a serine, homoserine, threonine or phosphoserine residue,
wherein
Pam2Cys in the compound has the structure:

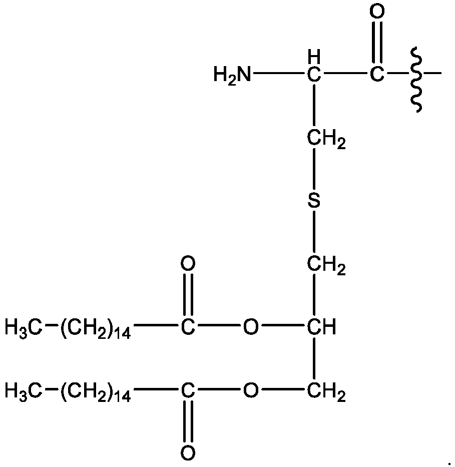

10. A compound according to any one of claims 1 to 7, wherein the compound comprises: 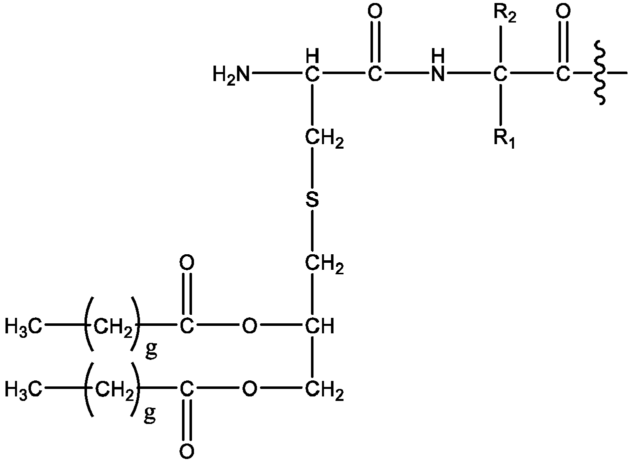 wherein R₁ and R₂ are independently selected from the group consisting of H, - CH₂OH, -CH₂CH₂OH, -CH(CH₃)OH and -CH₂OPO(OH)₂, wherein any one of the alkyl hydrogens can be replaced with a halogen, and wherein R₁ and R₂ are not both H; and
each g is independently selected from 12, 14 or 16;
covalently linked to polyethylene glycol (PEG),
or a pharmaceutically acceptable salt or prodrug thereof.

11. A compound according to any one of claims 1 to 7, wherein the compound has the structure of compound (1): 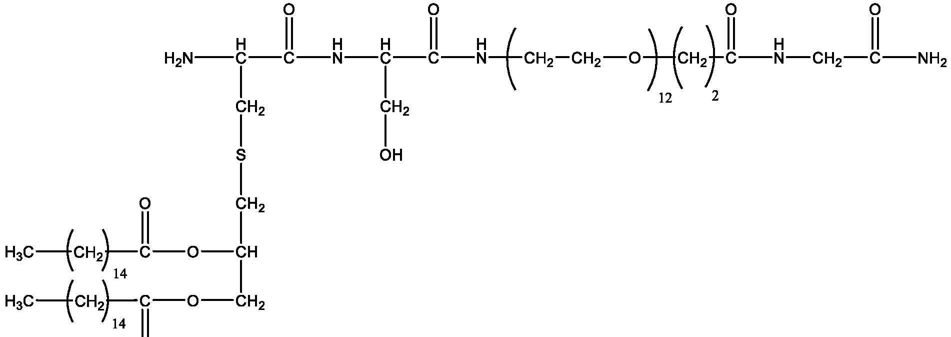 or a pharmaceutically acceptable salt or prodrug thereof.

12. A compound according to any one of claims 1 to 7, wherein the compound has the structure of compound (5):

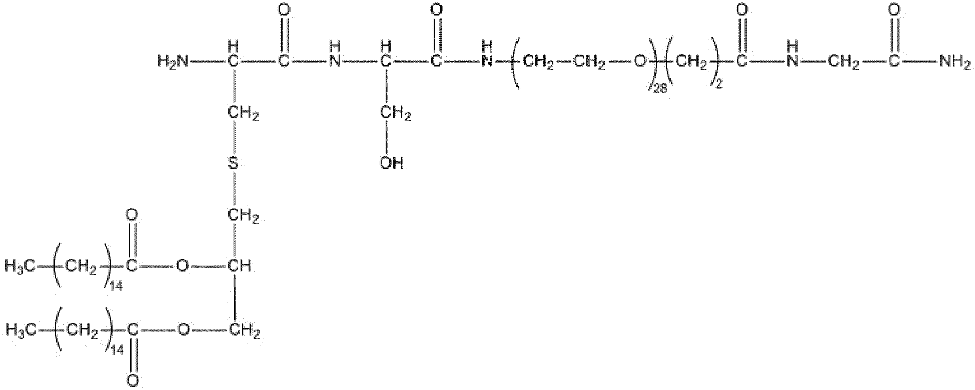

13. A compound according to any one of claims 1 to 7, wherein the compound is of formula (IVa):
Pam2Cys-Ser-Ser-NH-(CH₂)ₚ-O-(CH₂-CH₂-O)ₙ-[(CH₂)ₘ₋CO-L-]_{q}R₃ (IVa)
wherein
Pam2Cys has the structure: 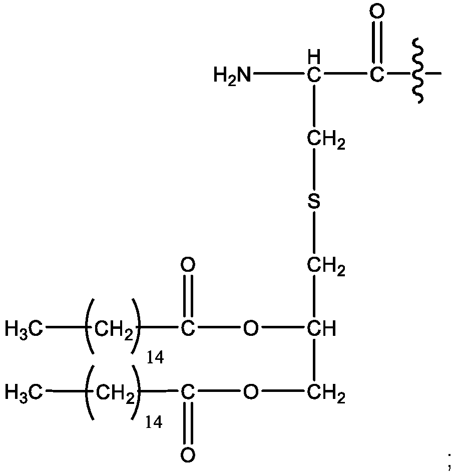
n is 3 to 100;
m is 1, 2, 3 or 4;
p is 2, 3 or 4;
q is null or 1;
R₁, R₁', R₂ and R₂' are independently selected from the group consisting of H, -CH₂OH, -CH₂CH₂OH, -CH(CH₃)OH and -CH₂OPO(OH)₂, wherein any one of the alkyl hydrogens can be replaced with a halogen, and wherein R₁ and R₁' are not both H, and R₂ and R₂' are not both H;
wherein when q is null, R₃ is H;
wherein when q is 1, R₃ is -NH₂ or -OH;
L is null or consists of 1 to 10 units, wherein each unit is a natural alpha amino acid or derived from a natural alpha amino acid, and has the formula: 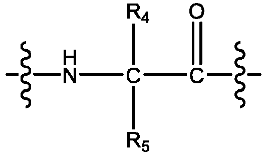
wherein R₄ is H; and
R₅ is the side chain, or second hydrogen of the amino acid,
or a pharmaceutically acceptable salt or prodrug thereof.

14. A compound according to any one of claims 1 to 7, wherein the compound is of formula (Va): 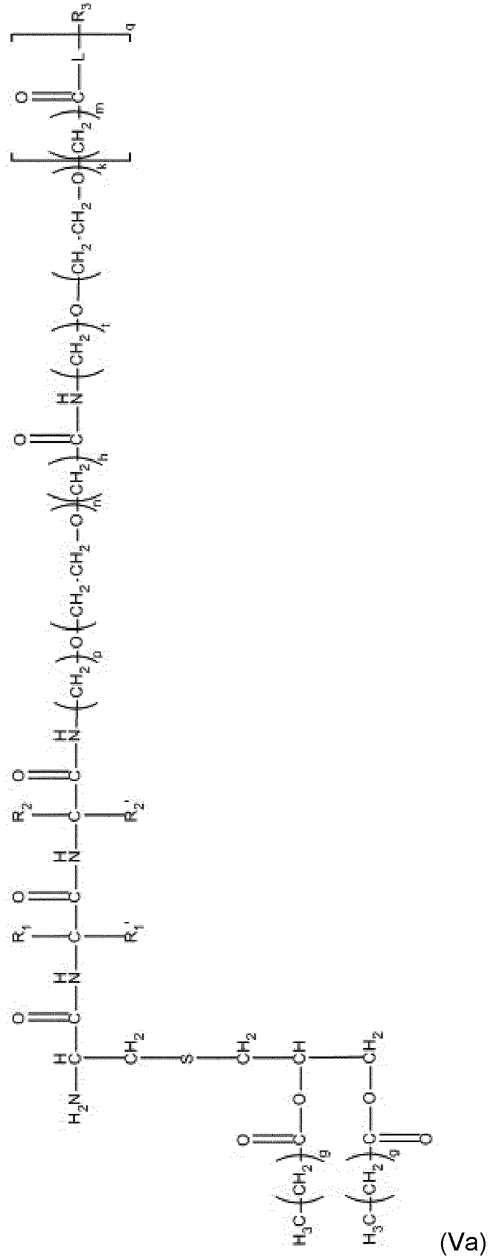
wherein
n is 3 to 100;
k is 3 to 100;
h is 1, 2, 3 or 4;
m is 1, 2, 3 or 4;
each g is independently 12, 14 or 16;
p is 2, 3 or 4;
t is 2, 3 or 4;
q is null or 1;
R₁, R₁', R₂ and R₂' are independently selected from the group consisting of H, -CH₂OH, -CH₂CH₂OH, -CH(CH₃)OH and -CH₂OPO(OH)₂, wherein any one of the alkyl hydrogens can be replaced with a halogen, and wherein R₁ and R₁' are not both H, and R₂ and R₂' are not both H;
wherein when q is null, R₃ is H;
wherein when q is 1, R₃ is -NH₂ or -OH;
L is null or consists of 1 to 10 units, wherein each unit is a natural alpha amino acid or derived from a natural alpha amino acid, and has the formula: 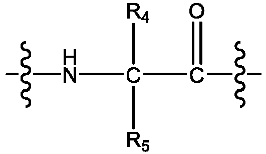
wherein R₄ is H; and
R₅ is the side chain, or second hydrogen of the amino acid,
or a pharmaceutically acceptable salt or prodrug thereof.

15. A compound according to any one of claims 1 to 14, wherein the TLR2 agonist is administered once daily or once weekly.

16. A compound according to any one of claims 1 to 15, wherein the use further comprises administering a corticosteroid, preferably a glucocorticoid.

17. A compound according to any one of claims 1 to 16, wherein the compound is formulated or adapted for administration to the respiratory tract.

18. A compound or composition according to claim 17, wherein the compound or composition is formulated or adapted for inhalation or intranasal administration.

19. A compound or composition according to claim 18, wherein the compound or composition is formulated as a nasal spray or as nasal drops.

## Patentansprüche

1. Verbindung, umfassend einen TLR2-Agonisten zur Verwendung bei der Behandlung oder Vorbeugung eine Atemwegserkrankung, die mit Rhinovirus in einem Subjekt assoziiert ist, wobei der TLR2-Agonist ein Lipopeptid oder eine Lipideinheit umfasst, wobei die Lipideinheit aus Palmitoyl, Myristoyl und Stearoyl ausgewählt ist, und wobei der TLR2-Agonist mit einer auflösenden Einheit verbunden ist, wobei die auflösende Einheit ein Polyethylenglykol (PEG) umfasst.

2. Verbindung, umfassend einen TLR2-Agonisten zur Verwendung bei der Behandlung oder Vorbeugung einer viral vermittelten Verschlimmerung einer Atemwegserkrankung in einem Subjekt, wobei die viral vermittelte Verschlimmerung eine Rhinovirus-vermittelte Verschlimmerung ist, wobei der TLR2-Agonist ein Lipopeptid oder eine Lipideinheit umfasst, wobei die Lipideinheit aus Palmitoyl, Myristoyl und Stearoyl ausgewählt ist, und wobei der TLR2-Agonist mit einer auflösenden Einheit verbunden ist, wobei die auflösende Einheit ein Polyethylenglycol (PEG) umfasst.

3. Verbindung nach Anspruch 1 oder 2, wobei die Atemwegserkrankung Asthma ist.

4. Verbindung nach einem der Ansprüche 1 bis 3, wobei die Verbindung in einer Zusammensetzung verabreicht ist, die ferner einen pharmazeutisch akzeptablen Träger, Verdünnungsmittel oder Hilfsstoff umfasst.

5. Verbindung nach Anspruch 1, wobei die Atemwegserkrankung chronisch obstruktive Lungenerkrankung (COPD), Asthma, zystische Fibrose, oder Lungenerkrankungen, die mit einer Lungentransplantation oder chronischer Glukokortikosteroid-Verwendung assoziiert sind, ist.

6. Verbindung nach einem der Ansprüche 1 bis 5, wobei die Atemwegserkrankung eine Rhinovirusinfektion ist.

7. Verbindung nach Anspruch 1 oder 2, wobei der TLR2-Agonist ein Lipopeptid umfasst, das ausgewählt ist aus der Gruppe bestehend aus: Pam2Cys, Pam3Cys, Ste2Cys, und Oct2Cys, vorzugsweise Pam2Cys.

8. Verbindung nach einem der Ansprüche 1 bis 7, wobei die Verbindung, die einen TLR2-Agonisten umfasst, die Struktur umfasst:
A-Y-B
wobei A Folgendes umfasst oder aus Folgendem besteht: 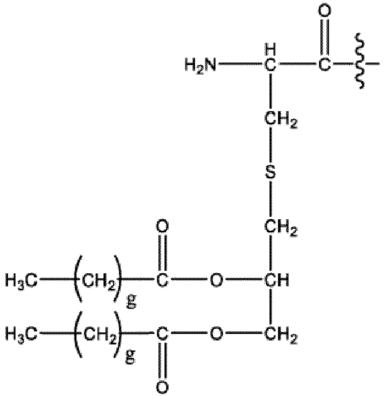 wobei jedes g unabhängig 12, 14 oder 16 ist;
Y ist 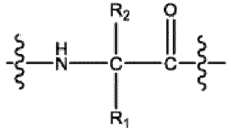
wobei R₁ und R₂ unabhängig aus der Gruppe ausgewählt sind, die aus H, -CH₂OH, -CH₂CH₂OH, -CH(CH₃)OH und -CH₂OPO(OH)₂ besteht, wobei jeder der Alkylwasserstoffe durch ein Halogen ersetzt sein kann, und wobei R₁ und R₂ nicht beide H sind;
und
B Polyethylenglykol (PEG) umfasst oder aus Polyethylenglykol (PEG) besteht,
oder ein pharmazeutisch akzeptables Salz oder Prodrug davon.

9. Verbindung nach einem der Ansprüche 1 bis 7, wobei die Verbindung, die einen TLR2-Agonisten umfasst, Pam2Cys und PEG umfasst, wobei die Pam2Cys und PEG durch einen Serin-, Homoserin-, Threonin- oder Phosphoserin-Rest verbunden sind,
wobei
Pam2Cys in der Verbindung die Struktur aufweist:

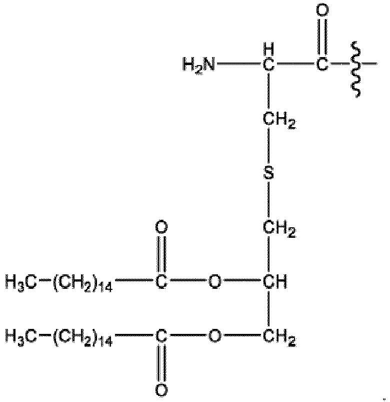

10. Verbindung nach einem der Ansprüche 1 bis 7, wobei die Verbindung umfasst: 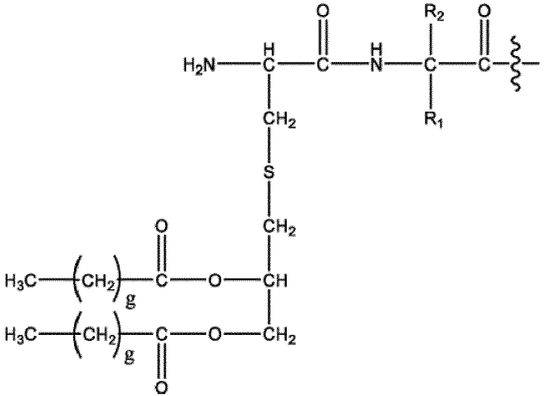
wobei R₁ und R₂ unabhängig aus der Gruppe ausgewählt sind, die aus H, -CH₂OH, -CH₂CH₂OH, -CH(CH₃)OH und -CH₂OPO(OH)₂ besteht, wobei jeder der Alkylwasserstoffe durch ein Halogen ersetzt sein kann, und wobei R₁ und R₂ nicht beide H sind; und
jedes g unabhängig aus 12, 14 oder 16 ausgewählt ist;
kovalent an Polyethylenglykol (PEG) gebunden,
oder ein pharmazeutisch akzeptables Salz oder Prodrug davon.

11. Verbindung nach einem der Ansprüche 1 bis 7, wobei die Verbindung die Struktur von Verbindung (1) aufweist: 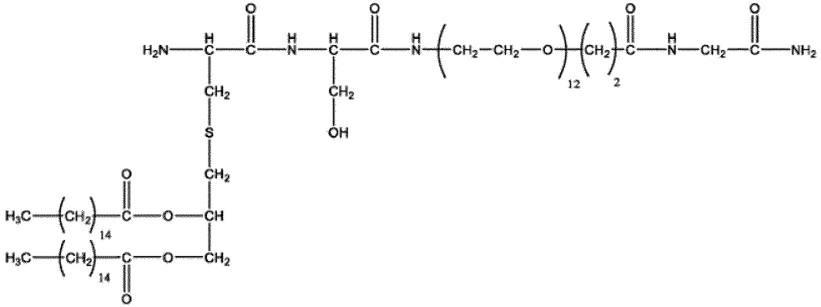 oder ein pharmazeutisch akzeptables Salz oder Prodrug davon.

12. Verbindung nach einem der Ansprüche 1 bis 7, wobei die Verbindung die Struktur von Verbindung (5) aufweist:

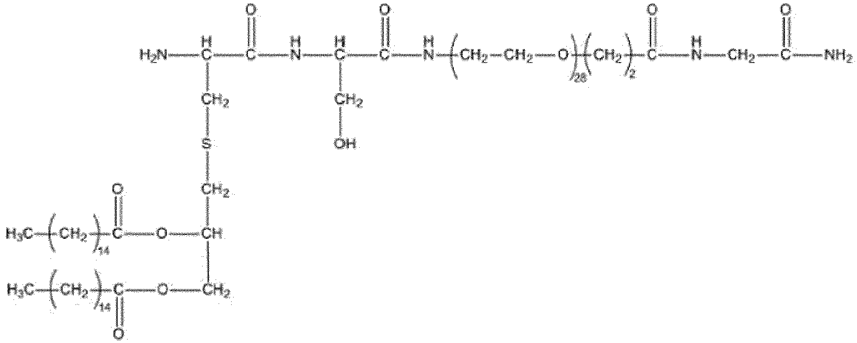

13. Verbindung nach einem der Ansprüche 1 bis 7, wobei die Verbindung die Formel (IVa) aufweist:
Pam2Cys-Ser-Ser-NH-(CH₂)ₚ-O-(CH₂-CH₂-O)ₙ-(CH₂)ₘ₋CO-L-]_{q}R₃ (IVa)
wobei
Pam2Cys die Struktur aufweist: 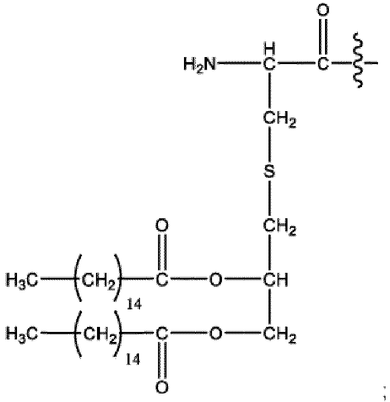
n 3 bis 100 ist;
m 1, 2, 3 oder 4 ist;
p 2, 3 oder 4 ist;
q 0 oder 1 ist;
R₁, R₁', R₂ und R₂' unabhängig aus der Gruppe ausgewählt sind, die aus H, -CH₂OH, -CH₂CH₂OH, -CH(CH₃)OH und -CH₂OPO(OH)₂ besteht, wobei jeder der Alkylwasserstoffe durch ein Halogen ersetzt sein kann, und wobei R₁ und R₁' nicht beide H sind, und R₂ und R₂' nicht beide H sind;
wobei, wenn q Null ist, R₃ H ist;
wobei, wenn q 1 ist, R₃ -NH₂ oder -OH ist;
L Null ist oder aus 1 bis 10 Einheiten besteht, wobei jede Einheit eine natürliche Alpha-Aminosäure ist oder von einer natürlichen Alpha-Aminosäure abgeleitet ist, und die Formel aufweist: 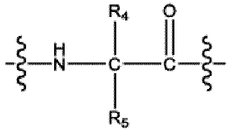
wobei R₄ H ist; und
R₅ die Seitenkette ist, oder ein zweiter Wasserstoff der Aminosäure,
oder ein pharmazeutisch akzeptables Salz oder Prodrug davon.

14. Verbindung nach einem der Ansprüche 1 bis 7, wobei die Verbindung Formel (Va) aufweist: 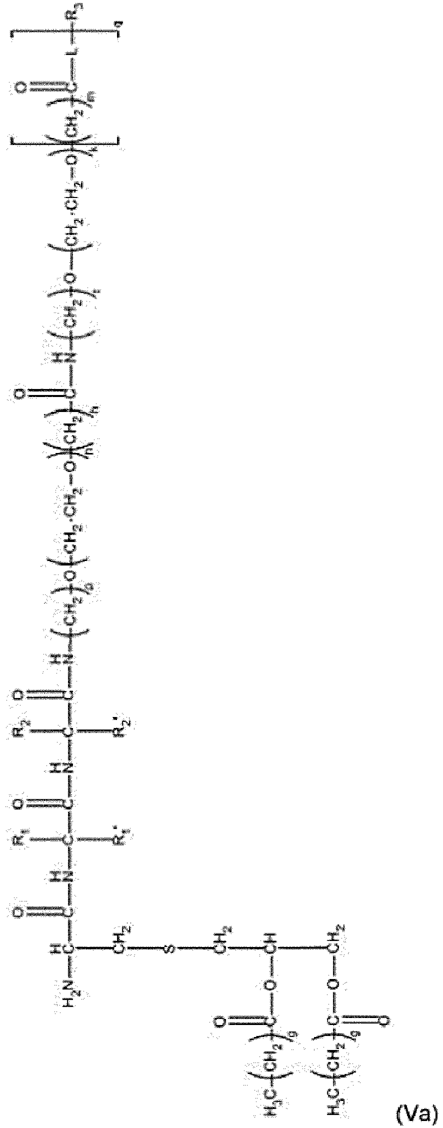 wobei
n 3 bis 100 ist;
k 3 bis 100 ist;
h 1, 2, 3 oder 4 ist;
m 1, 2, 3 oder 4 ist;
jedes g unabhängig 12, 14 oder 16 ist;
p 2, 3 oder 4 ist;
t 2, 3 oder 4 ist;
q Null oder 1 ist;
R₁, R₁', R₂ und R₂' unabhängig aus der Gruppe ausgewählt sind, die aus H, -CH₂OH, -CH₂CH₂OH, -CH(CH₃)OH und -CH₂OPO(OH)₂ besteht, wobei jeder der Alkylwasserstoffe durch ein Halogen ersetzt sein kann, und wobei R₁ und R₁' nicht beide H sind, und R₂ und R₂' nicht beide H sind;
wobei, wenn q Null ist, R₃ H ist;
wobei, wenn q 1 ist, R₃ -NH₂ oder -OH ist;
L Null ist oder aus 1 bis 10 Einheiten besteht, wobei jede Einheit eine natürliche Alpha-Aminosäure ist oder von einer natürlichen Alpha-Aminosäure abgeleitet ist, und die Formel aufweist: 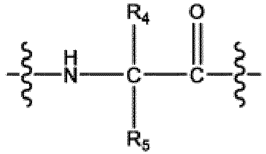
wobei R₄ H ist; und
R₅ die Seitenkette ist, oder ein zweiter Wasserstoff der Aminosäure, oder ein pharmazeutisch akzeptables Salz oder Prodrug davon.

15. Verbindung nach einem der Ansprüche 1 bis 14, wobei der TLR2-Agonist einmal täglich oder einmal wöchentlich verabreicht wird.

16. Verbindung nach einem der Ansprüche 1 bis 15, wobei die Verwendung ferner ein Verabreichen eines Corticosteroids, vorzugsweise eines Glucocorticoids, umfasst.

17. Verbindung nach einem der Ansprüche 1 bis 16, wobei die Verbindung zur Verabreichung an die Atemwege formuliert oder angepasst ist.

18. Verbindung oder Zusammensetzung nach Anspruch 17, wobei die Verbindung oder Zusammensetzung zur Inhalation oder intranasalen Verabreichung formuliert oder angepasst ist.

19. Verbindung oder Zusammensetzung nach Anspruch 18, wobei die Verbindung oder Zusammensetzung als ein Nasenspray oder als Nasentropfen formuliert ist.

## Revendications

1. Composé comprenant un agoniste de TLR2 pour son utilisation dans le traitement ou la prévention d'un trouble respiratoire associé à un rhinovirus chez un sujet, dans lequel l'agoniste de TLR2 comprend un lipopeptide ou une fraction lipidique, dans lequel la fraction lipidique est sélectionnée à partir d'un palmitoyle, d'un myristoyle et d'un stéaroyle, et dans lequel l'agoniste de TLR2 est lié à une fraction de solubilisation, dans lequel la fraction de solubilisation comprend un polyéthylèneglycol (PEG).

2. Composé comprenant un agoniste de TLR2 pour son utilisation dans le traitement ou la prévention d'une exacerbation médiée par virus d'un trouble respiratoire chez un sujet, l'exacerbation médiée par virus étant une exacerbation médiée par rhinovirus, dans lequel l'agoniste de TLR2 comprend un lipopeptide ou une fraction lipidique, dans lequel la fraction lipidique est sélectionnée à partir d'un palmitoyle, d'un myristoyle et d'un stéaroyle, et dans lequel l'agoniste de TLR2 est lié à une fraction de solubilisation, dans lequel la fraction de solubilisation comprend un polyéthylèneglycol (PEG).

3. Composé selon la revendication 1 ou la revendication 2, dans lequel le trouble respiratoire est de l'asthme.

4. Composé selon l'une quelconque des revendications 1 à 3, dans lequel le composé est administré dans une composition qui comprend en outre un support, un diluant ou un excipient pharmaceutiquement acceptable.

5. Composé selon la revendication 1, dans lequel le trouble respiratoire est une bronchopneumopathie chronique obstructive (BCPO), de l'asthme, la mucoviscidose, ou des troubles pulmonaires associés à une transplantation pulmonaire ou à une utilisation chronique de glucocorticostéroïde.

6. Composé selon l'une quelconque des revendications 1 à 5, dans lequel le trouble respiratoire est une infection à rhinovirus.

7. Composé selon la revendication 1 ou la revendication 2, dans lequel l'agoniste de TLR2 comprend un lipopeptide sélectionné dans le groupe constitué de : Pam2Cys, Pam3Cys, Ste2Cys et Oct2Cys, de préférence Pam2Cys.

8. Composé selon l'une quelconque des revendications 1 à 7, dans lequel le composé comprenant un agoniste de TLR2 comprend la structure :
A-Y-B
dans laquelle A comprend ou consiste en : 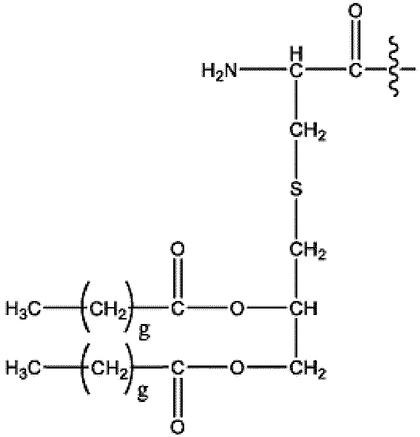 dans laquelle chaque g est indépendamment 12, 14 ou 16 ;
Y est 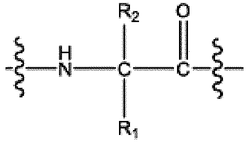
dans laquelle R₁ et R₂ sont indépendamment sélectionnés dans le groupe constitué de H, -CH₂OH, -CH₂CH₂OH, -CH(CH₃)OH et -CH₂OPO(OH)₂, dans laquelle l'un quelconque des hydrogènes d'alkyle peut être remplacé par un halogène, et dans laquelle R₁ et R₂ ne sont pas tous les deux H ;
et
B comprend ou consiste en du polyéthylèneglycol (PEG),
ou un de ses sels ou une de ses prodrogues pharmaceutiquement acceptables.

9. Composé selon l'une quelconque des revendications 1 à 7, dans lequel le composé comprenant un agoniste de TLR2 comprend Pam2Cys et le PEG, dans lequel Pam2Cys et le PEG sont liés par un résidu de sérine, d'homosérine, de thréonine ou de phosphosérine,
dans lequel
Pam2Cys dans le composé a la structure :

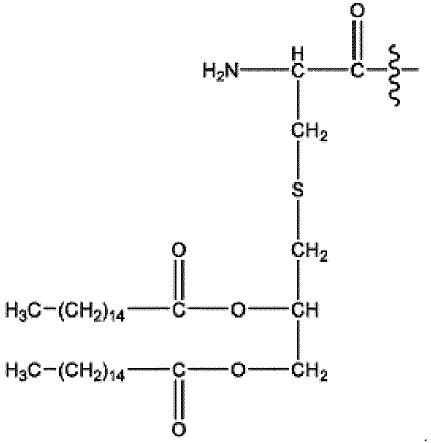

10. Composé selon l'une quelconque des revendications 1 à 7, dans lequel le composé comprend : 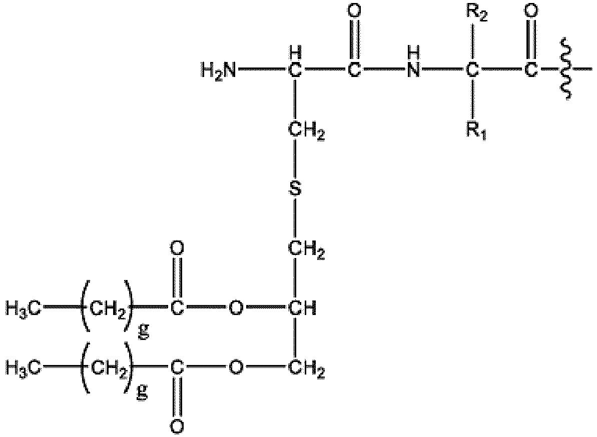
où R₁ et R₂ sont indépendamment sélectionnés dans le groupe constitué de H, -CH₂OH, -CH₂CH₂OH, -CH(CH₃)OH et -CH₂OPO(OH)₂, où l'un quelconque des hydrogènes d'alkyle peut être remplacé par un halogène, et où R₁ et R₂ ne sont pas tous les deux H ; et
chaque g est indépendamment sélectionné parmi 12, 14 ou 16 ;
lié de manière covalente au polyéthylèneglycol (PEG),
ou un de ses sels ou une de ses prodrogues pharmaceutiquement acceptables.

11. Composé selon l'une quelconque des revendications 1 à 7, dans lequel le composé a la structure de composé (1) : 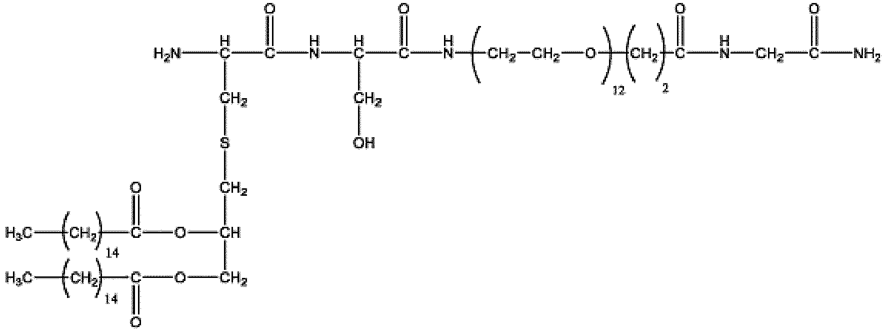 ou un de ses sels ou une de ses prodrogues pharmaceutiquement acceptables.

12. Composé selon l'une quelconque des revendications 1 à 7, dans lequel le composé a la structure de composé (5) :

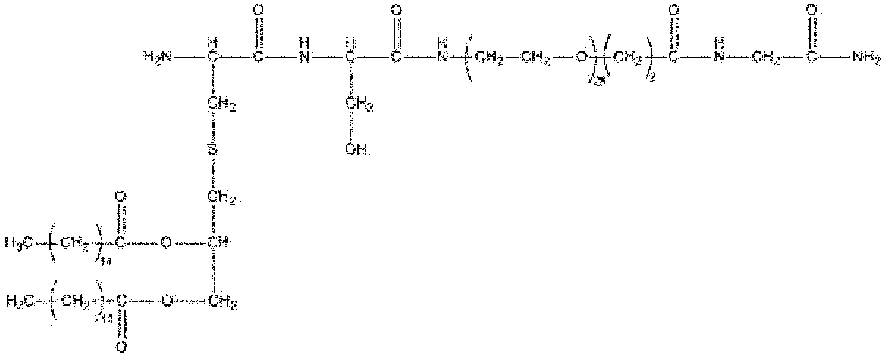

13. Composé selon l'une quelconque des revendications 1 à 7, dans lequel le composé est de formule (IVa) :
Pam2Cys-Ser-Ser-NH-(CH₂)ₚ-O-(CH₂-CH₂-O)ₙ-[(CH₂)ₘ-CO-L-]_{q}R₃ (IVa)
dans laquelle
Pam2Cys a la structure : 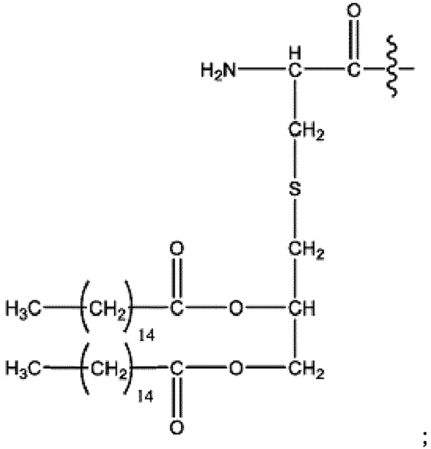
n est 3 à 100 ;
m est 1, 2, 3 ou 4;
p est 2, 3 ou 4 ;
q est nul ou 1 ;
R₁, R₁', R₂ et R₂' sont indépendamment sélectionnés dans le groupe constitué de H, -CH₂OH, -CH₂CH₂OH, -CH(CH₃)OH et -CH₂OPO(OH)₂, dans laquelle l'un quelconque des hydrogènes d'alkyle peut être remplacé par un halogène, et dans laquelle R₁ et R₁' ne sont pas tous les deux H et R₂ et R₂' ne sont pas tous les deux H ;
dans laquelle, lorsque q est nul, R₃ est H ;
dans laquelle, lorsque q est 1, R₃ est -NH₂ ou -OH ;
L est nul ou est constitué de 1 à 10 unités, dans laquelle chaque unité est un acide alpha-aminé naturel ou est dérivé d'un acide alpha-aminé naturel, et a la formule : 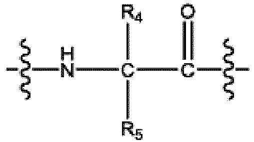
dans laquelle R₄ est H ; et
R₅ est la chaîne latérale, ou un second hydrogène de l'acide aminé,
ou un de ses sels ou une de ses prodrogues pharmaceutiquement acceptables.

14. Composé selon l'une quelconque des revendications 1 à 7, dans lequel le composé est de formule (Va) : 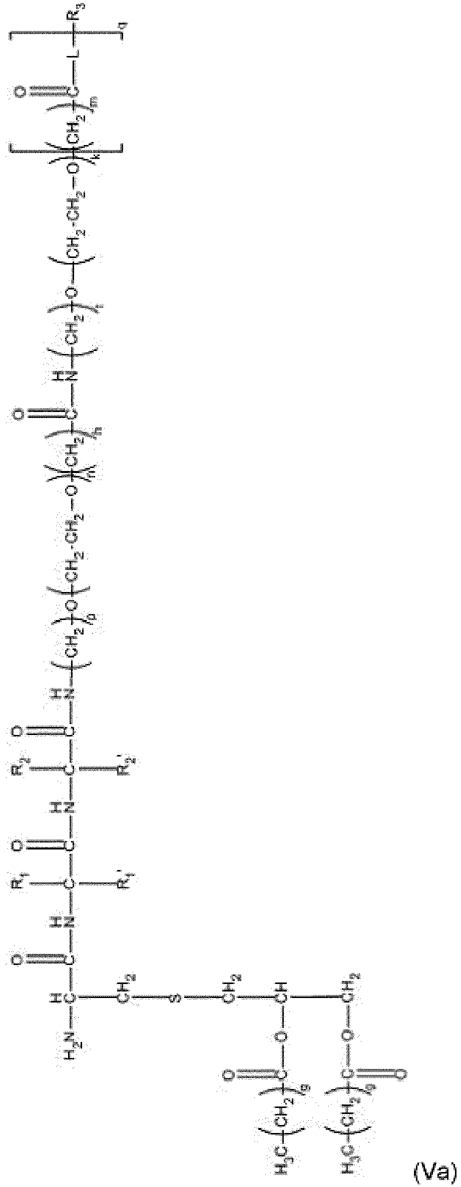 dans laquelle
n est 3 à 100 ;
k est 3 à 100 ;
h est 1, 2, 3 ou 4 ;
m est 1, 2, 3 ou 4;
chaque g est indépendamment 12, 14 ou 16 ;
p est 2, 3 ou 4 ;
test 2, 3 ou 4 ;
q est nul ou 1 ;
R₁, R₁', R₂ et R₂' sont indépendamment sélectionnés dans le groupe constitué de H, -CH₂OH, -CH₂CH₂OH, -CH(CH₃)OH et -CH₂OPO(OH)₂, dans laquelle l'un quelconque des hydrogènes d'alkyle peut être remplacé par un halogène, et dans laquelle R₁ et R₁' ne sont pas tous les deux H et R₂ et R₂' ne sont pas tous les deux H ;
dans laquelle, lorsque q est nul, R₃ est H ;
dans laquelle, lorsque q est 1, R₃ est -NH₂ ou -OH ;
L est nul ou est constitué de 1 à 10 unités, dans laquelle chaque unité est un acide alpha-aminé naturel ou est dérivé d'un acide alpha-aminé naturel, et a la formule : 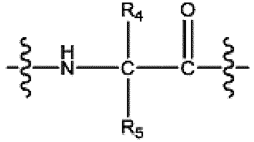
dans laquelle R₄ est H ; et
R₅ est la chaîne latérale, ou un second hydrogène de l'acide aminé,
ou un de ses sels ou une de ses prodrogues pharmaceutiquement acceptables.

15. Composé selon l'une quelconque des revendications 1 à 14, dans lequel l'agoniste de TLR2 est administré une fois par jour ou une fois par semaine.

16. Composé selon l'une quelconque des revendications 1 à 15, dans lequel l'utilisation comprend en outre l'administration d'un corticostéroïde, de préférence un glucocorticoïde.

17. Composé selon l'une quelconque des revendications 1 à 16, dans lequel le composé est formulé ou adapté pour une administration aux voies respiratoires.

18. Composé ou composition selon la revendication 17, dans lequel le composé ou la composition est formulé ou adapté pour une administration par inhalation ou intranasale.

19. Composé ou composition selon la revendication 18, dans lequel le composé ou la composition est formulé en tant que pulvérisation nasale ou gouttes nasales.
